# EUROPEAN PATENT SPECIFICATION

(11) **EP 2 231 697 B1**
(45) Date of publication and mention of the grant of the patent: **12.02.2014**
(21) Application number: 08859973.3
(22) Date of filing: 12.12.2008
(51) Int. Cl.: C12N 9/22, C07K 14/195, C12N 15/62

(54) **IMPROVED CHIMERIC MEGANUCLEASE ENZYMES AND USES THEREOF**
VERBESSERTE CHIMÄRE MEGANUKLEASEENZYME UND ANWENDUNGEN DAVON
ENZYMES AMÉLIORÉES DE MÉGANUCLÉASE CHIMÈRE ET LEURS UTILISATIONS

(30) Priority: 13.12.2007 WO PCT/IB2007/004418
(43) Date of publication of application: 29.09.2010
(73) Proprietor: Cellectis, 93235 Romainville Cedex (FR)
(72) Inventor: GRIZOT, Sylvestre, F-92250 La Garenne Colombes (FR); DUCHATEAU, Philippe, F-02810 Gandelu (FR)
(74) Representative: Vial, Lionel
(86) International application number: PCT/IB2008/003744
(87) International publication number: WO 2009/074873

(56) References cited:
- WO-A-2005/105989
- CHEVALIER B S ET AL: "Design, activity, and structure of a highly specific artificial endonuclease" MOLECULAR CELL, CELL PRESS, CAMBRIDGE, MA, US, vol. 10, no. 4, 20 October 2002 (2002-10-20), pages 895-905, XP002248750 ISSN: 1097-2765 cited in the application
- PRIETO JESUS ET AL: "Generation and analysis of mesophilic variants of the thermostable archaeal I-DmoI homing endonuclease"[Online] XP002493369 Manuscript M706323200 online 12-11-2007 Retrieved from the Internet: URL:http.//www.jbc.org/cgi/doi/10.1074/jbc .M706323200> cited in the application
- EPINAT J-C ET AL: "A novel engineered meganuclease induces homologous recombination in yeast and mammalian cells" NUCLEIC ACIDS RESEARCH, OXFORD UNIVERSITY PRESS, SURREY, GB, vol. 31, no. 11, 1 June 2003 (2003-06-01), pages 2952-2962, XP002248751 ISSN: 0305-1048 cited in the application
- SMITH JULIANNE ET AL: "A combinatorial approach to create artificial homing endonucleases cleaving chosen sequences" 27 November 2006 (2006-11-27), NUCLEIC ACIDS RESEARCH, OXFORD UNIVERSITY PRESS, SURREY, GB, PAGE(S) E149 - 1 , XP002457876 ISSN: 0305-1048 cited in the application the whole document
- SILVA G H ET AL: "Analysis of the LAGLIDADG interface of the monomeric homing endonuclease I-DmoI" NUCLEIC ACIDS RESEARCH, OXFORD UNIVERSITY PRESS, SURREY, GB, vol. 32, no. 10, 1 June 2004 (2004-06-01), pages 3156-3168, XP002364698 ISSN: 0305-1048
- PÂQUES FRÉDÉRIC ET AL: "Meganucleases and DNA double-strand break-induced recombination: perspectives for gene therapy." CURRENT GENE THERAPY FEB 2007, vol. 7, no. 1, February 2007 (2007-02), pages 49-66, XP002493370 ISSN: 1566-5232

## Description

The invention relates to chimeric meganuclease enzymes comprising a modified *I-DmoI* domain having improved activity and altered DNA target sequences. In particular the invention relates to chimeric meganuclease enzymes comprising a modified *I-DmoI* domain linked to an *I-CreI* monomer.

Among the strategies to engineer a given genetic locus, the use of rare cutting DNA endonucleases such as meganucleases has emerged as a powerful tool to increase the rate of successful gene targeting through the generation of a DNA double strand break (DSB) by a rare cutting DNA endonuclease and a homologous recombination event at the site of the break.

Meganucleases are endonucleases, which recognize large (12-45 bp) DNA target sites. In the wild, meganucleases essentially comprise homing endonucleases, a family of very rare-cutting endonucleases. This family was first characterized by the use *in vivo* of the protein *I-SceI* (Omega nuclease), originally encoded by a mitochondrial group I intron of the yeast *Saccharomyces cerevisiae.* Homing endonucleases encoded by intron ORFs, independent genes or intervening sequences (inteins) present striking structural and functional properties that distinguish them from "classical" restriction enzymes which generally have been isolated from the bacterial system R/MII.

Homing endonucleases have recognition sequences that span 12-40 bp of DNA, whereas "classical" restriction enzymes recognize much shorter stretches of DNA, in the 3-8 bp range (up to 12 bp for a so called rare-cutter). Therefore homing endonucleases have a very low frequency of cleavage, even in a genome as large and complex as that of a human.

Several homing endonucleases encoded by group I intron or inteins have been shown to promote the homing of their respective genetic elements into allelic intronless or inteinless sites. By making a site-specific double-strand break in the intronless or inteinless alleles, these nucleases create recombinogenic ends, which engage in a gene conversion process that duplicates the coding sequence and leads to the insertion of an intron or an intervening sequence at the DNA level.

Homing endonucleases fall into four separate families, classified on the basis of conserved amino acids motifs. For review, see Chevalier and Stoddard (Nucleic Acids Research, 2001, 29, 3757-3774).

One of these families and the subject of the present invention is the LAGLIDADG family, the largest of the homing endonucleases families. This family is characterized by a conserved tridimensional structure (see below), but displays very poor conservation at the primary sequence level, except for a short peptide above the catalytic center. This family has been called LAGLIDADG, after a consensus sequence for this peptide, found in one or two copies in each LAGLIDADG protein.

Homing endonucleases with one LAGLIDADG (L) are around 20 kDa in molecular mass and act as homodimers. Those with two copies (LL) range from 25 kDa (230 amino acids) to 50 kDa (HO, 545 amino acids) with 70 to 150 residues between each motif and act as a monomer. Cleavage of the target sequence occurs inside the recognition site, leaving a 4 nucleotide staggered cut with 3'OH overhangs.

I-*Ceu*I and *I-CreI* (163 amino acids) are homing endonucleases with one LAGLIDADG motif (mono-LAGLIDADG). *I-DmoI* (194 amino acids, SWISSPROT accession number P21505 (SEQ ID NO: 22)), I-*SceI,* PI*-PfuI* and PI-*Sce*I are homing endonucleases with two LAGLIDADG motifs.

In the present invention, unless otherwise mentioned, the residue numbers refer to the amino acid numbering of the *I-DmoI* sequence SWISSPROT number P21505 (SEQ ID NO: 22) or the structure PDB code 1b24.

Structural models using X-ray crystallography have been generated for *I-CreI* (PDB code 1g9y), *I-DmoI* (PDB code 1b24), PI-*Sce* I, PI-*Pfu*I. Structures of *I-CreI* and PI-*Sce*I (Moure et al., Nat Struct Biol, 2002, 9: 764-70) bound to their DNA site have also been elucidated leading to a number of predictions about specific protein-DNA contacts.

LAGLIDADG proteins with a single motif, such as *I-CreI* (SEQ ID NO: 24), form homodimers and cleave palindromic or pseudo-palindromic DNA sequences, whereas the larger, double motif proteins, such as *I-SceI are* monomers and cleave non-palindromic targets. Several different LAGLIDADG proteins have been crystallized and they exhibit a striking conservation of the core structure that contrasts with a lack of similarity at the primary sequence level (Jurica et al., Mol Cell. 1998; 2:469-76, Chevalier et al., Nat Struct Biol. 2001; 8:312-6, Chevalier et al., J Mol Biol. 2003; 329:253-69, Moure et al., J Mol Biol. 2003; 334:685-95, Moure et al., Nat Struct Biol. 2002; 9:764-70, Ichiyanagi et al., J Mol Biol. 2000; 300:889-901, Duan et al., Cell. 1997; 89:555-64, Bolduc et al., Genes Dev. 2003; 17:2875-88, Silva et al., J Mol Biol. 1999; 286:1123-36).

In this core structure, two characteristic αββαββα folds, contributed by two monomers in dimeric LAGLIDADG proteins or by two domains in monomeric LAGLIDADG proteins, face each other with a two-fold symmetry. DNA binding depends on the four β strands from each domain, folded into an antiparallel β-sheet, and forming a saddle on the DNA helix major groove. The catalytic core is central, with a contribution of both symmetric monomers/domains. In addition to this core structure, other domains can be found: for example, *PI-SceI,* an intein, has a protein splicing domain, and an additional DNA-binding domain (Moure et al., Nat Struct Biol. 2002; 9:764-70, Grindl et al., Nucleic Acids Res. 1998; 26:1857-62).

Despite an apparent lack of sequence conservation between individual members of the LAGLIDADG family, structural comparisons indicate that LAGLIDADG proteins, should they cut as dimers like *I-CreI* or as a monomer like *I-DmoI,* adopt a similar active conformation. In all structures, the LAGLIDADG motifs are central and form two packed α-helices where a 2-fold (pseudo-) symmetry axis separates two monomers or apparent domains.

The LAGLIDADG motif corresponds to residues 13 to 21 in *I-CreI,* and to positions 14 to 22 and 110 to 118, *in I-DmoI.* On either side of the LAGLIDADG α-helices, a four β-sheet provides a DNA binding interface that drives the interaction of the protein with the half site of the target DNA sequence. *I-DmoI* is similar to *I-CreI* dimers, except that the first domain (residues 1 to 95) and the second domain (residues 105 to 194) are separated by a linker (residues 96 to 104) (Epinat et al., Nucleic Acids Res, 2003, 31: 2952-62).

*I-SceI* was the first homing endonuclease used to stimulate homologous recombination over 1000-fold at a genomic target in mammalian cells (Choulika et al., Mol Cell Biol. 1995; 15:1968-73, Cohen-Tannoudji et al., Mol Cell Biol. 1998; 18:1444-8, Donoho et al., Mol Cell Biol. 1998; 18:4070-8, Alwin et al., Mol Ther. 2005; 12:610-7, Porteus., Mol Ther. 2006; 13:438-46, Rouet et al., Mol Cell Biol. 1994; 14:8096-106).

Recently, *I-SceI* was also used to stimulate targeted recombination in mouse liver *in vivo*, and recombination could be observed in up to 1% of hepatocytes (Gouble et al., J Gene Med. 2006; 8:616-22). An inherent limitation of such a methodology is that it requires the prior introduction of the natural *I-SceI* cleavage site into the locus of interest.

To circumvent this limitation, significant efforts have been made over the past years to generate zinc finger nucleases with tailored cleavage specificities (Porteus M H et al.,. Nat Biotechnol. 2005; 23:967-73, Ashworth et al., Nature. 2006; 441:656-9, Umov et al., Nature. 2005; 435, 646-651, Smith et al., Nucleic Acids Res. 2006, 2006; 34:e149).

Given their high level of specificity, homing endonucleases represent ideal scaffolds for engineering tailored endonucleases. Several studies have shown that the DNA binding domain from LAGLIDADG proteins, (Chevalier et al., Nucleic Acids Res. 2001; 29:3757-74) could be engineered.

Several LAGLIDADG proteins, including *PI-SceI* (Gimble et al., J Mol Biol. 2003; 334:993-1008), *I-CreI* (Seligman et al., Nucleic Acids Res. 2002; 30:3870-9, Sussman et al., J Mol Biol. 2004; 342:31-41, Rosen et al., Nucleic Acids Res. 2006; Arnould et al., J Mol Biol. 2006; 355:443-58), *I-SceI* (Doyon et al., J Am Chem Soc. 2006; 128:2477-84) and *I-MsoI* (Ashworth et al., Nature. 2006; 441:656-9) have been modified by rational or semi-rational mutagenesis and screening to acquire new sequence binding or cleavage specificities.

Recently, semi rational design assisted by high throughput screening methods have allowed the Applicants to derive thousands of novel proteins from *I-CreI,* an homodimeric protein from the LAGLIDADG family (Smith et al., Nucleic Acids Res. 2006; 34: e149; Arnould et al., J Mol Biol. 2006; 355:443-58).

The Applicants have previously identified the DNA-binding sub-domains of *I-CreI* and shown that these were independent enough to allow for a combinatorial assembly of mutations (Smith et al., Nucleic Acids Res. 2006; 34: e149). These findings allowed for the production of a second generation of engineered *I-CreI* derivatives, cleaving chosen targets.

This combinatorial strategy, has been illustrated by the generation of meganucleases cleaving a natural DNA target sequence located within the human RAG1 and XPC genes (Smith et al., Nucleic Acids Res. 2006; 34: e149; Arnould et al., J Mol Biol. 2007; 371:49-65).

However, although the capacity to combine up to four sub-domains considerably increases the number of DNA sequences that can be targeted, it is still difficult to prepare a suite of enzymes which can act upon the complete range of sequences possible for a natural target sequence of a given size.

One of the most elusive factors is the impact of the four central nucleotides of the *I-CreI* target site. Despite the absence of base specific protein-DNA interactions in this region, *in vitro* selection of cleavable *I-CreI* targets from a library of randomly mutagenized sites revealed the importance of these four base-pairs for cleavage activity (Argast et al., J Mol Biol. 1998; 280:345-53). More generally, it is unlikely that engineered meganucleases cleaving every possible 22 base pair sequence could be derived solely from the *I-CreI* scaffold.

Another strategy is to combine domains from distinct meganucleases. This approach has been illustrated by the creation of new meganucleases by domain swapping between *I-CreI* and *I-DmoI,* leading to the generation of a meganuclease cleaving the hybrid sequence corresponding to the fusion of the two half parent target sequences (Epinat et al., Nucleic Acids Res. 2003; 31:2952-62, Chevalier et al., Mol. Cell. 2002; 10:895-905).

*I-DmoI* is a 22 kDa endonuclease from the hyperthermophilic archae *Desulfurococcus mobilis.* It is a monomeric protein comprising two similar domains, which have both a LAGLIDADG motif. The structure of the protein alone, without its DNA target henceforth referred to as D1234 (SEQ ID NO: 30), has been solved (Silva et al., J Mol Biol. 1999; 286:1123-36).

The research group of Chevalier et al., (Mol Cell. 2002; 10:895-905) has built a chimeric protein based on the two endonucleases *I-DmoI* and *I-CreI* that was called *E-DreI* (Engineered *I-DmoI*/*I-CreI*). *E-DreI* consists of the fusion of the N-terminal domain of *I-DmoI* to a single subunit of the *I-CreI* homodimer linked by a flexible linker to create the initial scaffold for the enzyme. Chevalier et al., then made a number of residue modifications based upon the predictions of computational interface algorithms so as to alleviate any potential steric clashes predicted from a 3D model generated by combining elements of previously generated *I-DmoI* and *I-CreI* models.

In Chevalier et al., 2002 precited, residues were identified between the facing surfaces of the two component molecules; in particular residues at positions 47, 51, 55, 108, 193 and 194 of the *E-DreI* scaffold were identified as potentially clashing. These residues were replaced with alanine residues but such a modified protein was found to be insoluble.

Residue numbers refer to the *E-DreI* open reading frame which comprises 101 residues (beginning at the first methionine) from the N-terminal domain of *I-DmoI* fused to the last 156 residues of *I-CreI* separated by a three amino acid NGN linker which mimics the native *I-DmoI* linker in length.

The interface was then optimised through a combination of computational redesign for residues 47, 51, 55, 108, 193 and 194 as well as residues 12, 13, 17, 19, 52, 105, 109 and 113; followed by an *in vivo* protein folding assay upon selected sequences to determine the solubility of *E-DreI* enzymes modified at these residues. A final scaffold was designed with modifications: I19, H51 and H55 of *I-DmoI* and E8, L11, F16, K96 and L97 of *I-CreI* (corresponding to E105, L108, F113, K193 and L194).

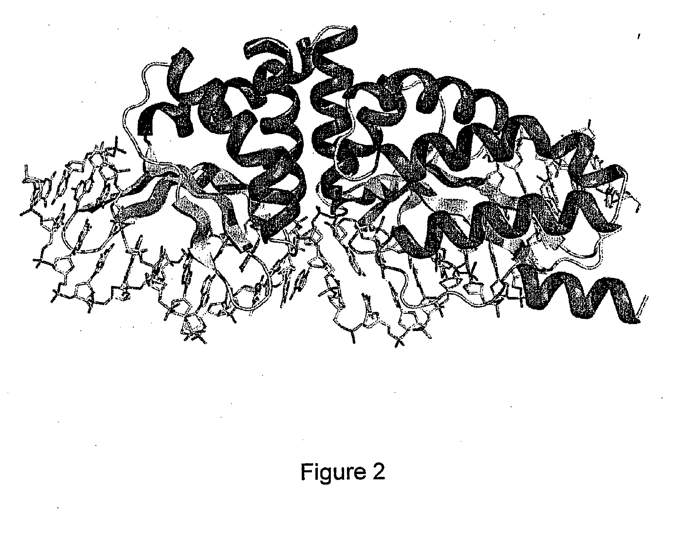

The *E-DreI* (Chevalier et al., Mol Cell. 2002; 10:895-905) structure in complex with its chimeric DNA target dre3 (C12D34 (SEQ ID NO: 31) using the applicants nomenclature) was solved as shown in Figure 2 herein. *E-DreI* was shown able to recognise and cut this hybrid C12D34 (SEQ ID NO: 31) target only. From this structure a number of residues were predicted to be base-specific contacts of *E-DreI* to its target hybrid site, these residues were 25, 29, 31, 33, 34, 35, 37, 70, 75, 76, 77, 79, 81 of *I-DmoI;* and residues 123, 125, 127, 130, 135, 137, 139, 141, 163, 165, 167, 172 of *I-CreI* in *E-DreI.*

The Applicants have also previously conducted experiments with a *DmoCre* scaffold to seek to broaden the range of DNA target sequences cleaved by engineered homing nuclease enzymes. *DmoCre* is a chimeric molecule built from the two homing endonucleases *I-DmoI* and *I-CreI.* It includes the N-terminal portion from *I-DmoI* linked to an *I-CreI* monomer. *DmoCre* could have a tremendous advantage as *CreI* domain, and thousands of such variant *I-CreI* molecules have already been identified and profiled (Smith J et al., Nucleic Acids Res. 2006;34(22):e149 , Arnould S et al., J Mol Biol. 2006; 355:443-58, Arnould S et al., J Mol Biol. 2007; 371:49-65).

Based upon the structure of the *I-DmoI* protein alone, without its DNA target (Silva et al., J Mol Biol. 1999; 286:1123-36) and on the structure of the complex between *I-CreI* and its DNA target C1234 (SEQ ID NO: 28) (Jurica et al., Mol Cell. 1998; 2:469-76, Chevalier et al., J Mol Biol. 2003; 329:253-69), a chimeric *DmoCre* endonuclease has been built (Epinat et al., Nucleic Acids Res, 2003, 31: 2952-62). *DmoCre* is a monomeric protein that corresponds to *I-DmoI* up to residue F109 followed by *I-CreI* from residue L13. To avoid a steric clash, I107 has been mutated into a leucine residue. In addition, residues 47, 51 and 55 of *I-DmoI,* which were found to be close to residues 96 and 97 of *I-CreI,* were mutated to alanine, alanine and aspartic acid respectively.

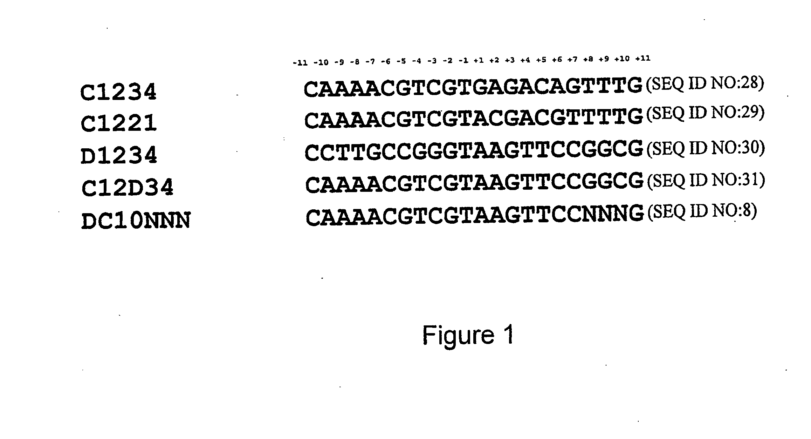

*DmoCre* has been shown to be active *in vitro* (Epinat et al., Nucleic Acids Res, 2003, 31: 2952-62) and was able to cleave the hybrid target C12D34 (SEQ ID NO: 31) composed from the left part of C1234 (SEQ ID NO: 28) or C1221 (SEQ ID NO: 29) (the palindromic target derived from C1234) and the D1234 (SEQ ID NO: 30) right part (Figure 1). Furthermore *I-DmoI* and *DmoCre* variants able to cleave their DNA target sequences more efficiently at 37°C were identified by random mutagenesis and screening in yeast cells (WO 2005/105989; Prieto et al., J. Biol Chem. [2007 Nov 12; Epub ahead of print], 283 : 4364-4374).

The *E-DreI* and *DmoCre* chimeric enzymes are therefore only capable of recognizing and cutting the hybrid target C12D34 (SEQ ID NO: 31). In addition the scaffolds of *E-DreI* and *DmoCre* have in common the modification of residues 47, 51 and 55.

The inventors are interested in creating a new generation of chimeric enzymes which recognize a wider set of target sequences and therefore they have investigated the further enhancement of the first domain of the *I-DmoI* enzyme for use as either a component in a chimeric *I-DmoI* enzyme or a chimeric enzyme comprising catalytic domains from two different nucleases. By being able to target new DNA sequences and so induce a double-strand break in a site of interest comprising a DNA target sequence, the applicants provide the tools to thereby induce a DNA recombination event, a DNA loss or cell death.

This double-strand break can be used to: repair a specific sequence, modifying a specific sequence, restoring a functional gene in place of a mutated one, attenuating or activating an endogenous gene of interest, introducing a mutation into a site of interest, introducing an exogenous gene or a part thereof, inactivating or detecting an endogenous gene or a part thereof, translocating a chromosomal arm or leaving the DNA unrepaired and degraded. Such modified meganuclease enzymes therefore give a user a wide variety of potential options in the therapeutic, research or other productive use of such modified meganuclease enzymes.

The inventors have therefore sought to improve chimeric meganuclease enzymes comprising at least one *I-DmoI* domain by seeking to increase the number of DNA targets these chimeric enzymes can recognize and cut.

Therefore the present invention relates to a polypeptide as defined in claims 1 to 8.

The specification provides a polypeptide encoding an improved *I-DmoI* endonuclease or a derivative thereof, such as a chimeric enzyme comprising the first domain of *I-DmoI* in combination with another functional endonuclease domain or monomer. This polypeptide has two or more amino acid residue changes in the first *I-DmoI* domain corresponding to residues 1 to 99 of the native *I-DmoI* protein. In particular the first *I-DmoI* domain corresponds to positions 1 to 99 in the *I-DmoI* amino acid sequence, the *I-DmoI* linker to positions 100 to 104 and the beginning of the second *I-DmoI* domain to positions 105 to 109 which is the complete fragment used in *DmoCre2* and *DmoCre4,* two new chimeric meganuclease scaffolds which the applicants have developed and describe herein. Preferably the complete 109 residue fragment is used as the first *I-DmoI* domain fragment in a chimeric enzyme, but it is also possible to use smaller portions of this, such as just the 1 to 95 residue *I-DmoI* domain so long as such smaller fragments retain the ability to recognize and cleave specific target sequences.

Changes to residues 15, 19 and 20 have been experimentally shown by the inventors to result in increased activity of the chimeric protein called *DmoCre2* by the inventors. Changes to residues 29, 30 and 35 have been shown for the first time by the applicants to alter the sequence recognised by this modified domain of *I-DmoI* at positions ±*8* to ±*10* of the *I-DmoI* DNA target half-site (SEQ ID NO: 23). Changes to residues 75, 76 and 77 have been shown by the inventors for the first time to alter the sequence recognised by this modified domain of *I-DmoI* at positions ±2 to ±4 of the I-DmoI DNA target half-site (SEQ ID NO: 23). Therefore the inventors provide an improved first *I-DmoI* domain which is capable of recognising target sequences different to the hybrid sequence C12D34. The *I-DmoI* DNA target half-site (SEQ ID NO:23) is AAGTTCCGGCG, the +*2 to* +*4* and +8 to +10 regions are in bold.

Such a polypeptide comprises a modified meganuclease and allows a wider range of DNA target sequences to be recognised and cut, other than the hybrid target sequence recognised and cut by *DmoCre* and *E-DreI.*

In particular, the polypeptide according to the invention may comprise the modification of the lysine in position 15 which is changed to a glutamine, a L 15Q change.

The polypeptide according to the invention comprises the modification of the isoleucine in position 19 changed to aspartic acid, a I19D change. Modification of residue 19 has been shown by the applicants to render the *I-DmoI* domain more active.

The polypeptide according to the invention comprises the modification of the glycine in position 20 which is changed to serine a G20S change.

In particular the polypeptide may also comprise at least one modified residue at position 107. I19D change. Modification of residue 19 has been shown by the applicants to render the *I-DmoI* domain more active.

In particular, the polypeptide according to the invention may comprise the modification of the glycine in position 20 which is changed to serine or alanine, a G20S or G20A change.

In particular the polypeptide may also comprise at least one modified residue at position 107.

In particular the polypeptide according to the invention comprises the modification of the isoleucine in position 107 to a lysine, a I107L modification. Modification of residue 107 should prevent a steric clash between the *I-DmoI* domain and the other domain of the enzyme for instance *I-CreI.*

In particular the substitution of at least one of the residues in positions 29, 33 or 35 by any amino acid, alters the recognition of said polypeptide for an *I-DmoI* DNA target half-site which differs from a wildtype *I-DmoI* DNA target half-site SEQ ID NO: 30, in at least one of positions ±8, ±9, ±10.

In particular the substitution of at least one of the residues in positions 75, 76 or 77 by any amino acid, alters the recognition of said polypeptide for an *I-DmoI* DNA target half-site which differs from a wildtype *I*-*DmoI* DNA target half-site SEQ ID NO: 30, in at least one of positions ±2 ±3, ±4.

In particular the substitution of at least one of the residues in positions 27, 37 or 81 by any amino acid, alters the recognition of said polypeptide for an *I-DmoI* DNA target half-site which differs from a wildtype *I-DmoI* DNA target half-site SEQ ID NO: 30, in at least one of positions ±5, ±6, ±7.

In particular, the polypeptide is derived from the sequence SEQ ID *NO:* 1.

In the current application derived from, means any nucleic acid or protein sequence which is created from an original sequence and then modified so as to retain its original functionality but has residue changes and/or additions or deletions relative to the original sequence whilst retaining its functionality.

SEQ ID NO: 1 is the sequence of an *I-DmoI* domain modified at residues 15 and 19 used in the current invention as the *I-DmoI* domain in *DmoCre2* (SEQ ID NO: 2). This *I-DmoI* domain also contains a modification to residue 107, but no modifications to L47A, H51A and L55D as per Epinat et al., (Nucleic Acids Res, 2003, 31: 2952-62).

In particular, the polypeptide is derived from the sequence SEQ ID NO: 27.

SEQ ID NO: 27 is the sequence of a modified *I-DmoI* domain modified at residues 19, 20 and 109 used in the current invention as the *I-DmoI* domain in *DmoCre4* (SEQ ID NO: 9) by the applicants. This *I-DmoI* domain does not contain the modifications to L47A, H51A and L55D as per Epinat et al., (Nucleic Acids Res, 2003, 31: 2952-62).

In particular, the polypeptide is a chimeric *I-DmoI* endonuclease consisting of the fusion of the first *I-DmoI* domain to a sequence of a dimeric LAGLIDADG homing endonuclease or to a domain of another monomeric LAGLIDADG homing endonuclease.

The current invention concerns modified *I-DmoI* endonuclease enzymes comprising both a modified first *I-DmoI* domain and a second wildtype *I-DmoI* domain comprising residues 1-95 of SEQ ID NO:22 in a single monomeric protein or alternatively the combination of two *I-DmoI* domains altered according to the current invention. It is also an aspect of the present invention that the modified I-*DmoI* domain may be combined with a domain of another LAGLIDADG endonuclease, such as *I-Sce I, I-Chu I, I-Cre I, I-Csm I, PI-Sce I, PI-Tli I, PI-Mtu I, I-Ceu I, I-Sce II, I-Sce III, HO, PI-Civ I, PI-Ctr I, PI-Aae-1, PI-Bsu I, PI-Dha I, PI-Dra I, PI-Mav I, PI-Mch I, PI-Mfu I, PI-Mfl I, PI-Mga I, PI-Mgo I, PI-Min I, PI-Mka I, PI-Mle I, PI-Mma I, PI-Msh I, PI-Msm I PI-Mth I, PI-Mtu I, PI-Mxe I, PI-Npu I, PI-Pfu I, PI-Rma I, PI-Spb I, PI-Ssp I, PI-Fac I, PI-Mja I, PI-Pho I, PI-Tag I, PI-Thy I, PI-Tko I, I-MsoI, and PI-Tsp I ;* preferably, *I-Sce I, I-Chu I, I-Dmo I, I-Csm I, PI-Sce I, PI-Pfu* I, *PI-Tli* I, *PI-Mtu I,* and *I-Ceu I.*

In addition the current invention concerns a polypeptide wherein the sequence of the first domain of *I-DmoI,* also comprises the substitution of at least one further residue selected from the group: (i) one of the residues in positions 4, 49, 52, 92, 94 and/or 95 of said first *I-DmoI* domain, and/or (ii) one of the residues in positions 101, 102, and/or 109 of the linker or the beginning of the second domain of *I-DmoI.*
- the phenylalanine in position 109 (if present) is changed to isoleucine (F109I].

In particular, the first *I-DmoI* domain of the polypeptide is at the NH₂-terminus of said chimeric-*Dmo* endonuclease.

In particular, the dimeric LAGLIDADG homing endonuclease forming part of the chimeric-Dmo endonuclease is *I-CreI.*

In particular, the chimeric *I-DmoI* endonuclease derives from the sequence SEQ ID NO: 2.

SEQ ID NO: 2 is the peptide sequence of the preferred *DmoCre2* chimeric endonuclease of the current invention comprising an *I-DmoI* domain modified at residues 15, 19 and 107.

In particular the polypeptide is derived from the sequence SEQ ID NO: 9.

SEQ ID NO: 9 is the peptide sequence of the preferred *DmoCre4* chimeric endonuclease of the current invention comprising an *I-DmoI* domain modified at residues 19, 20 and 109.

In particular, the polypeptide according to this first aspect of the present invention may comprise a detectable tag at its NH₂ and/or COOH terminus.

The present invention also relates to a polynucleotide, this polynucleotide is characterized in that it encodes a polypeptide according to the present invention.

The present invention also relates to a vector, characterized in that it comprises a polynucleotide according to the present invention.

The present invention also relates to a host cell, characterized in that it is modified by a polynucleotide according or a vector according to the present invention.

The recombinant vectors comprising said polynucleotide may be obtained and introduced in a host cell by the well-known recombinant DNA and genetic engineering techniques.

The polypeptide of the invention may be obtained by culturing the host cell containing an expression vector comprising a polynucleotide sequence encoding said polypeptide, under conditions suitable for the expression of the polypeptide, and recovering the polypeptide from the host cell culture.

The present invention also relates to a non-human transgenic animal, characterized in that all or part of its constituent cells are modified by a polynucleotide or a vector according to the present invention.

The present invention also relates to a transgenic plant, characterized in that all or part of its constituent cells are modified by a polynucleotide or a vector according to the present invention.

The present invention also relates to a polypeptide including at least a variant of the first *I-DmoI* as defined in claim 1, fused to the sequence of an *I-CreI* monomer, wherein said *I-CreI* monomer sequence comprising the modification of at least one of the residues in positions 44, 68, 70, 75, 77 of said first *I-CreI* domain.

Such a polypeptide is able to cleave for example the 5CAGD34 (SEQ ID NO: 33) target 5CAGD34 (SEQ ID NO: 33) is the first half of the 5CAG_P target (SEQ ID NO: 32) fused to the second half of the *I-DmoI* target DNA sequence (SEQ ID NO: 30). The 5CAG_P target (SEQ ID NO: 32) refers to the wildtype *I-CreI* target DNA sequence which has been modified at positions ±3, ±4 and ±5 to the sequence CAG.

The present invention also relates to a polypeptide, comprising the sequence of an *I-DmoI* endonuclease or a chimeric derivative thereof including at least a variant of the first *I-DmoI* domain as defined in claim 1, fused to the sequence of an *I-CreI* monomer, wherein said *I-CreI* monomer sequence comprising the modifcation of at least one of the residues in positions 30, 33, 44 of said first *I-CreI* domain.

Such a polypeptide is able to cleave for example the RAG1.10.2D34 target (SEQ ID NO: 35). RAG1.10.2D34 is the first half of the RAG1.10.2 DNA target (SEQ ID NO: 34) fused to the second half of the *I-DmoI* target DNA sequence (SEQ ID NO: 30).

The present invention also relates to a polypeptide, comprising the sequence of an *I-DmoI* endonuclease or a chimeric derivative thereof including at least a variant of the first *I*-*DmoI* domain as defined in claim 1, fused to the sequence of an *I-CreI* monomer, wherein said *I-CreI* monomer sequence comprising the modification of at least one of the residues in positions 37, 79, 81 of said first *I-CreI* domain.

Such a polypeptide is able to cleave a target in which the 7NNN portion of the *DmoCre*, +5 to +7 of the C12D34 (SEQ ID NO: 31) DNA target sequence differs from the wildtype nucleotide sequence target GGA.

For a better understanding of the invention and to show how the same may be carried into effect, there will now be shown by way of example only, specific embodiments, methods and processes according to the present invention with reference to the accompanying drawings in which:
Figure 1: Sequence comparison in which the different 22 bp DNA targets are represented, wherein C1234 (SEQ ID NO: 28) is the wild-*type I-CreI* target, C1221 (SEQ ID NO: 29) is the palindromic sequence derived from the left part of C1234 (SEQ ID NO: 28), D1234 (SEQ ID NO: 30) is the wild-type *I-DmoI* target and C12D34 (SEQ ID NO: 31) is the hybrid target for the chimeric *DmoCre* protein and a DC10NNN target (SEQ ID NO: 8) is a derivative from C12D34 (SEQ ID NO: 31), where degeneracy at positions +8, +9 and +10 has been introduced.
Figure 2: Shows the structure of *E-DreI* bound to its DNA target (PDB code 1MOW).
Figure 3A: Shows the molecular surface of *E-DreI* bound to its DNA target.
Figure 3B: is a zoom showing residues 29, 33 and 35 in interaction with the DNA. Dashed lines represent hydrogen bonds.
Figure 4: Schematic Restriction map of pCLS1055
Figure 5: Schematic Restriction map of pCLS0542
Figure 6: Showing an example of primary screening of *DmoCre2* mutants from the DClib2 library against 8 DC10NNN targets.

Figure 1: Sequence comparison in which the different 22 bp DNA targets are represented, wherein C1234 (SEQ ID NO: 28) is the wild-*type I-CreI* target, C1221 (SEQ ID NO: 29) is the palindromic sequence derived from the left part of C1234 (SEQ ID NO: 28), D1234 (SEQ ID NO: 30) is the wild-type *I-DmoI* target and C12D34 (SEQ ID NO: 31) is the hybrid target for the chimeric *DmoCre* protein and a DC10NNN target (SEQ ID NO: 8) is a derivative from C12D34 (SEQ ID NO: 31), where degeneracy at positions +8, +9 and +10 has been introduced.
Figure 2: Shows the structure of *E-DreI* bound to its DNA target (PDB code 1MOW).

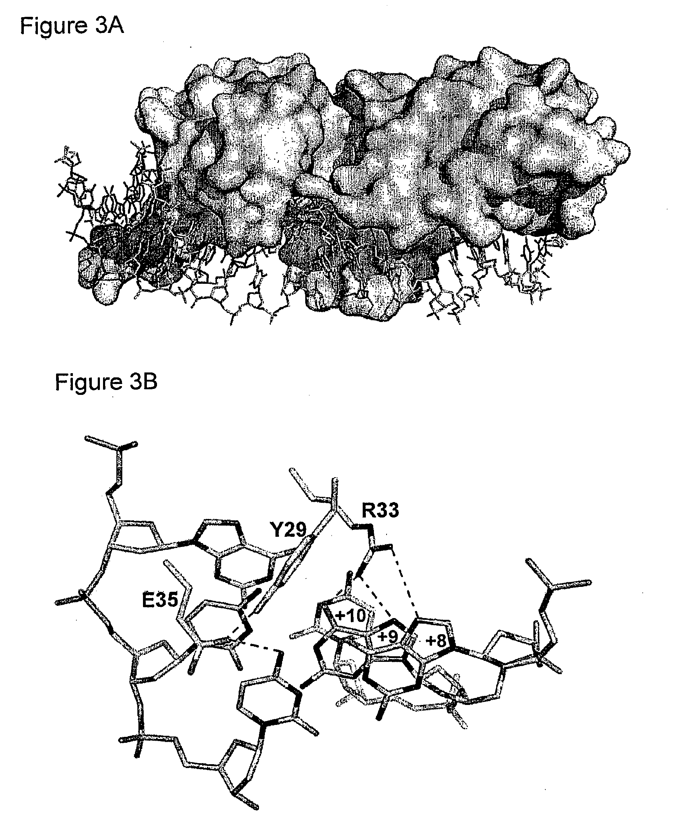

Figure 3A: Shows the molecular surface of *E-DreI* bound to its DNA target.
Figure 3B: is a zoomed in showing residues 29, 33 and 35 in interaction with the DNA. Dashed lines represent hydrogen bonds.

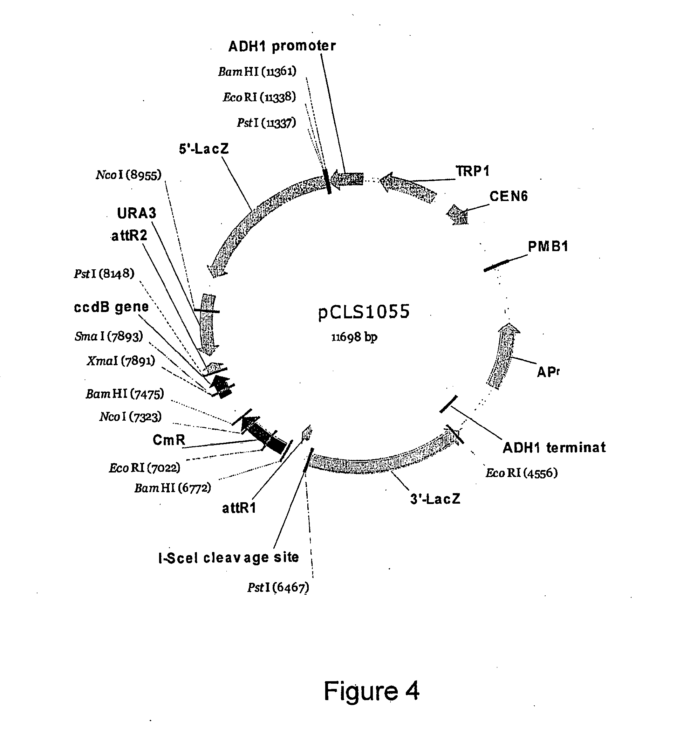

Figure 4: Schematic Restriction map of pCLS1055

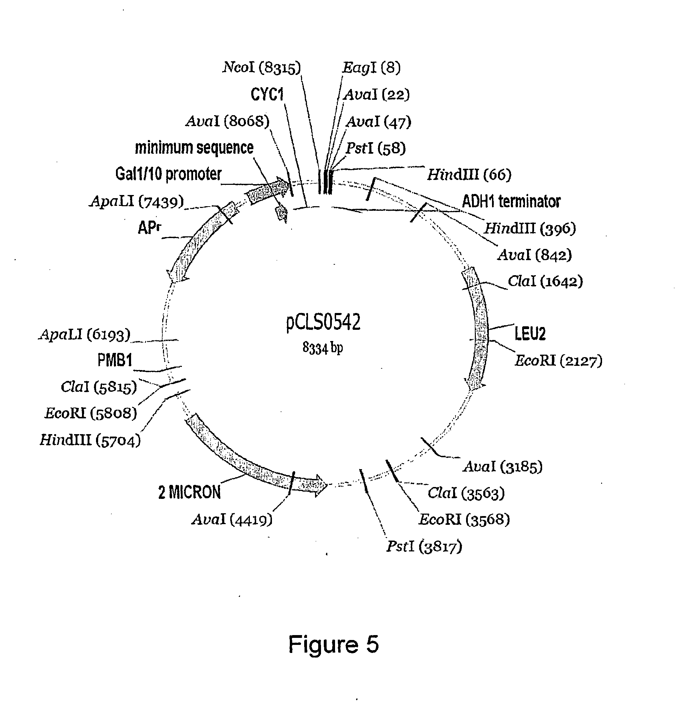

Figure 5: Schematic Restriction map of pCLS0542

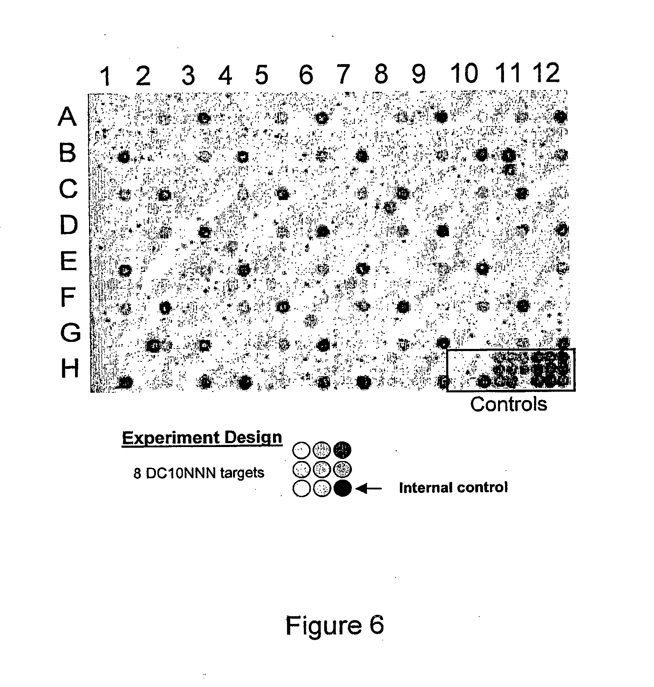

Figure 6: Showing an example of primary screening of *DmoCre2* mutants from the DClib2 library against 8 DC10NNN targets.

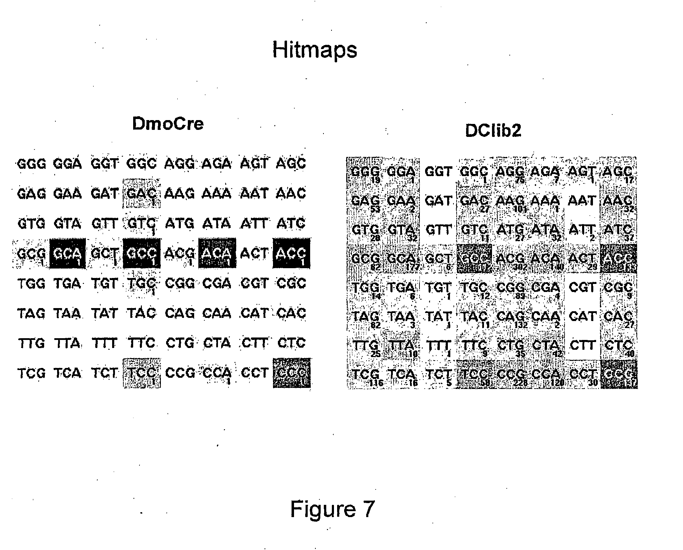

Figure 7: Shows a hit map of *DmoCre2* and the Dclib2 library against the 64 DC10NNN targets. Each target represented in the hit map refers to the complementary strands of C12D34 (SEQ ID NO: 31); for example, CGG in the hit map corresponds to the DC10CCG as defined in example 2 below.

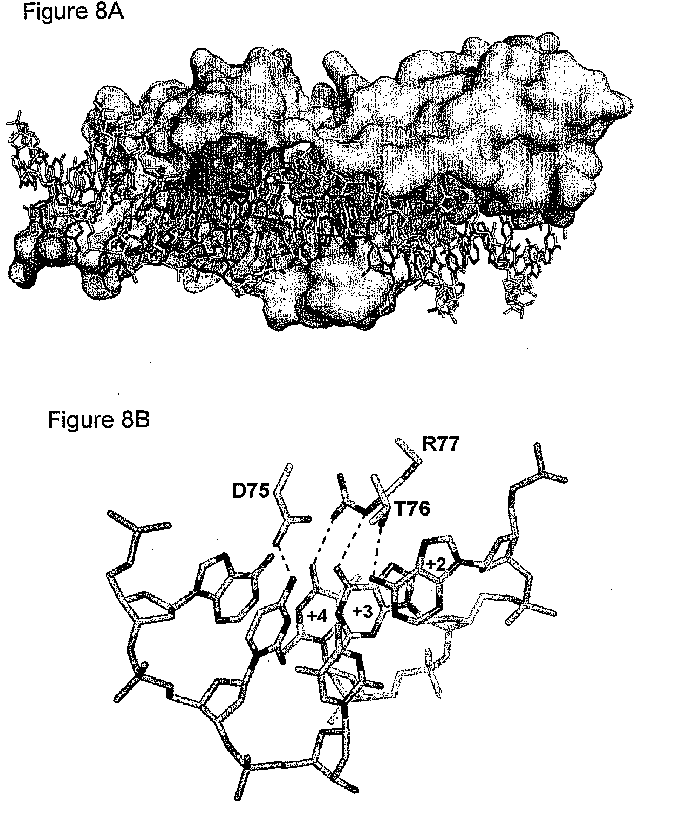

Figure 8: Molecular surface of *E-DreI* bound to its DNA target (Figure 8A). The area of binding that has been chosen for randomization (base pairs at positions +2, +3, +4 and protein residues 75, 76 and 77) has been highlighted in red. Figure 8B is a zoom showing residues 75, 76 and 77 in interaction with the DNA. Dashed lines represent hydrogen bonds.

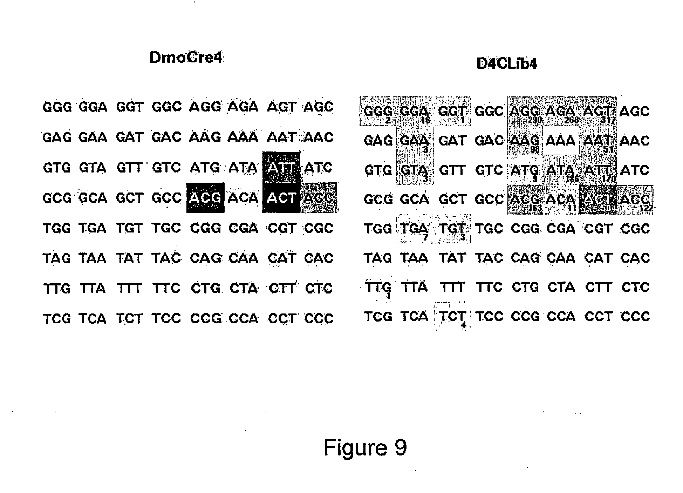

Figure 9: Hitmap of *DmoCre4* and the D4Clib4 library against 63 out of the 64 DC4NNN targets (DC4TTC is absent). For the D4Clib4 hitmap, the number below each cleaved target is the number of clones that cleaved this target. Some of these clones can have redundant sequences. For each target, the grey level is proportional to the mean of cleavage intensity. Each target represented in the hit map refers to the complementary strands of C12D34 (SEQ ID NO: 31); for example, AGG in the hit map corresponds to the DC4CCT as defined in example 3 below.

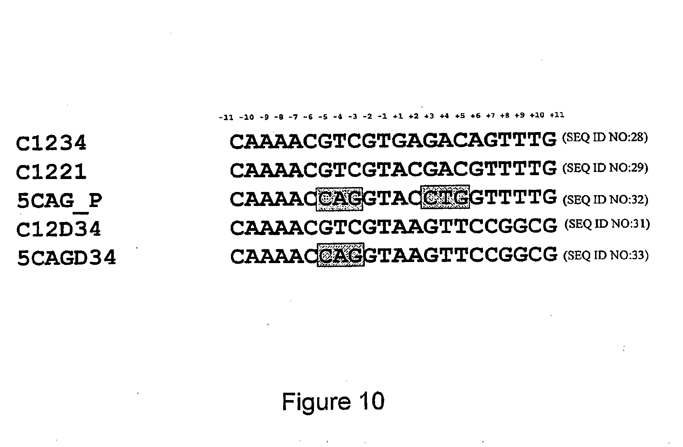

Figure 10: Some different 22 bp DNA targets are represented. The 5CAG_P (SEQ ID NO: 32) palindrome target is derived from C1221 with differences at positions ±5, ±4, ±3 that are highlighted in grey boxes. For the *I-CreI* target moiety, the 5CAGD34 (SEQ ID NO: 33) target differ from C12D34 (SEQ ID NO: 31) in the same way than 5CAG_P (SEQ ID NO: 32) differ from C1221.

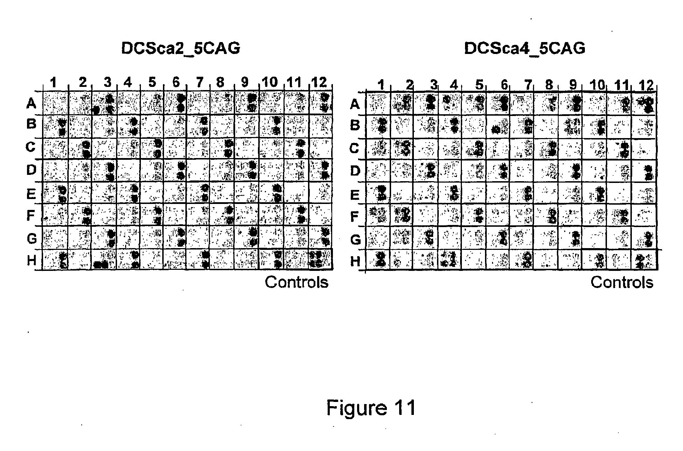

Figure 11: The figure displays an example of primary screening of DCSca2_5CAG mutants and DCSca4_5CAG mutants against the 5CAGD34 target (SEQ ID NO: 33). For the DCSca2_5CAG library screen, we used a nine dots cluster format. In each nine dots yeast cluster, a different mutant is tested against the 5CAGD34 (SEQ ID NO: 33) target in the upper left dot. For the DCSca4_5CAG library screen, we used a four dots cluster format. In each four dots cluster, a different mutant is tested against the 5CAGD34 (SEQ ID NO: 33) target in the two left dots, while the two right dots are cluster internal controls. The H10, H11 and H12 clusters contain positive and negative controls.

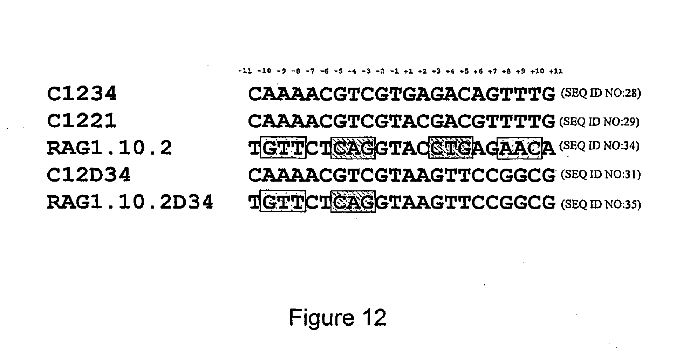

Figure 12: Different 22 bp DNA targets are represented. The RAG1.10.2 DNA sequence (SEQ ID NO: 34) is a palindromic target derived from C1221 (SEQ ID NO: 29). The 10GTT and 5CAG modules are highlighted in grey boxes. For the *I-CreI* target moiety, the RAG1.10.2D34 target (SEQ ID NO: 35) differ from C12D34 (SEQ ID NO: 31) in the same way than RAG1.10.2 (SEQ ID NO: 32) differ from C1222.

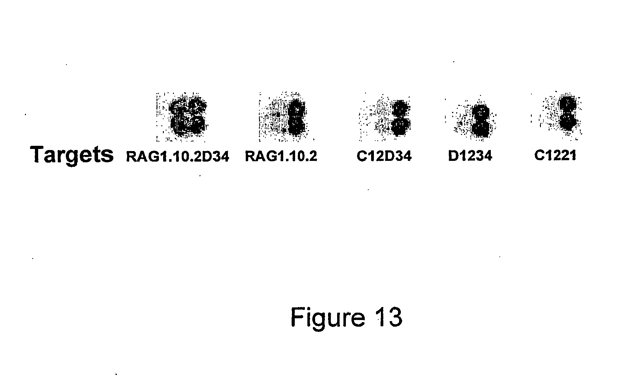

Figure 13: Yeast cleavage assay for the DmoM2 mutant against the RAG1.10.2D34 (SEQ ID NO: 35), RAG1.10.2 (SEQ ID NO: 34), C12D34 (SEQ ID NO: 31), D1234 (SEQ ID NO: 30) and C1221 (SEQ ID NO: 29) DNA targets. In each four dots yeast cluster, the two left dots represent the cleavage assay of the DmoM2 mutant with the indicated target, while the two right dots are internal controls.

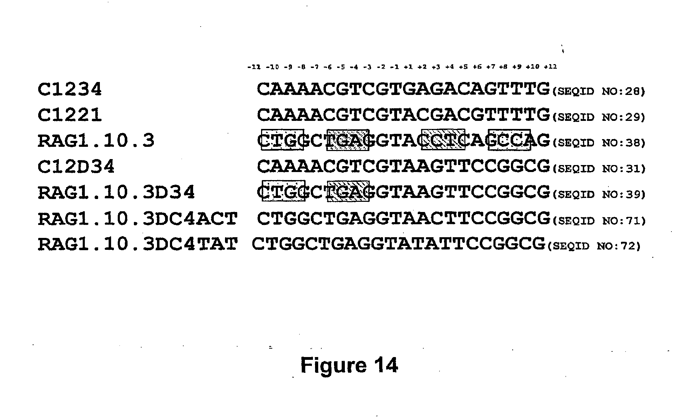

Figure 14: Different 22 bp DNA targets are represented. The RAG1.10.3 DNA sequence is a palindromic target derived from C1221. The 10TGG and 5GAG modules are highlighted in grey boxes. For the I-CreI target moiety, the RAG1.10.3D34 target differ from C12D34 in the same way than RAG1.10.3 differs from C1222.

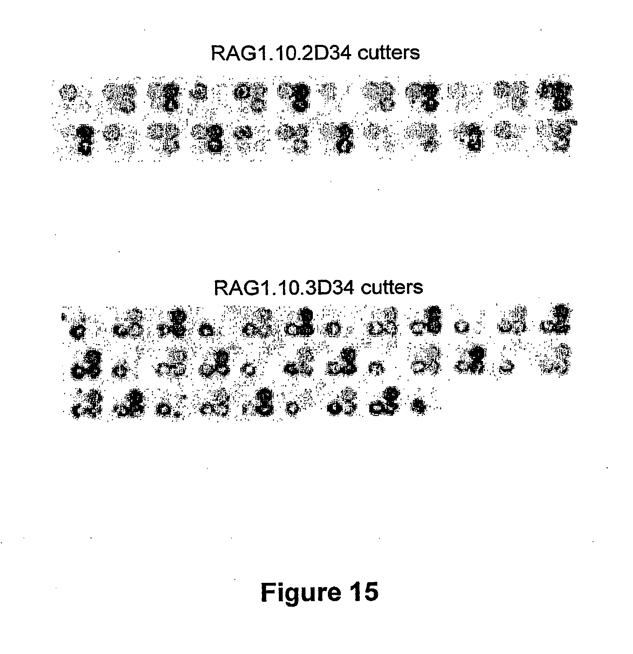

Figure 15: The figure displays an example of secondary screening of RAG1.10.2D34 and RAG1.10.3D34 cutters against their respective target. For the RAG1.10.2D34 target, a different mutant is tested against its target in the upper left dot of each yeast cluster. For the RAG1.10.3D34 target, a different mutant is tested against its target in the bottom left dot of each yeast cluster. In each four dots cluster, the two right dots are cluster internal controls.

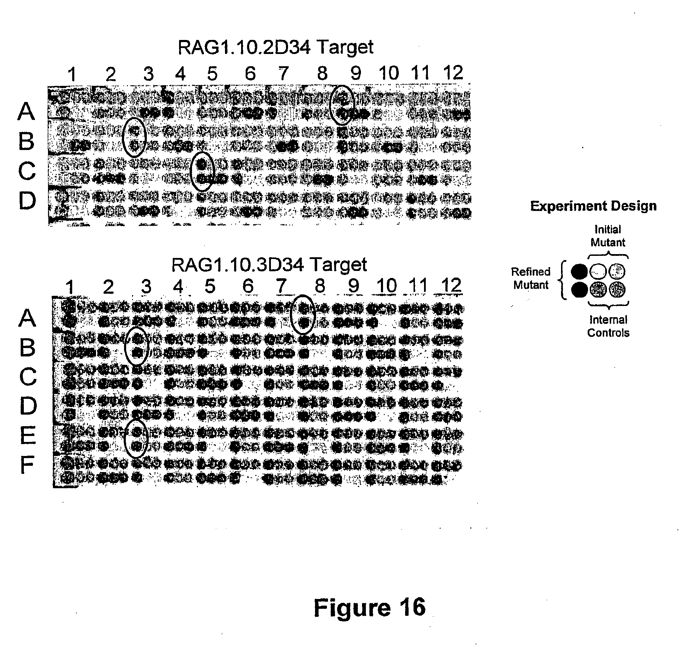

Figure 16: Secondary screening of refined RAG1.10.2D34 and RAG1.10.3D34 cutters against their respective targets. The experiment design is indicated. For the RAG1.10.2D34 screening, the initial mutant is RG2D2, while it is RG3D3 for the RAG1.10.3D34 screening. The refined RAG1.10.2D34 cutters located in A9, B3, C5 and circled in black are respectively Amell_RG2D, Amel2-RG2D, Amel3_RG2D. The refined RAG1:10.3D34 cutters located in A8, B3, E3 and circled in black are respectively AmeI1_RG3D, Amel2_RG3D, Amel3_RG3D.

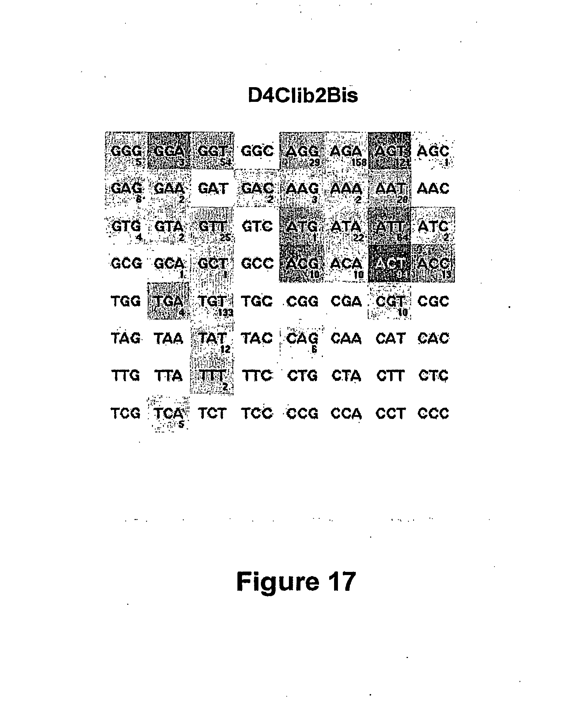

Figure 17: Hit map of the D4Clib2Bis library against the 64 DC4NNN targets. The number below each cleaved target is the number of DmoCre2 mutants with different sequences cleaving this target. For each target, the grey level is proportional to the mean of cleavage intensity. Each target represented in the hit map refers to the complementary strands of C12D34 (SEQ ID NO: 31); for example, CAG in the hit map corresponds to the DC4CTG as defined in example 3.

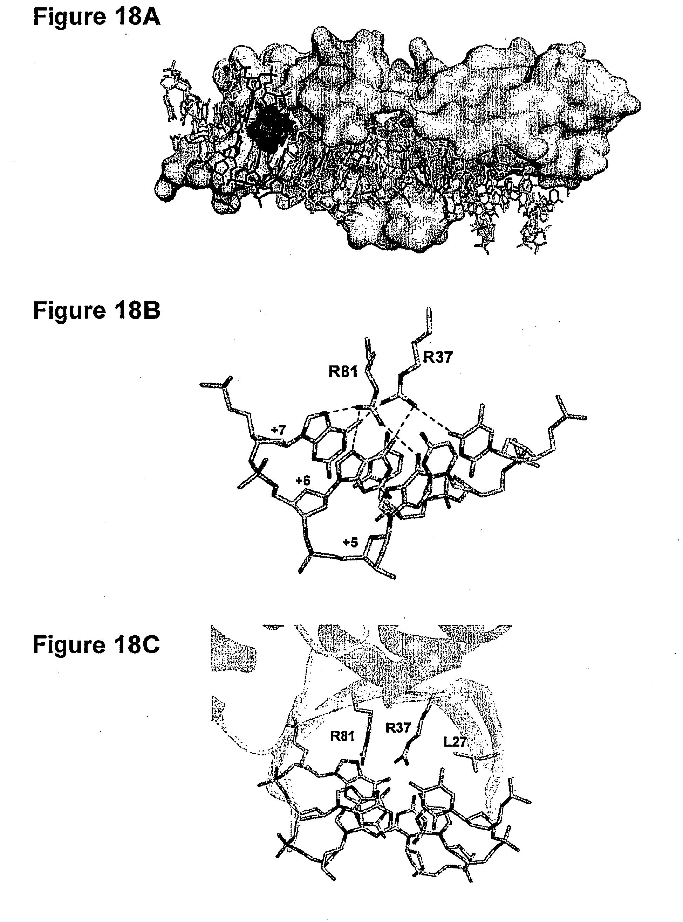

Figure 18: Molecular surface of E-DreI bound to its DNA target (Figure 17A). The area of binding that has been chosen for randomization (base pairs at positions +5, +6, +7 and protein residues 37 and 81) has been highlighted in black. Figure 16B is a zoom showing residues 37 and 81 in interaction with the DNA. Dashed lines represent hydrogen bonds. Figure 16C is another zoom showing residue 27 in the vicinity of residue 37.

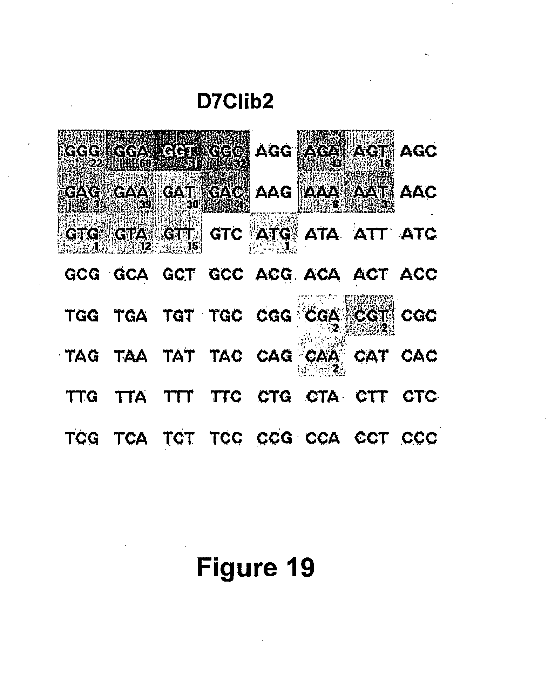

Figure 19: Hit map of the D7Clib2 library against the 64 DC7NNN targets. The number below each cleaved target is the number of DmoCre2 mutants with different sequences cleaving this target. For each target, the grey level is proportional to the mean of cleavage intensity. Each target represented in the hit map refers to the complementary strands of C12D34 (SEQ ID NO: 31); for example, GGA in the hit map corresponds to the DC7TCC as defined in example 9.

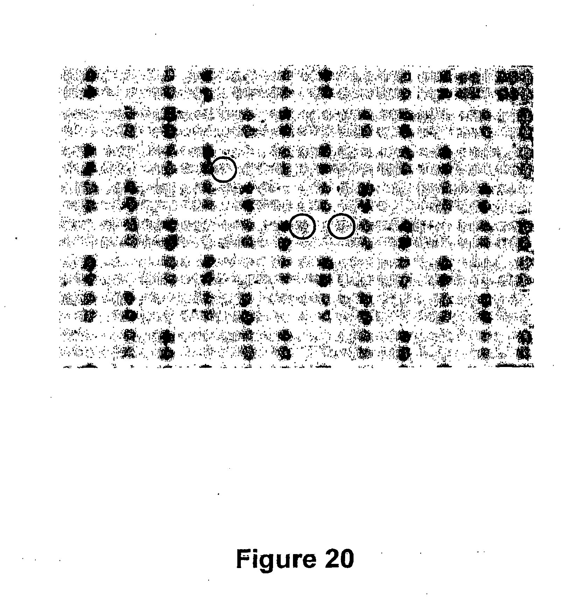

Figure 20: The figure displays an example of primary screening of *DmoCre2* mutants from the SeqDC10NNN4ACT library against the combined DC10TGG4ACT target. In each yeast cluster, the two right dots are experiment internal controls. For the other four dots, one dot corresponds to one mutant from the SeqDC10NNN4ACT library. Three positives clones are black circled.

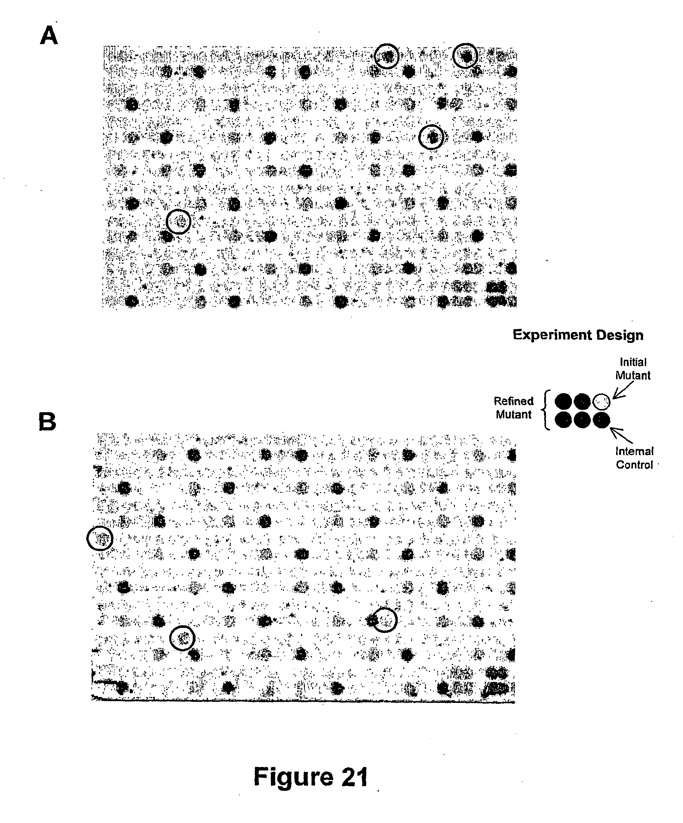

Figure 21: The figure displays an example of primary screening of mutants from the RAG1.10.3DC4NNN library against the RAG1.10.3DC4ACT target (A) and the RAG1.10.3DC4TAT target (B). In each yeast cluster, the top right dot corresponds to the Amel2-RG3D mutant and the down right dot is experiment internal control. For the other four dots, one dot corresponds to one mutant from the RAG1.10.3DC4NNN library. Some positive clones are black circled.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Definitions

- Amino acid residues in a polypeptide sequence are designated herein according to the one-letter code, in which, for example, Q means Gln or Glutamine residue, R means Arg or Arginine residue and D means Asp or Aspartic acid residue.
- hydrophobic amino acid refers to leucine (L), valine (V), isoleucine (I), alanine (A), methionine (M), phenylalanine (F), tryptophane (W) and tyrosine (Y).
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "meganuclease" is intended an endonuclease having a double-stranded DNA target sequence of 12 to 45 pb.
- by "parent LAGLIDADG homing endonuclease" is intended a wild-type LAGLIDADG homing endonuclease or a functional variant thereof. Said parent LAGLIDADG homing endonuclease may be a monomer, a dimer (homodimer or heterodimer) comprising two LAGLIDADG homing endonuclease core domains which are associated in a functional endonuclease able to cleave a double-stranded DNA target of 22 to 24 bp.
- by "homodimeric LAGLIDADG homing endonuclease" is intended a wild-type homodimeric LAGLIDADG homing endonuclease having a single LAGLIDADG motif and cleaving palindromic DNA target sequences, such as *I-CreI* or I*-MsoI* or a functional variant thereof.
- by "LAGLIDADG homing endonuclease variant" or "variant" is intended a protein obtained by replacing at least one amino acid of a LAGLIDADG homing endonuclease sequence, with a different amino acid.
- by "functional variant" is intended a LAGLIDADG homing endonuclease variant which is able to cleave a DNA target, preferably a new DNA target which is not cleaved by a wild type LAGLIDADG homing endonuclease. For example, such variants have amino acid variation at positions contacting the DNA target sequence or interacting directly or indirectly with said DNA target.
- by "homing endonuclease variant with novel specificity" is intended a variant having a pattern of cleaved targets (cleavage profile) different from that of the parent homing endonuclease. The variants may cleave less targets (restricted profile) or more targets than the parent homing endonuclease. Preferably, the variant is able to cleave at least one target that is not cleaved by the parent homing endonuclease.

The terms "novel specificity", "modified specificity", "novel cleavage specificity", "novel substrate specificity" which are equivalent and used indifferently, refer to the specificity of the variant towards the nucleotides of the DNA target sequence.
- by *"I-CreI"* is intended the wild-type *I-CreI* having the sequence SWISSPROT P05725 or pdb accession code 1g9y (SEQ ID NO:24).
- by *"I-DmoI"* is intended the wild-type *I-DmoI* having the sequence SWISSPROT number P21505 (SEQ ID NO: 22) or the structure PDB code 1b24
- by "domain" or "core domain" is intended the "LAGLIDADG homing endonuclease core domain" which is the characteristic αββαββα fold of the homing endonucleases of the LAGLIDADG family, corresponding to a sequence of about one hundred amino acid residues. Said domain comprises four beta-strands folded in an antiparallel beta-sheet which interacts with one half of the DNA target. This domain is able to associate with another LAGLIDADG homing endonuclease core domain which interacts with the other half of the DNA target to form a functional endonuclease able to cleave said DNA target. For example, in the case of the dimeric homing endonuclease *I-CreI* (163 amino acids), the LAGLIDADG homing endonuclease core domain corresponds to the residues 6 to 94. In the case of monomeric homing endonucleases, two such domains are found in the sequence of the endonuclease; for example in *I-DmoI* (194 amino acids), the first domain (at least residues 1 to 95 and the second domain (residues 105 to 194) are separated by a linker (residues 96 to 104).
- by "subdomain" is intended the region of a LAGLIDADG homing endonuclease core domain which interacts with a distinct part of a homing endonuclease DNA target half-site.
- by "beta-hairpin" is intended two consecutive beta-strands of the antiparallel beta-sheet of a LAGLIDADG homing endonuclease core domain which are connected by a loop or a turn,
- by "C1221" it is intended to refer to the first half of the *I-CreI* target site '12' repeated backwards so as to form a palindrome '21'.
- by "cleavage activity" the cleavage activity of the variant of the invention may be measured by a direct repeat recombination assay, in yeast or mammalian cells, using a reporter vector, as described in the PCT Application WO 2004/067736; Epinat et al., Nucleic Acids Res., 2003, 31, 2952-2962; Chames et al., Nucleic Acids Res., 2005, 33, e178, and Arnould et al., J. Mol. Biol., 2006, 355, 443-458. The reporter vector comprises two truncated, non-functional copies of a reporter gene (direct repeats) and a chimeric DNA target sequence within the intervening sequence, cloned in a yeast or a mammalian expression vector. The DNA target sequence is derived from the parent homing endonuclease cleavage site by replacement of at least one nucleotide by a different nucleotide. Preferably a panel of palindromic or non-palindromic DNA targets representing the different combinations of the 4 bases (g, a, c, t) at one or more positions of the DNA cleavage site is tested (4ⁿ palindromic targets for n mutated positions). Expression of the variant results in a functional endonuclease which is able to cleave the DNA target sequence. This cleavage induces homologous recombination between the direct repeats, resulting in a functional reporter gene, whose expression can be monitored by appropriate assay.
- by "DNA target", "DNA target sequence", "target sequence", "target-site", "target" , "site"; "recognition site", "recognition sequence", "homing recognition site", "homing site", "cleavage site" is intended a 22 to 24 bp double-stranded palindromic, partially palindromic (pseudo-palindromic) or non-palindromic polynucleotide sequence that is recognized and cleaved by a LAGLIDADG homing endonuclease. These terms refer to a distinct DNA location, preferably a genomic location, at which a double stranded break (cleavage) is to be induced by the endonuclease. The DNA target is defined by the 5' to 3' sequence of one strand of the double-stranded polynucleotide. For example, the palindromic DNA target sequence cleaved by wild type *I-CreI* is defined by the sequence 5'- t-₁₂c-₁₁a-₉a-₈a-₇c-₆g-₅t-ₐc-₃g-₂t-₁a+₁c+₂g+₃a+₄c+₅g+₆t+₇t+₈t+₉t+₁₀g+₁₁a+₁₂ (SEQ ID NO:29). Cleavage of the DNA target occurs at the nucleotides in positions +2 and -2, respectively for the sense and the antisense strand. Unless otherwise indicated, the position at which cleavage of the DNA target by a meganuclease variant occurs, corresponds to the cleavage site on the sense strand of the DNA target.
- by " DNA target half-site", "half cleavage site" or half-site" is intended the portion of the DNA target which is bound by each LAGLIDADG homing endonuclease core domain.
- by "DC10NNN", (SEQ ID NO: 8) it is intended that this is the target sequence of *DmoCre* with variability in positions +8, +9 and +10 of the sequence, hence ***D**mo**C**re* in position **10** variable at 3 nucleotides sequentially backwards from 10. Likewise DC4NNN (SEQ ID NO: 36) refers to the target sequence of *DmoCre2* with variability in positions +2, +3 and +4 of the sequence; and DC7NNN (SEQ ID NO: 37) refers to the target sequence of *DmoCre* with variability in positions +5, +6 and +7 of the sequence.
- by "chimeric DNA target"or "hybrid DNA target" is intended the fusion of a different half of two parent meganuclease target sequences. In addition at least one half of said target may comprise the combination of nucleotides which are bound by separate subdomains (combined DNA target).
- by "mutation" is intended the substitution, the deletion, and/or the addition of one or more nucleotides/amino acids in a nucleic acid/amino acid sequence.
- by "homologous" is intended a sequence with enough identity to another one to lead to a homologous recombination between sequences, more particularly having at least 95 % identity, preferably 97 % identity and more preferably 99 %.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and ruminants (e.g., cows, pigs, horses).
- "genetic disease" refers to any disease, partially or completely, directly or indirectly, due to an abnormality in one or several genes. Said abnormality can be a mutation, an insertion or a deletion. Said mutation can be a punctual mutation. Said abnormality can affect the coding sequence of the gene or its regulatory sequence. Said abnormality can affect the structure of the genomic sequence or the structure or stability of the encoded mRNA. This genetic disease can be recessive or dominant. Such genetic disease could be, but are not limited to, cystic fibrosis, Huntington's chorea, familial hyperchoiesterolemia (LDL receptor defect), hepatoblastoma, Wilson's disease, congenital hepatic porphyrias, inherited disorders of hepatic metabolism, Lesch Nyhan syndrome, sickle cell anemia, thalassaemias, xeroderma pigmentosum, Fanconi's anemia, retinitis pigmentosa, ataxia telangiectasia, Bloom's syndrome, retinoblastoma, Duchenne's muscular dystrophy, and Tay-Sachs disease.
- by "RAG gene" is intended the RAG1 or RAG2 gene of a mammal. For example, the human RAG genes are available in the NCBI database, under the accession number NC_000011.8: the RAG1 (GeneID:5896) and RAG2 (GeneID:5897) sequences are situated from positions 36546139 to 36557877 and 36570071 to 36576362 (minus strand), respectively. Both genes have a short untranslated exon 1 and an exon 2 comprising the ORF coding for the RAG protein, flanked by a short and a long untranslated region, respectively at its 5' and 3' ends
- "RAG1.10" is a 22 bp (non-palindromic) target located at position 5270 of the human RAG1 gene (accession number NC_000011.8, positions 836546139 to 36557877), 7 bp upstream from the coding exon of RAG1.
- "RAG1.10.2" (SEQ ID NO: 34) is a palindromic target (tgttctcagg tacctgagaaca) derived from the first half of the RAG1.10 target
- "RAG1.10.2D34" : by "RAG1.10.2D34" (SEQ ID NO:35) it is meant a sequence comprising the first portion of the RAG1.10.2 target sequence as defined above joined to the second half of the *I-DmoI* target sequence designated D34. The sequence is "tgttctcagg taagttccggcg".
- "RAG1.10.3" (SEQ ID NO: 38) is a palindromic target (ctggctgaggtacctcagccag) derived from the first half of the RAG1.10 target
- "RAG1.10.3D34" : by "RAG1.10.3D34" (SEQ ID NO:39) it is meant a sequence comprising the first portion of the RAG1.10.3 target sequence as defined above joined to the second half of the *I-DmoI* target sequence designated D34. The sequence is "ttggctgaggtaagttccggcg".
- "vectors": a vector which can be used in the present invention includes, but is not limited to, a viral vector, a plasmid, a RNA vector or a linear or circular DNA or RNA molecule which may consists of a chromosomal, non chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), para-myxovirus (e. g. measles and Sendai), positive strand RNA viruses such as picor-navirus and alphavirus, and double-stranded DNA viruses including adenovirus, herpesvirus (e. g., Herpes Simplex virus types 1 and 2, Epstein-Barr virus, cytomega-lovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996). The term "vector" refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors. A vector according to the present invention comprises, but is not limited to, a YAC (yeast artificial chromosome), a BAC (bacterial artificial), a baculovirus vector, a phage, a phagemid, a cosmid, a viral vector, a plasmid, a RNA vector or a linear or circular DNA or RNA molecule which may consist of chromosomal, non chromosomal, semi-synthetic or synthetic DNA. In general, expression vectors of utility in recombinant DNA techniques are often in the form of "plasmids" which refer generally to circular double stranded DNA loops which, in their vector form are not bound to the chromosome. Large numbers of suitable vectors are known to those of skill in the art.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for *S*. *cerevisiae;* tetracycline, rifampicin or ampicillin resistance in *E. coli*.

Preferably said vectors are expression vectors, wherein a sequence encoding a polypeptide of the invention is placed under control of appropriate transcriptional and translational control elements to permit production or synthesis of said protein. Therefore, said polynucleotide is comprised in an expression cassette. More particularly, the vector comprises a replication origin, a promoter operatively linked to said encoding polynucleotide, a ribosome site, an RNA-splicing site (when genomic DNA is used), a polyadenylation site and a transcription termination site. It also can comprise an enhancer. Selection of the promoter will depend upon the cell in which the polypeptide is expressed.

### EXAMPLE 1: Improvement of DmoCre with increased activity

The inventors set out to improve the existing *DmoCre* scaffold by increasing the overall activity of this enzyme. In particular three mutations were introduced into the *I-DmoI* N-terminal α-helix of *DmoCre* corresponding to residues 15, 19 and 20 of *I-DmoI* (SEQ ID NO: 22).

The G20S mutation leads to a more active *DmoCre* protein in yeast, whereas the two mutations L15Q and I19D, render the protein active in CHO cells as shown by an extrachromosomal SSA (Single Strand Annealing) recombination assay previously described (Arnould et al., Mol Biol. 2006 Jan 20;355(3):443-58). Hence, the final *DmoCre* scaffold that was used in the current experiments harbors the L15Q, I19D and G20S mutations, which are all localized in the *I-DmoI* N-terminal LAGLIDADG α-helix; said wild-type *I-DmoI* domain is provided as SEQ ID NO:1.

This scaffold is referred to as *DmoCre2* and was used in further experiments. The peptide sequence of *DmoCre2* is provided as SEQ ID NO: 2.

### EXAMPLE 2: Making of DmoCre2 derived mutants cleaving degenerated DC10NNN_P targets

To study the possibility of engineering new sequence specificities for the *DmoCre2* protein, the inventors investigated the three adjacent nucleotides at position +8 to +10 of the C12D34 DNA target The structure displayed in Figure 2 allowed the inventors to examine closely the contacts between these three base pairs and the *DmoCre2* protein residues.

Figure 3A shows the molecular surface of the hybrid enzyme bound to its DNA target. The area of binding that has been chosen for randomization (base pairs at positions +8, +9, +10 and protein residues corresponding to residues 29, 33 and 35 of SEQ ID NO: 22) has been highlighted. Figure 3B is a zoomed in view showing residues 29, 33 and 35 in interaction with the DNA target. Dashed lines represent hydrogen bonds. Using this analysis therefore the inventors pinpointed three *DmoCre2* residues: R33 and E35 that are in contact with the DNA and Y29, which is close to the DNA and appears to interact with E35.

In order to isolate new cleavage specificities for the *DmoCre2* protein, a *DmoCre2* mutant library mutated at positions 29, 33 and 35 (DClib2) was built, transformed into yeast and screened using a yeast screening assay, see below, against the 64 targets degenerated at position +8 to +10 that the applicants called DC10NNN (SEQ ID NO: 8). The DC10NNN target is 5' CAAAACGTCGTAAGTTCCNNNC 3' (SEQ ID NO 8), wherein NNN represent positions +8 to +10 and all combinations of A, C, G and T in these positions make up the 64 target DC10NNN sequences.

### Material and Methods

### Construction of the 64 target vectors:

The targets were cloned as follows: oligonucleotides corresponding to each of the 64 target sequences flanked by gateway cloning sequence were ordered from Proligo:

5'TGGCATACAAGTTTTCNNNGGAACTTACGACGTTTTGAC AATCGTCTGTCA 3' (SEQ ID NO: 3). Double-stranded target DNA, generated by PCR amplification of the single stranded oligonucleotide, was cloned using the Gateway protocol (Invitrogen) into yeast reporter vector (pCLS1055, Figure 4). The yeast reporter vector was transformed into *S*. *cerevisiae* strain FYBL2-7B (MAT-α, ura3 Δ 851, trp1 Δ 63, leu2 Δ 1, lys2 Δ 202).

### Construction of the DmoCre2 DClib2 mutant library:

In order to generate *DmoCre2* derived coding sequences containing mutations at positions 29, 33 or 35, two separate overlapping PCR reactions were carried out that amplify the 5' end (aa positions 1-43) or the 3' end (positions 36-264) of the *DmoCre* coding sequence. For the 3' end, PCR amplification is carried out using a primer specific to the vector (pCLS0542, Figure 5) (Gal10R 5'-ACAACCTTGATTGGAGACTTGACC-3' (SEQ ID NO: 4)) and a primer specific to the *DmoCre* coding sequence for amino acids 36-46 (Dmo10CreFor 5'-TATCGTGTTGTGATCACCCAGAAGTCTGAAAAC-3' (SEQ ID NO: 5)). For the 5' end, PCR amplification is carried out using a primer specific to the vector pCLS0542 (Gal10F 5'-GCAACTTTAGTGCTGACACATACAGG-3' (SEQ ID NO: 6)) and a primer specific to the *DmoCre* coding sequence for amino acids 23-43 (Dmo10CreRev 5'-CTTCTGGGTGATCACAACACGATAMNNGCTMNNGTT ACCTTTMNNTT TCAGCTTGTACAGGCC-3' (SEQ ID NO:7)).

The MNN code in the oligonucleotide resulting in a NNK codon at positions 29, 33 and 35 allows the degeneracy at these positions among the 20 possible amino acids. Then, 25 ng of each of the two overlapping PCR fragments and 75 ng of vector DNA (pCLS0542) linearized by digestion with NcoI and EagI were used to transform the yeast *Saccharomyces cerevisiae* strain strain FYC2-6A (MAT-α, trp1Δ63, leu2Δ1, his3Δ200) using a high efficiency LiAc transformation protocol (Gietz et al., Methods Enzymol. 2002; 350:87-96). An intact coding sequence containing both groups of mutations is generated by *in vivo* homologous recombination in yeast. The DClib2 nucleic diversity is 323 = 32768, after transformation, 2232 clones were picked, representing about 7% of the library diversity.

### Mating of meganuclease expressing clones and screening in yeast:

Screening was performed as described previously (Arnould et al., J Mol Biol. 2006; 355:443-58). Specifically, mating was performed using a colony gridder (QpixII, Genetix). Mutants were gridded on nylon filters covering YPD plates, using a low gridding density (about 4 spots/cm2). A second gridding process was performed on the same filters to spot a second layer consisting of different reporter-harboring yeast strains for each target. Membranes were placed on solid agar YPD rich medium, and incubated at 30°C for one night, to allow mating. Next, filters were transferred to synthetic medium, lacking leucine and tryptophan, with galactose (2%) as a carbon source, and incubated for five days at 37°C, to select for diploids carrying the expression and target vectors. After 5 days, filters were placed on solid agarose medium with 0.02% X-Gal in 0.5 M sodium phosphate buffer, pH 7.0, 0.1% SDS, 6% dimethyl formamide (DMF), 7mM β-mercaptoethanol, 1% agarose, and incubated at 37°C, to monitor β-galactosidase activity. Results were analyzed by scanning and quantification was performed using proprietary software.

### Sequencing of mutants

To recover the mutant expressing plasmids, yeast DNA was extracted using standard protocols and used to transform *E*. *coli.* Sequencing of mutant ORF were then performed on the plasmids by Millegen SA. Alternatively, ORFs were amplified from yeast DNA by PCR (Akada et al., Biotechniques. 2000; 28:668-70, 672, 674), and sequencing was performed directly on PCR product by Millegen SA.

### Results

Using the yeast screening assay that has been described above, the 2232 clones that constitute the *DmoCre2* DClib2 library were screened against the 64 DC10NNN targets. The screen gave 519 positive clones able to cleave at least one DC10NNN target (SEQ ID NO: 8) (Figure 6), resulting after sequencing in 432 unique meganucleases. The initial *DmoCre2* protein is able to cleave 13 out of the 64 DC10NNN targets. The DClib2 hitmap displayed in Figure 7 shows that by introducing mutations at positions 29, 33 and 35 in the *DmoCre* coding sequence, 57 DC10NNN targets are now being cleaved by *DmoCre2* derived mutants. The current screening approach has therefore allowed the inventors to widen the *DmoCre2* cleavage spectrum for DC10NNN targets and to isolate new cleavage specificities.

With reference to Table I below the various DClib2 clones identified by the inventors are listed showing the residue changes in each of these as well as the DC 10 target sequences which they have been shown to cleave. The top most row showing the three nucleotides in positions +8 to +10 and the figures representing the intensity of the colour reaction in comparison to a negative control from yeast lacking insert. Specifically values of '0' represent an experimental result equal to the tested level of background noise in this assay. Values of '-' indicate this sample has not been tested for this particular nucleotide combination.

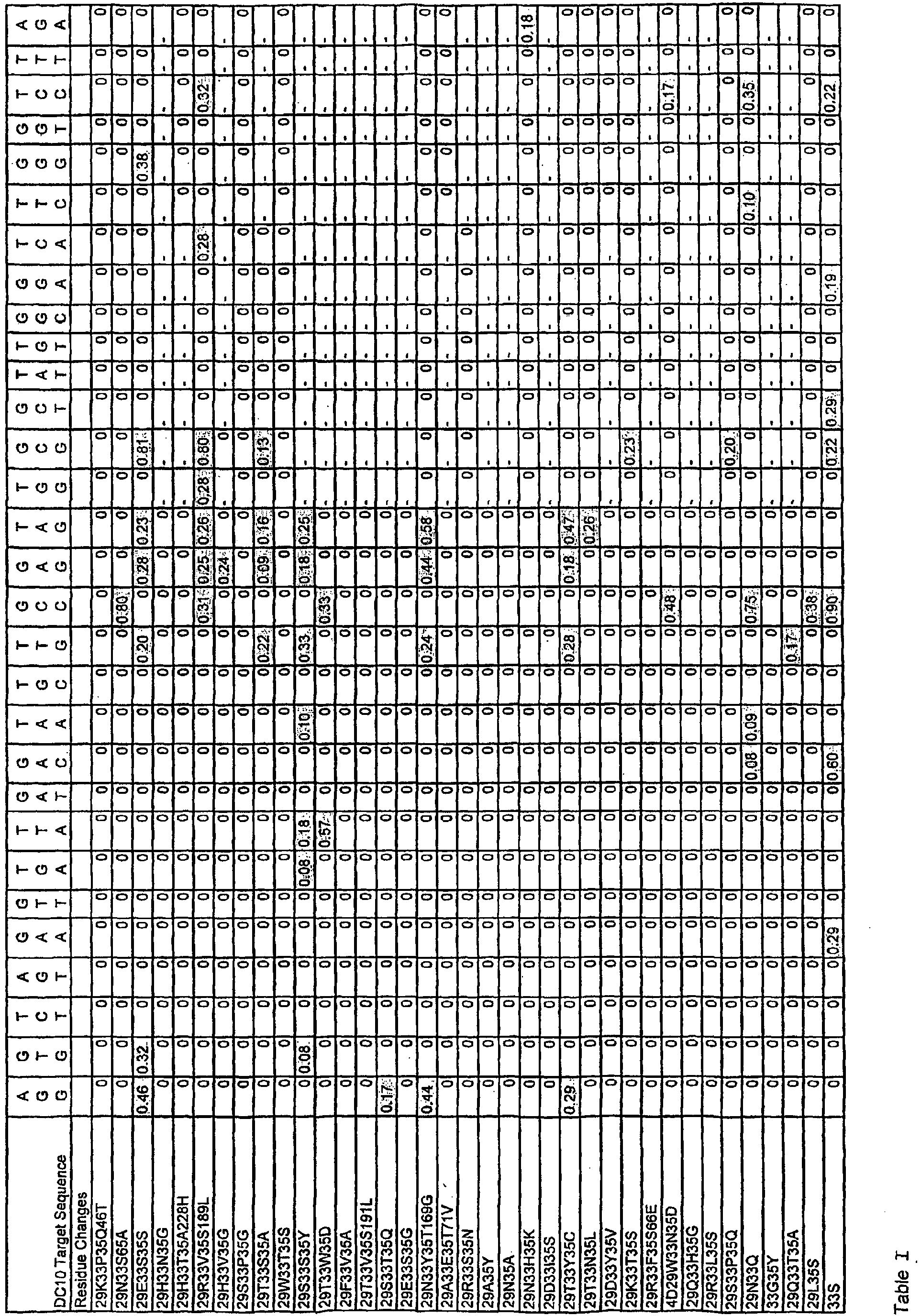

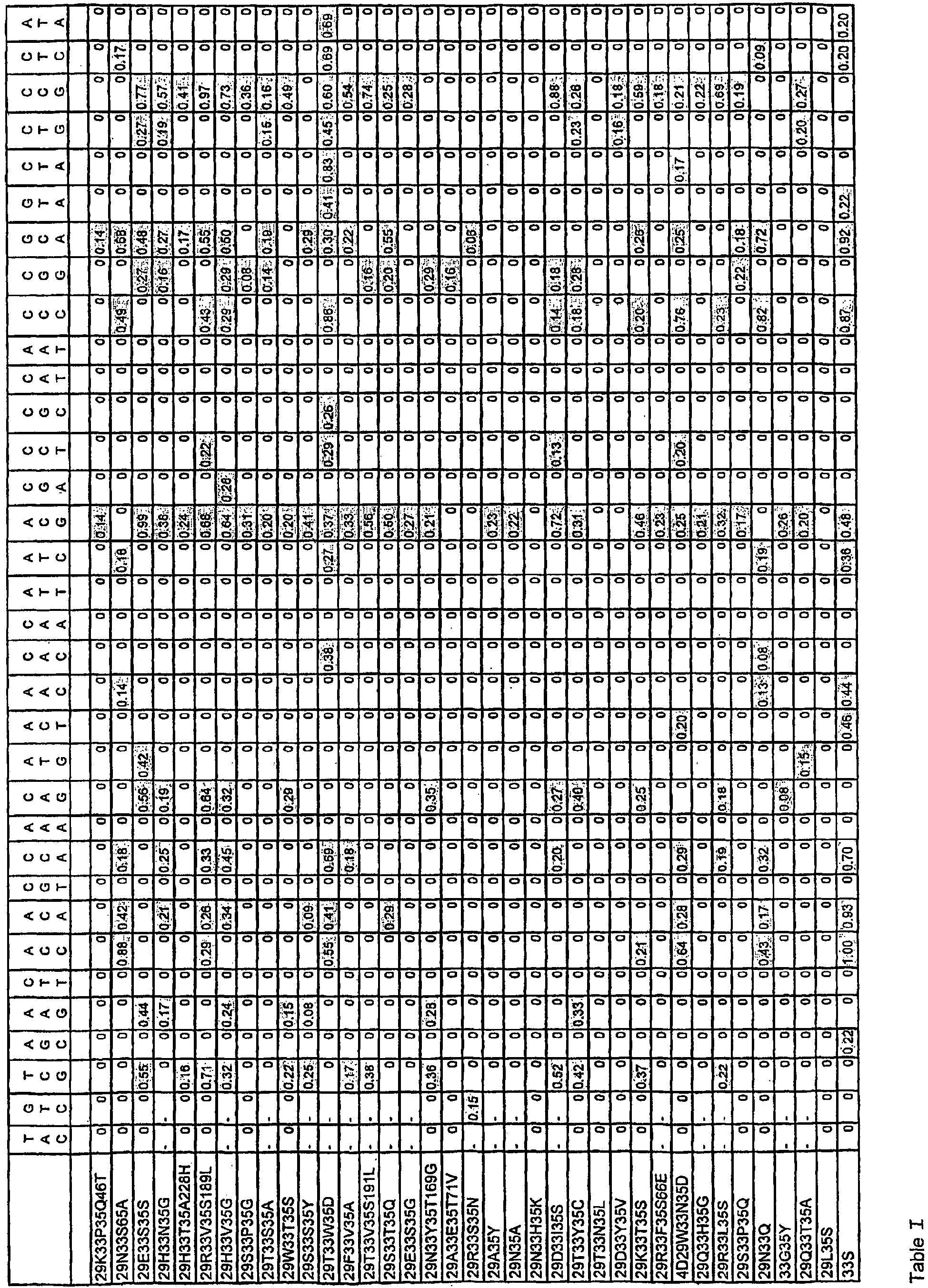

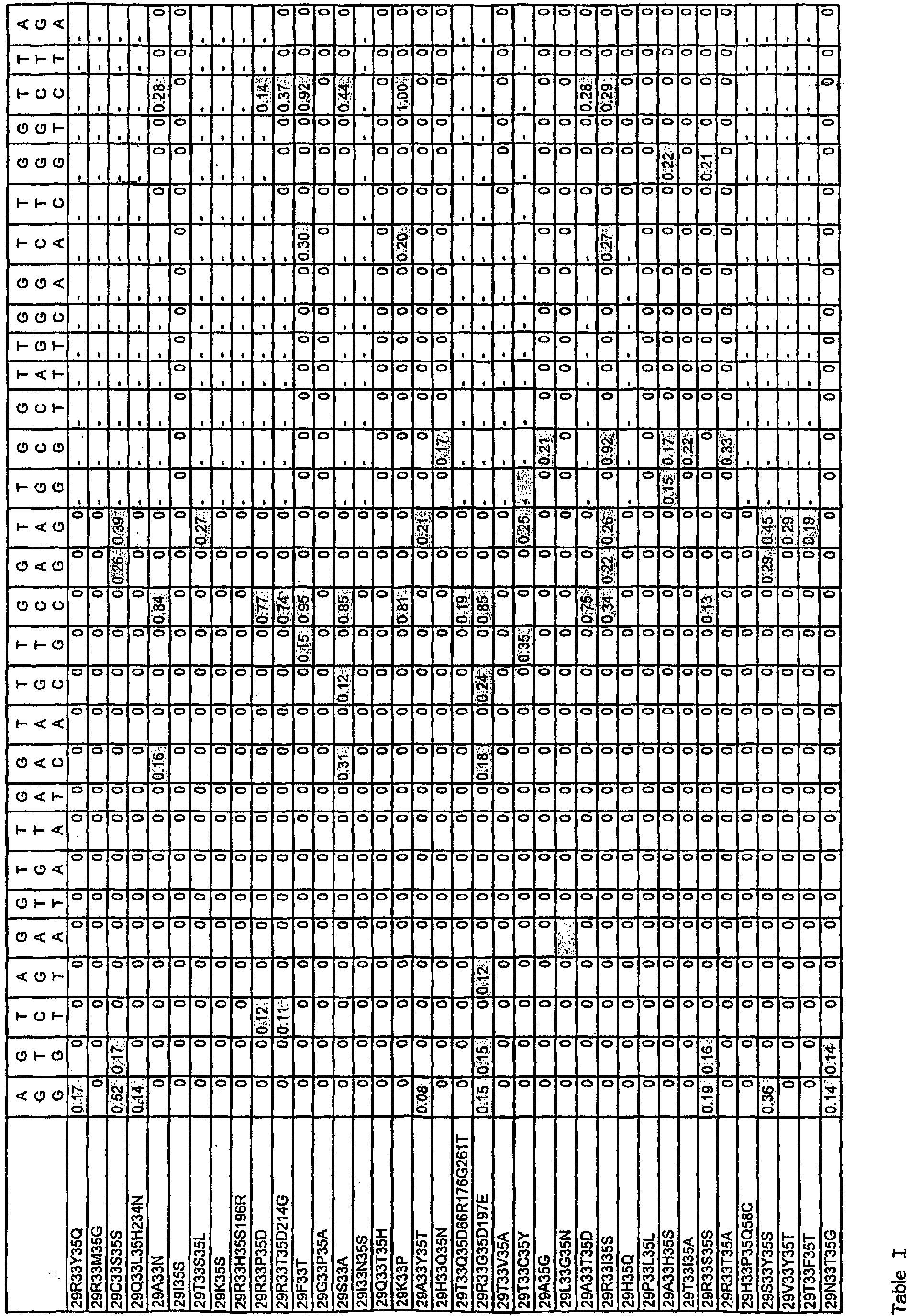

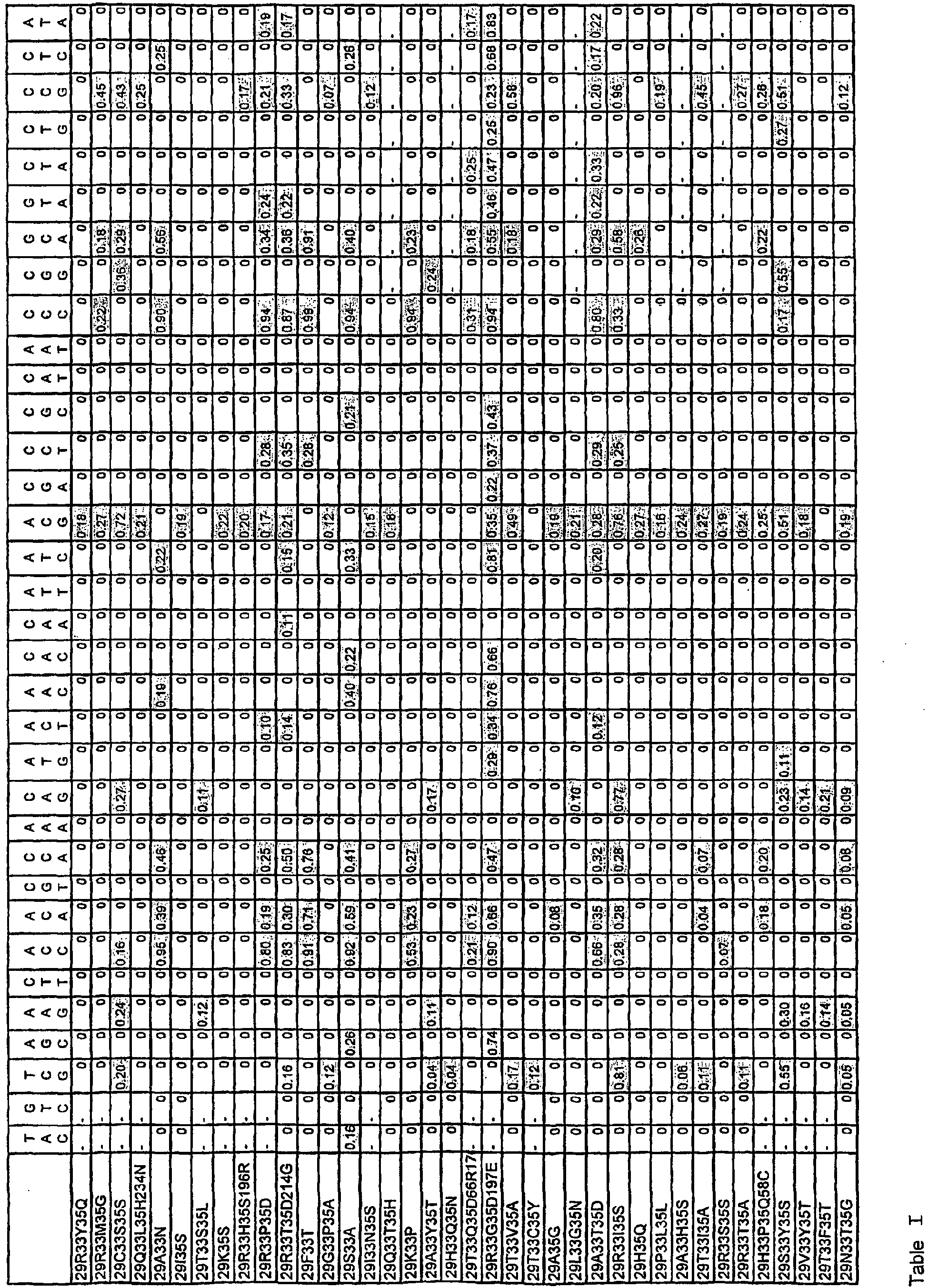

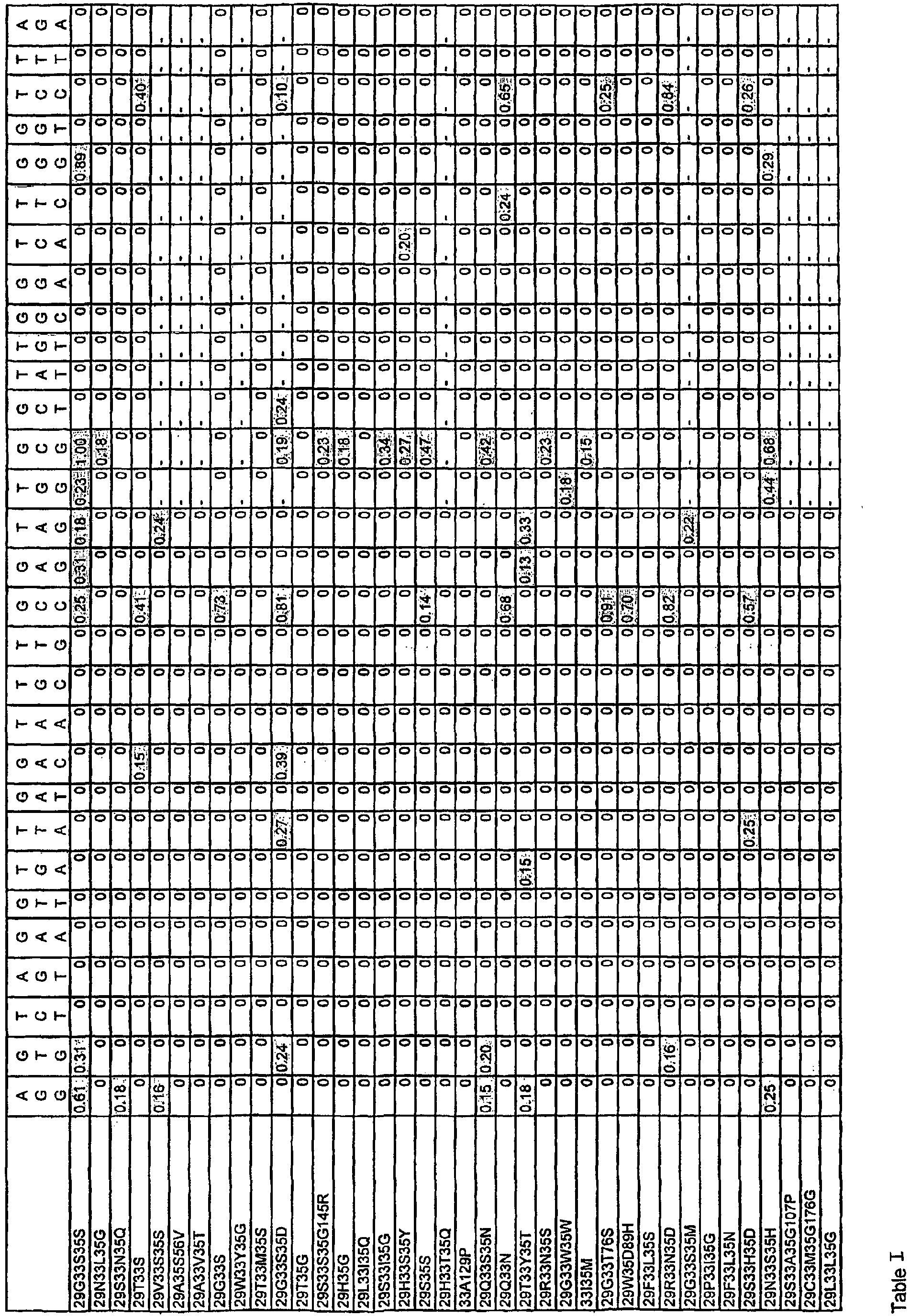

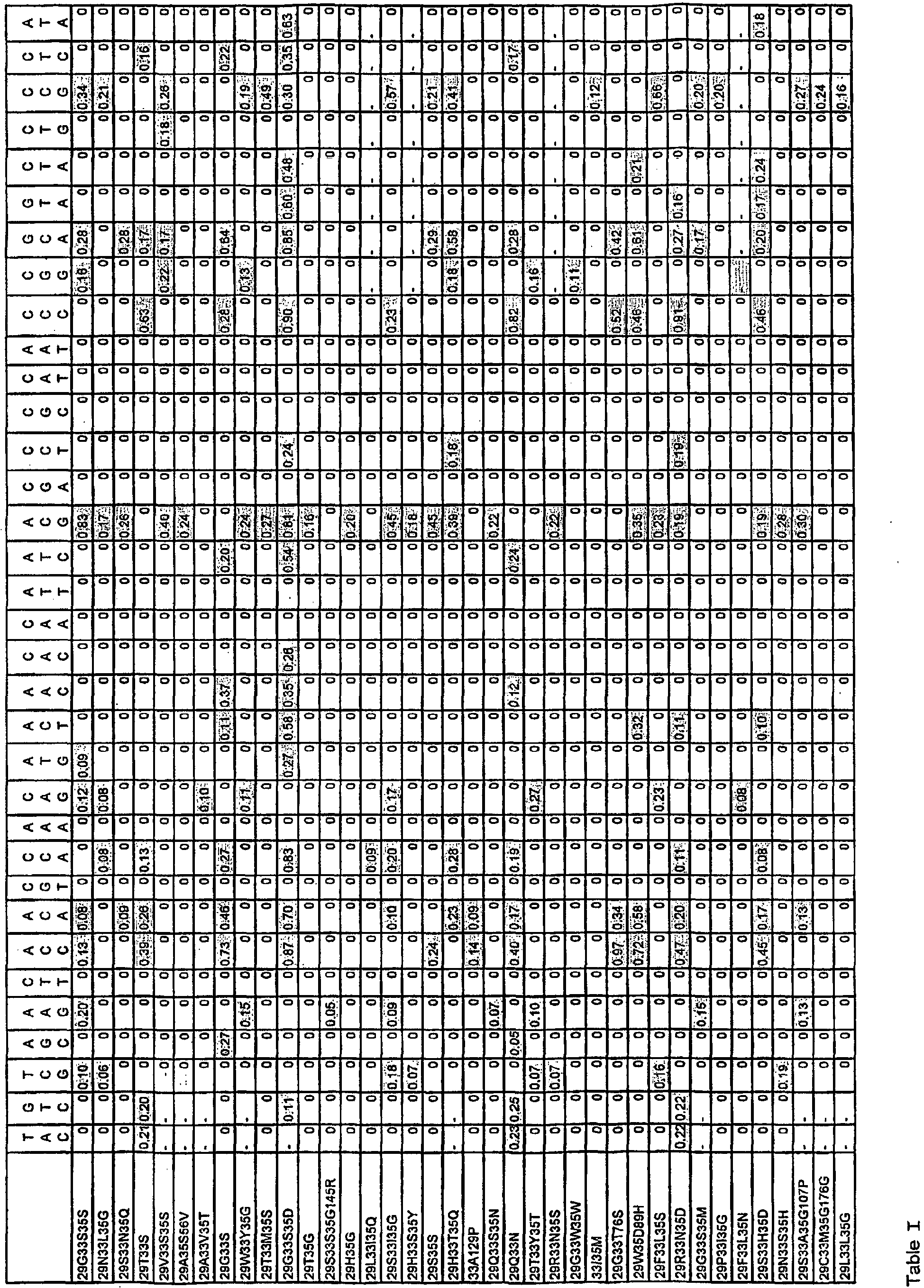

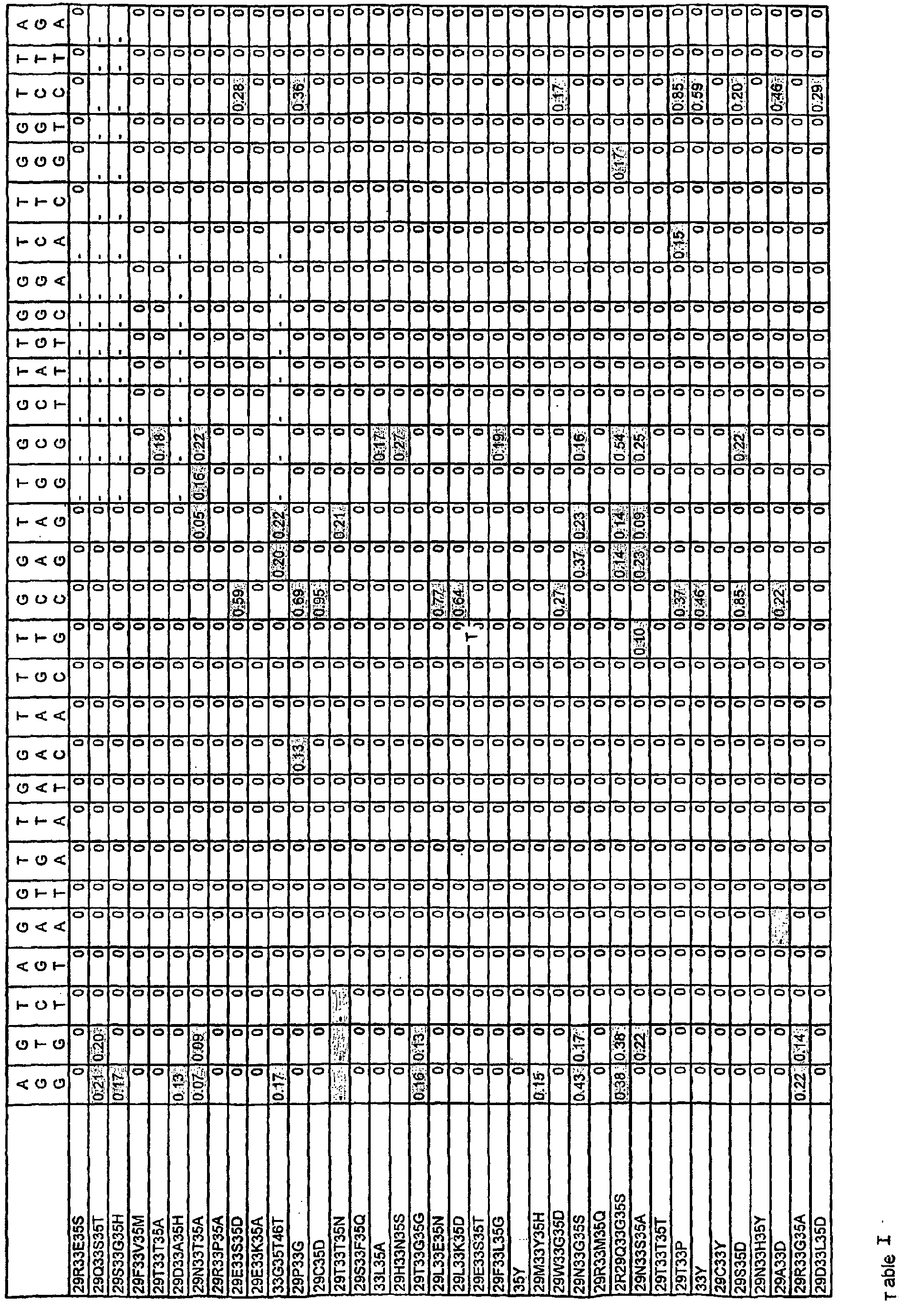

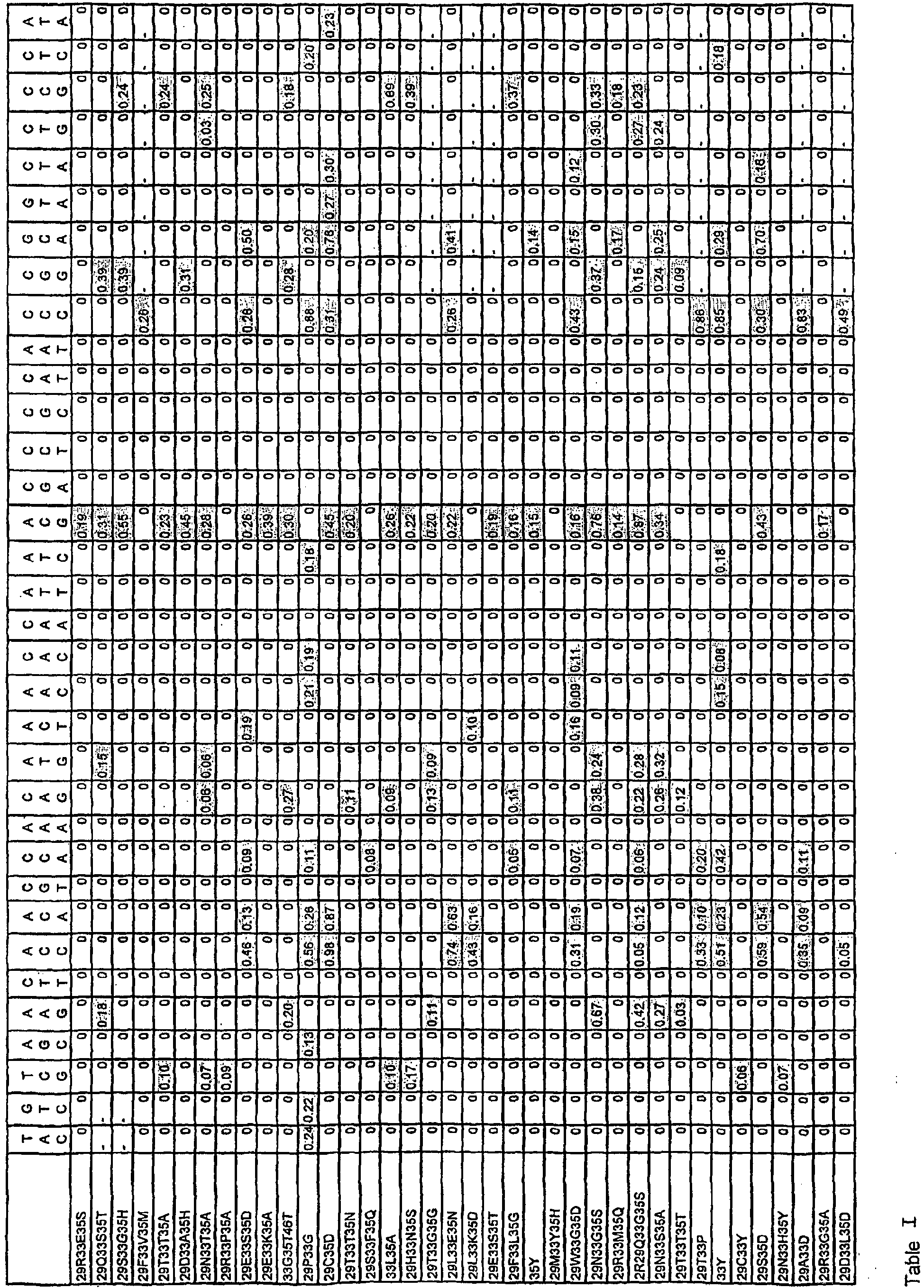

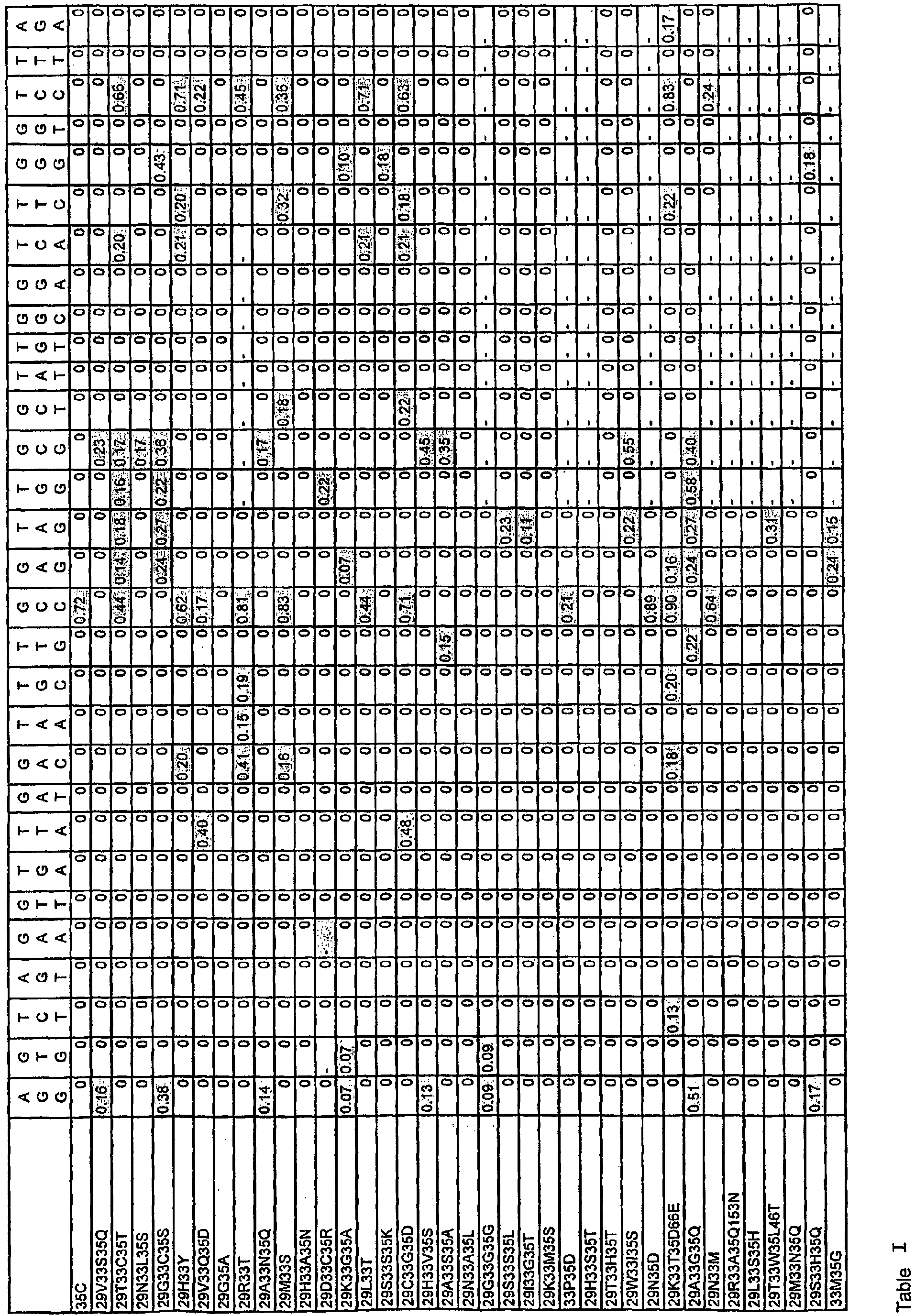

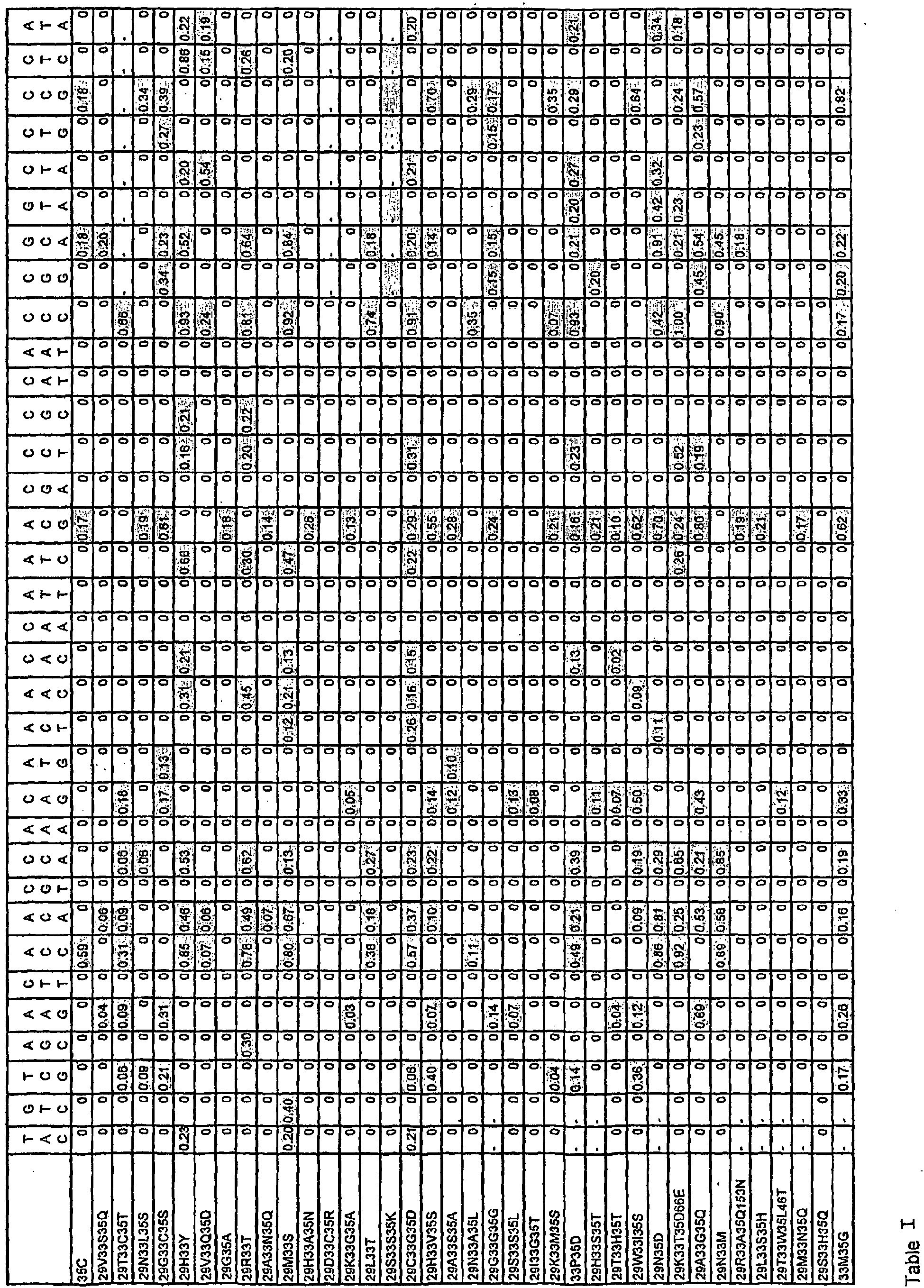

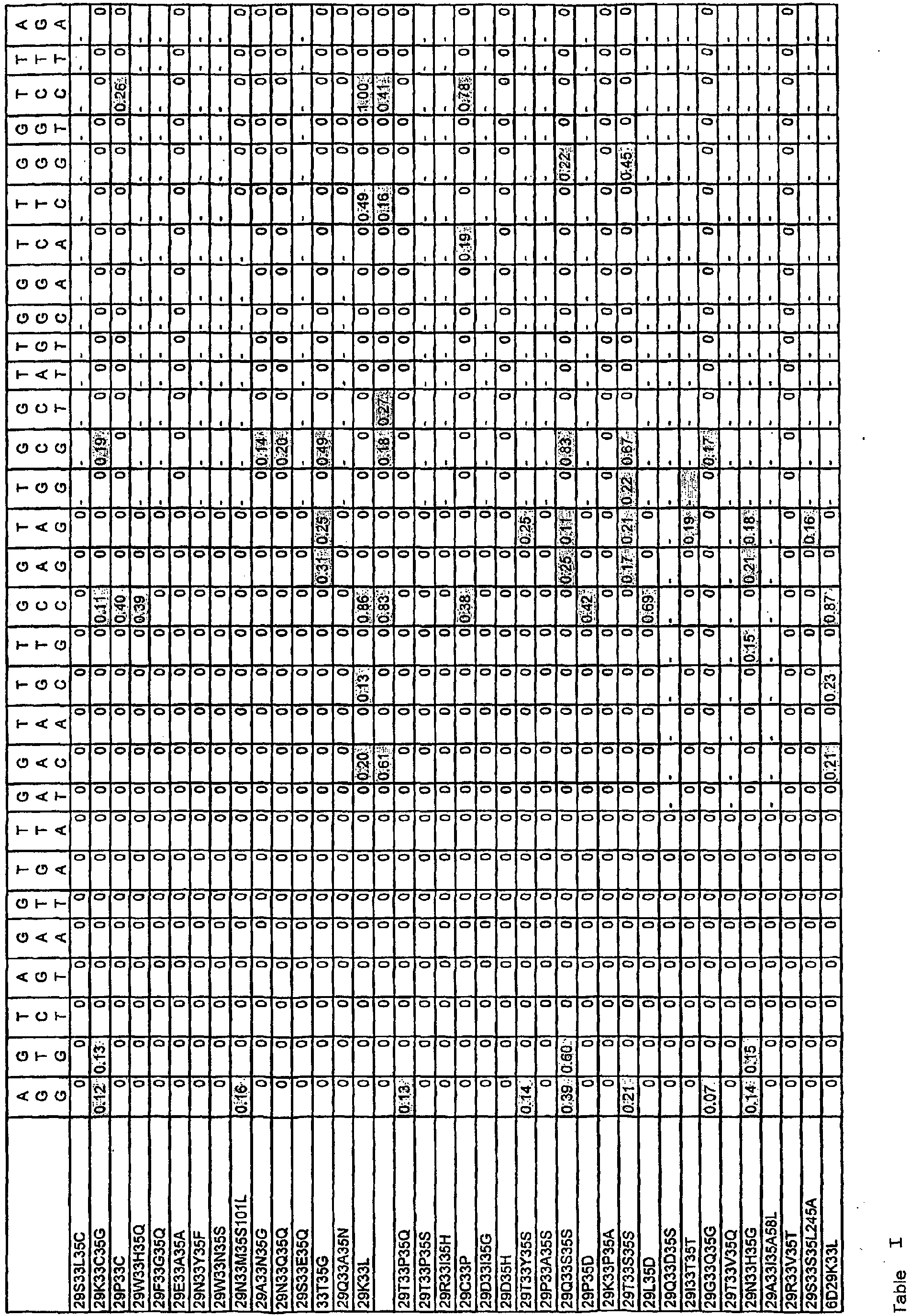

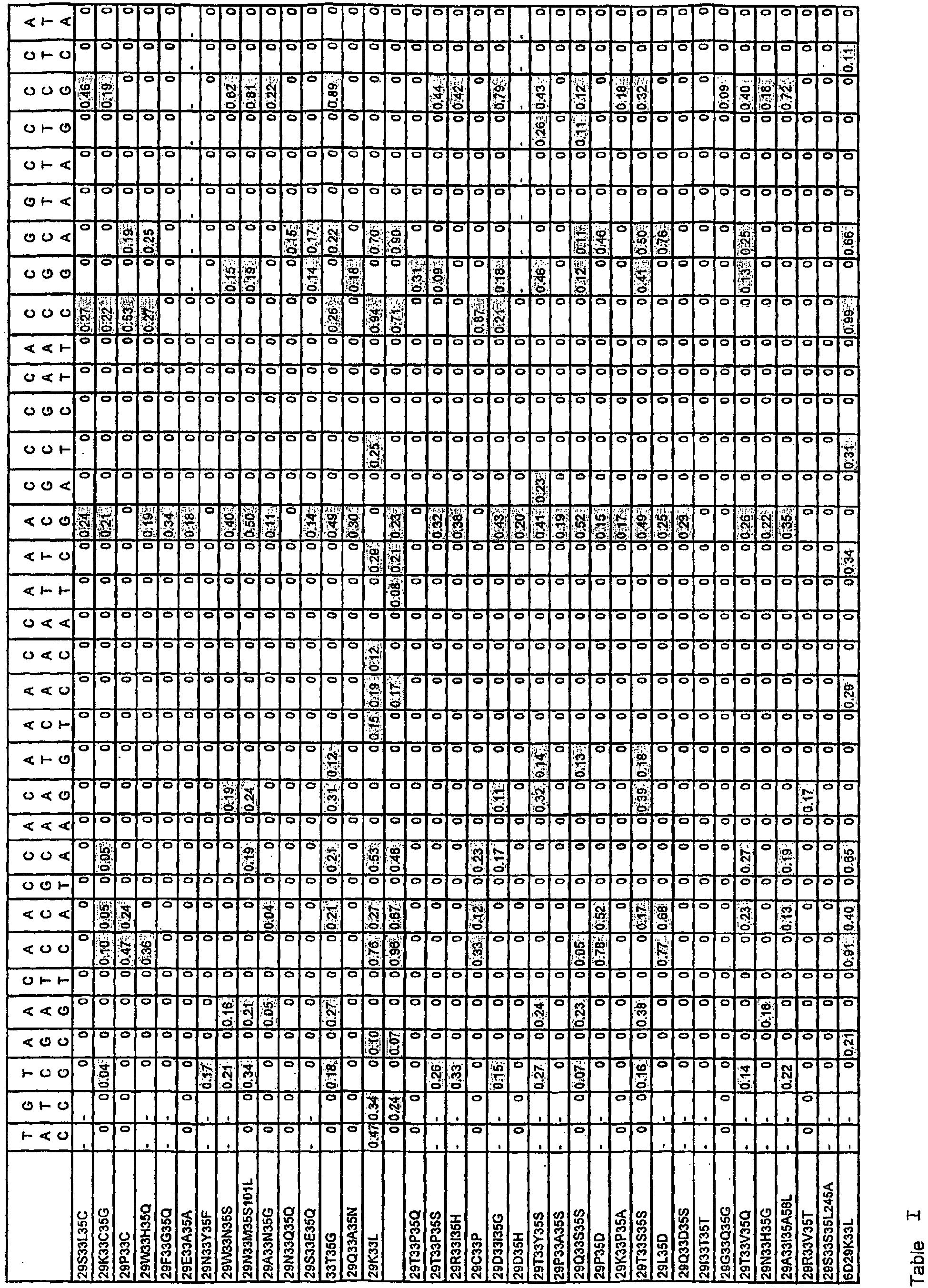

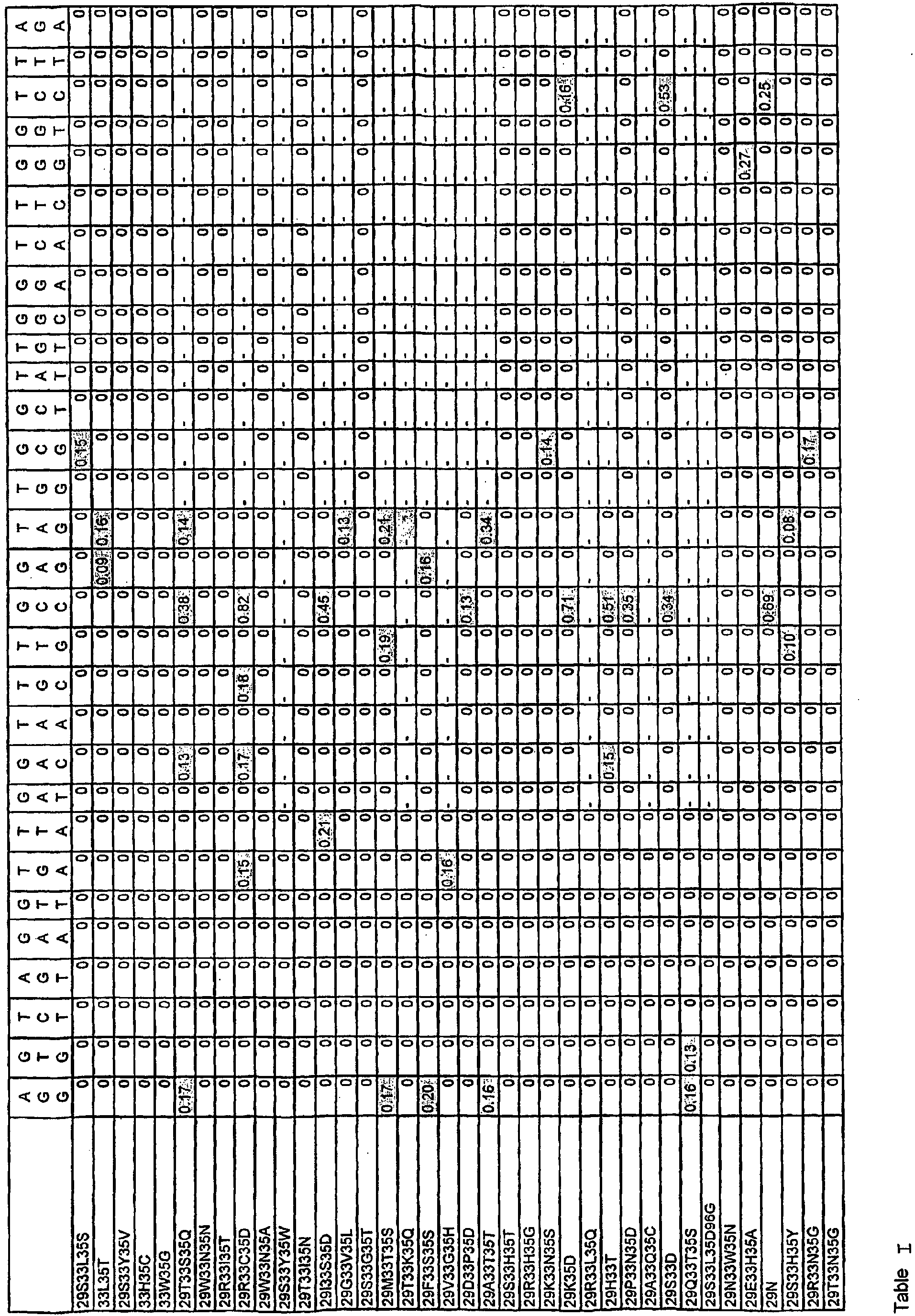

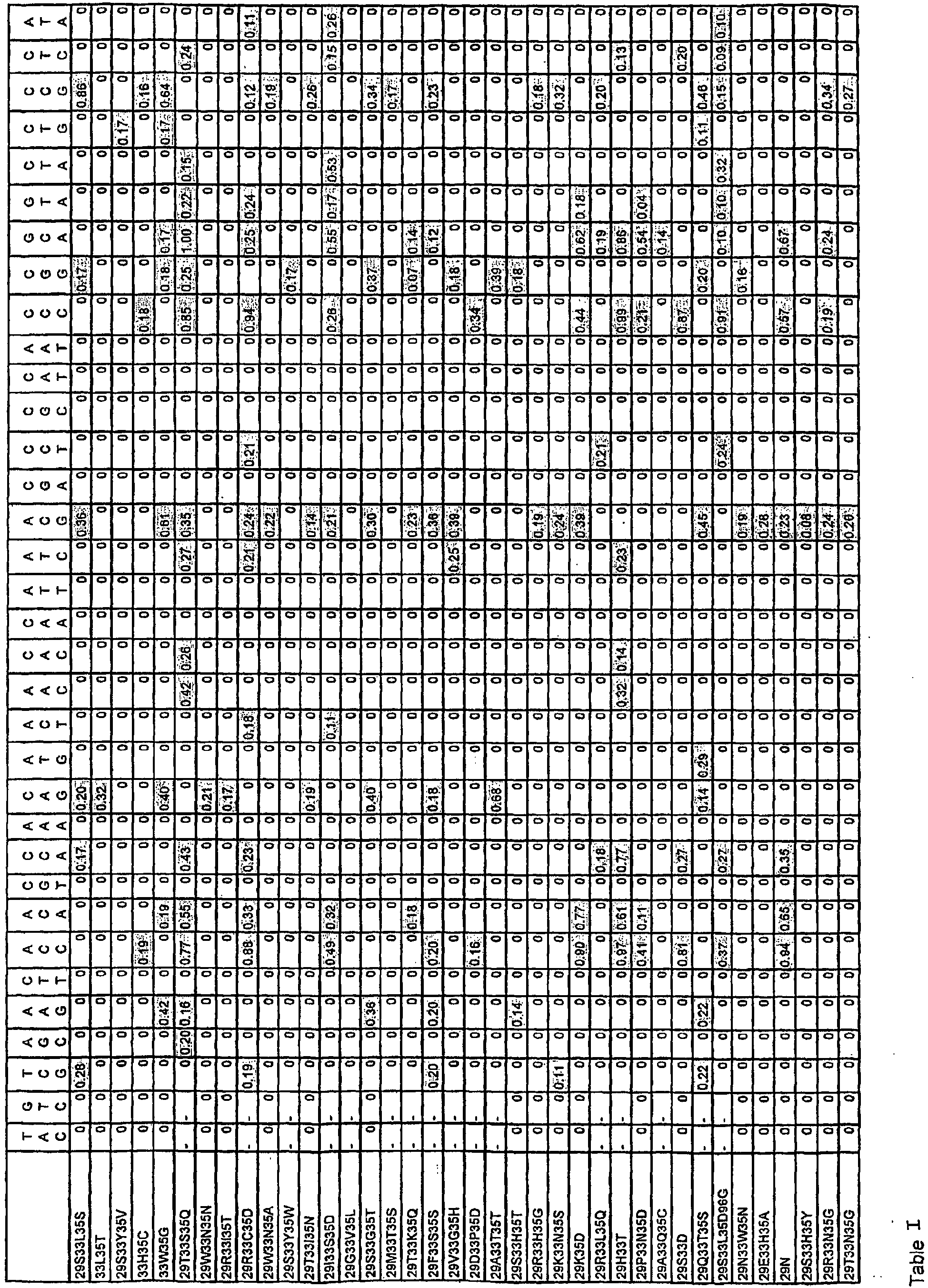

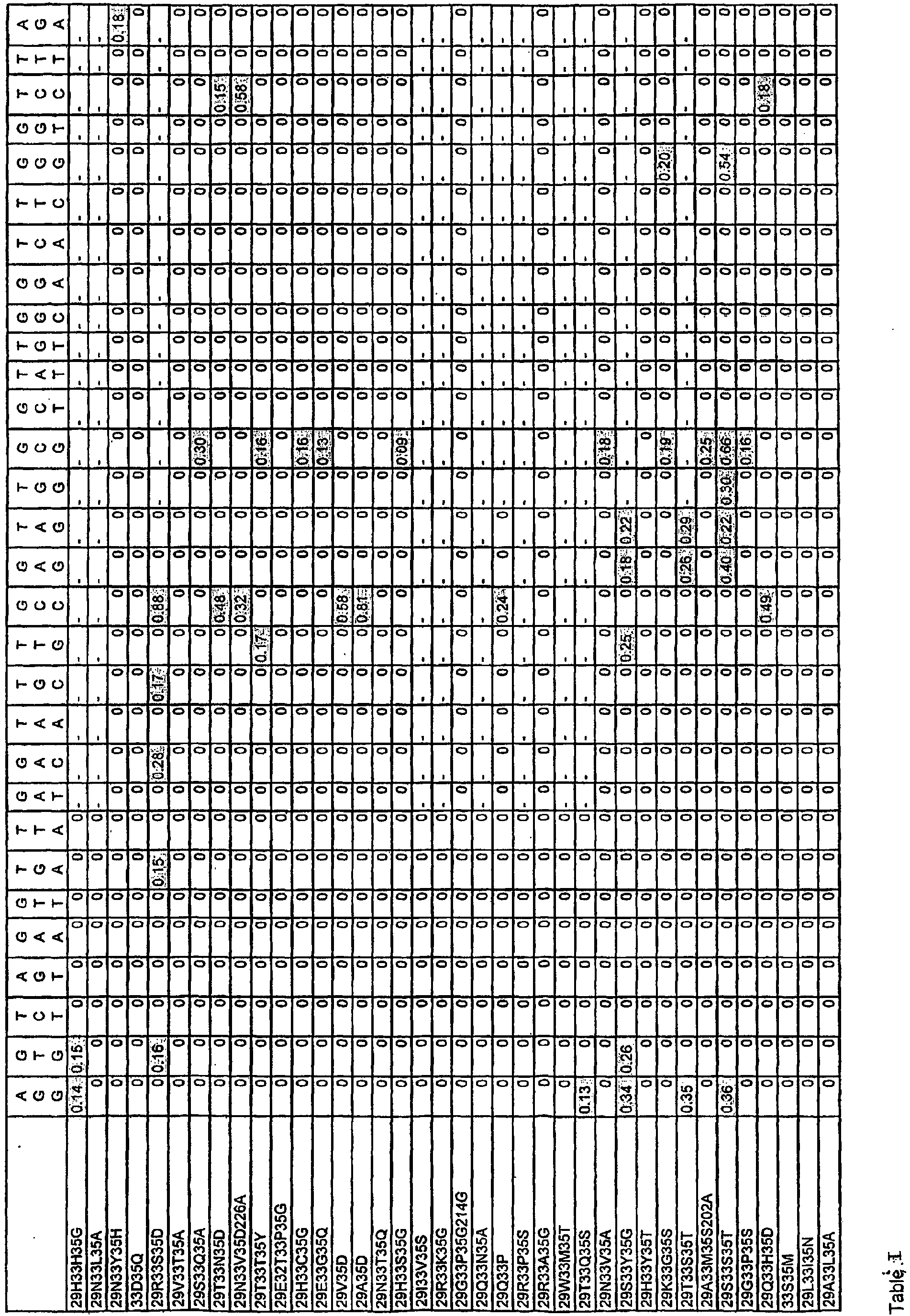

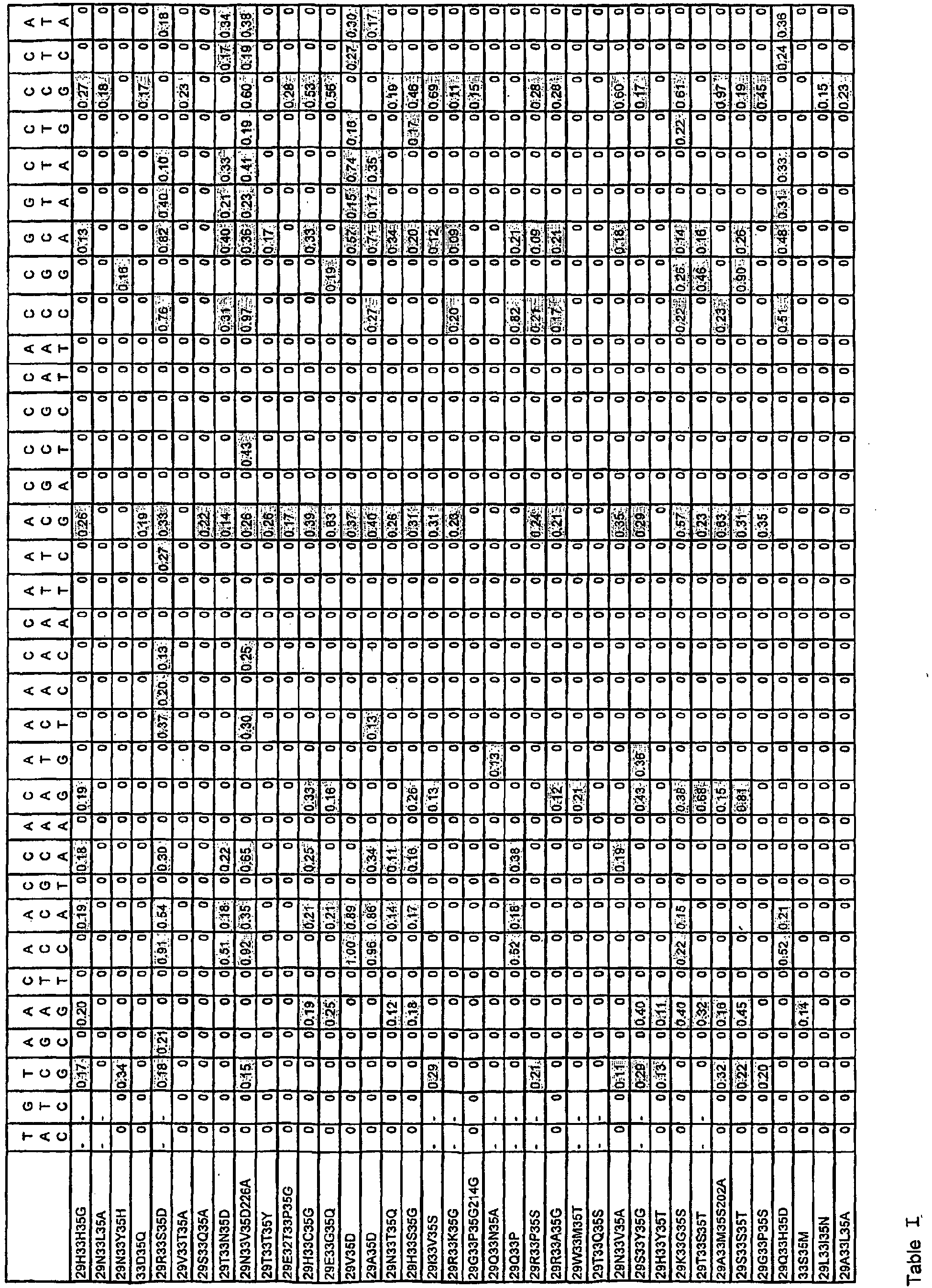

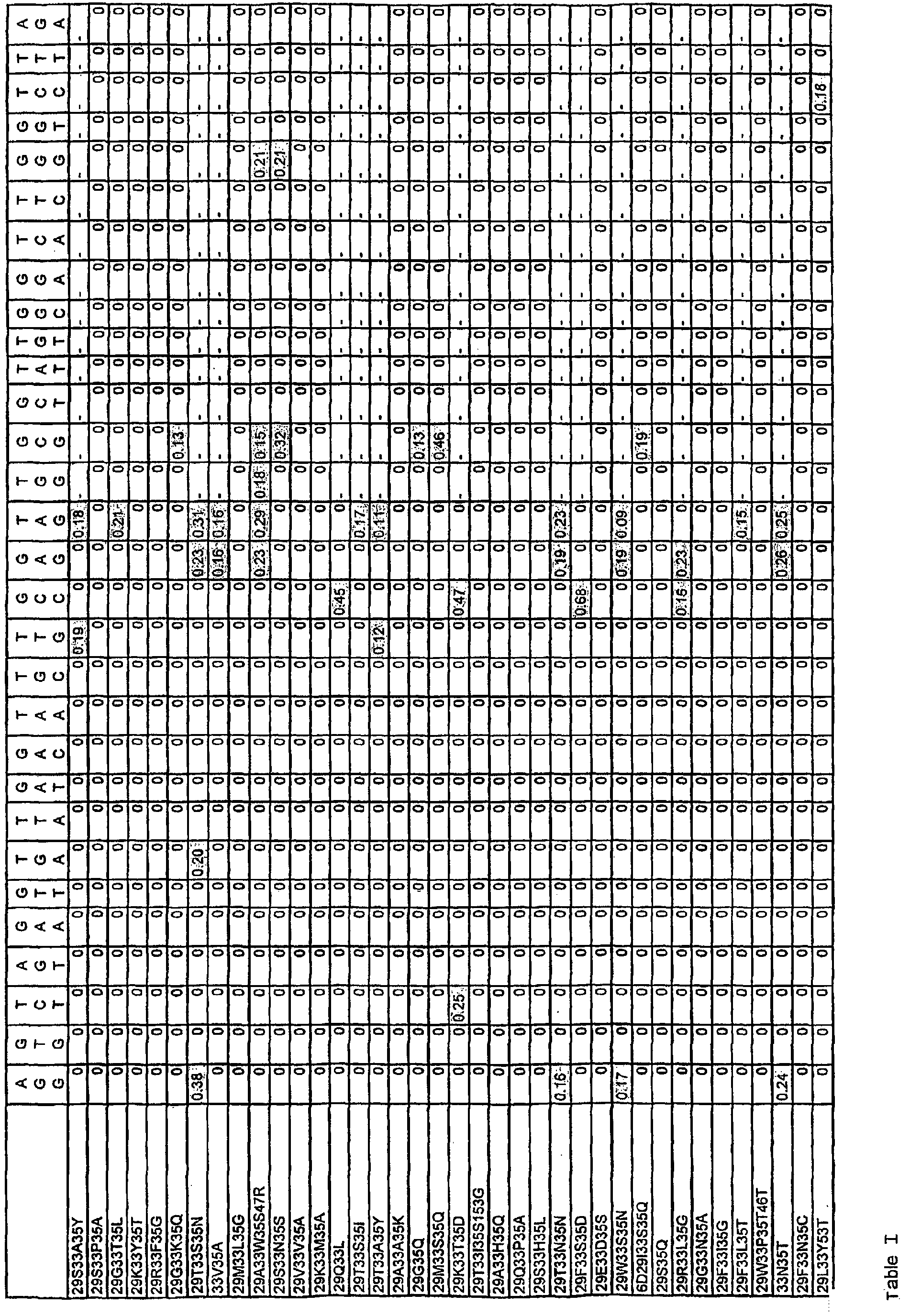

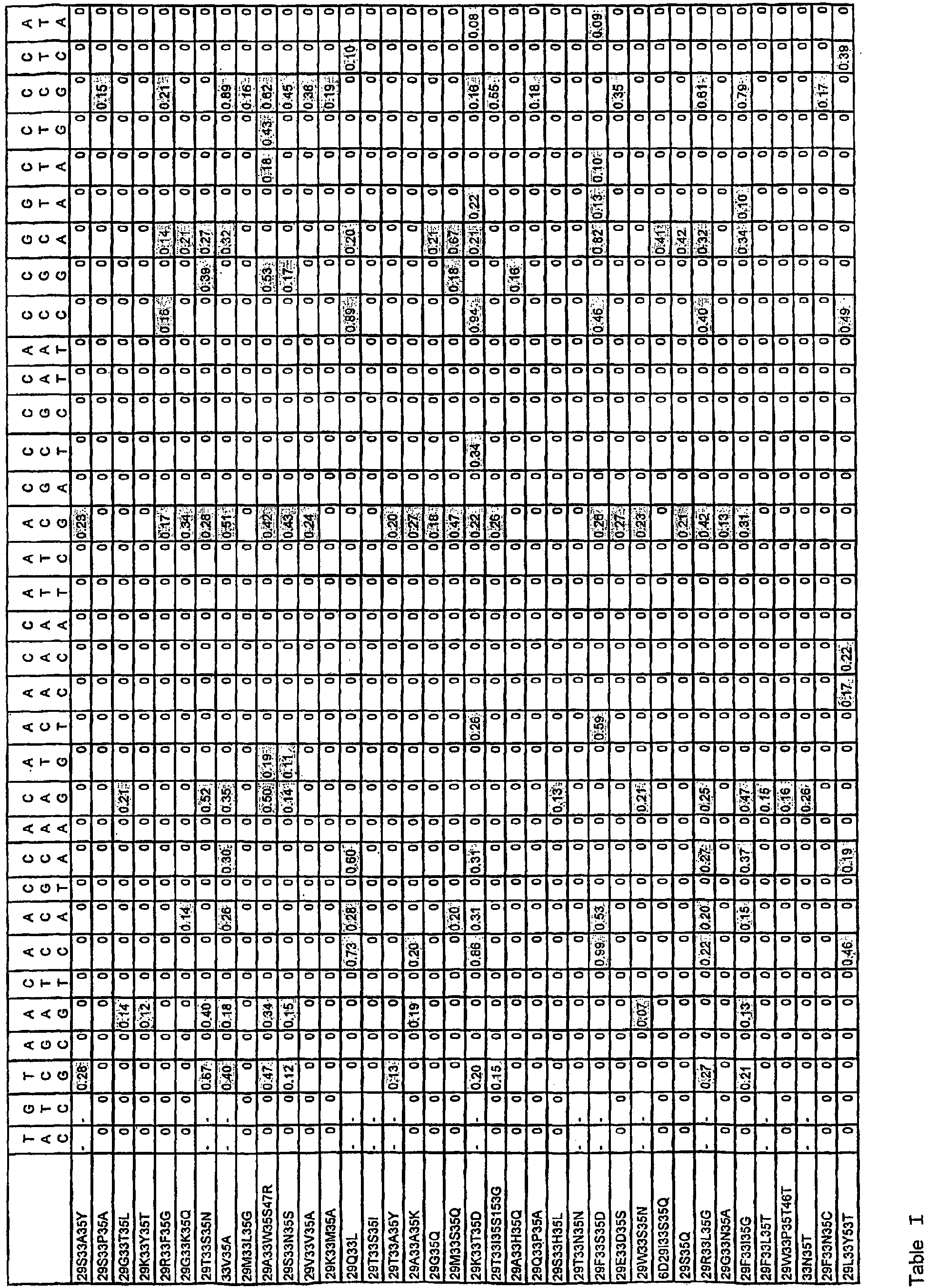

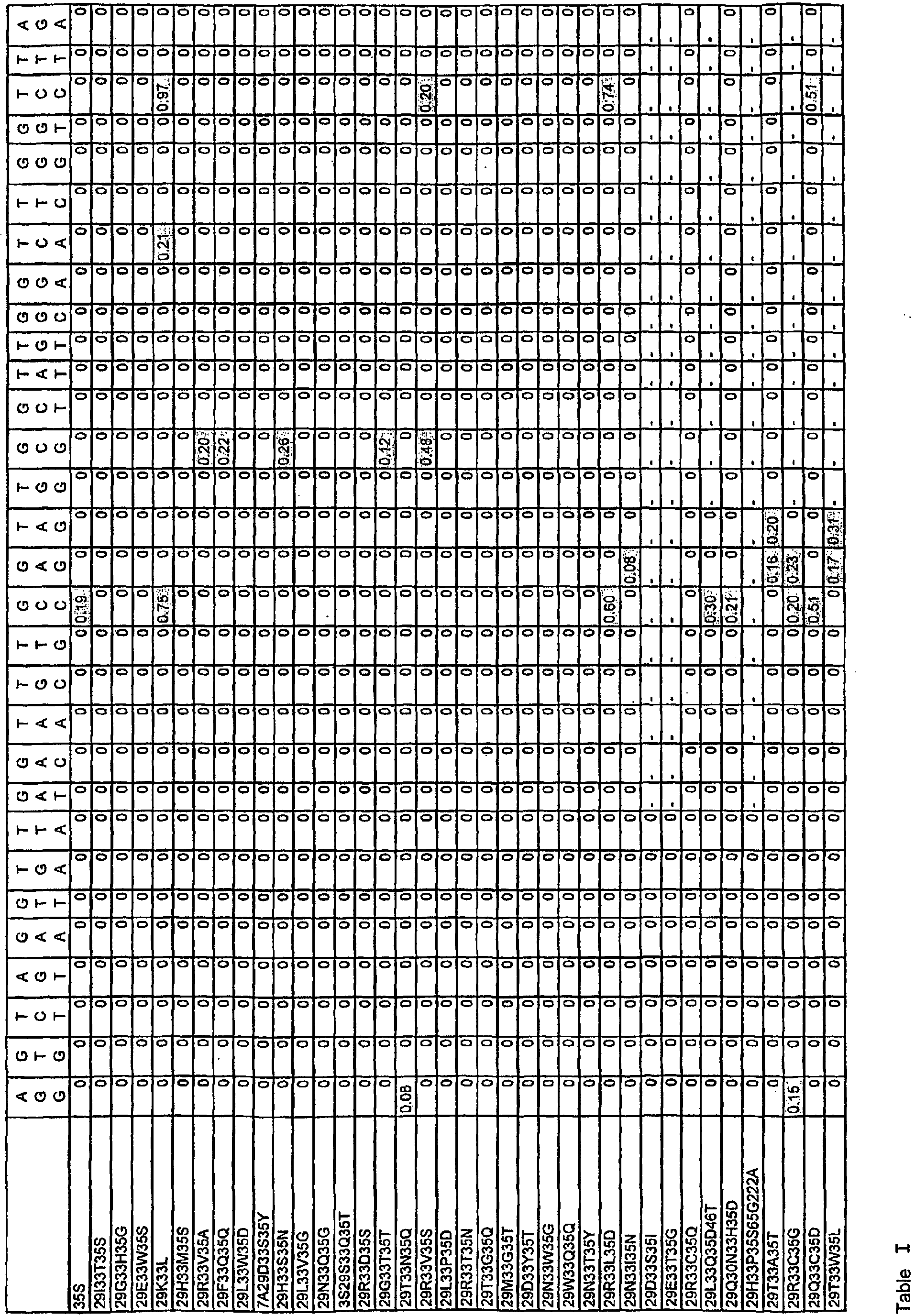

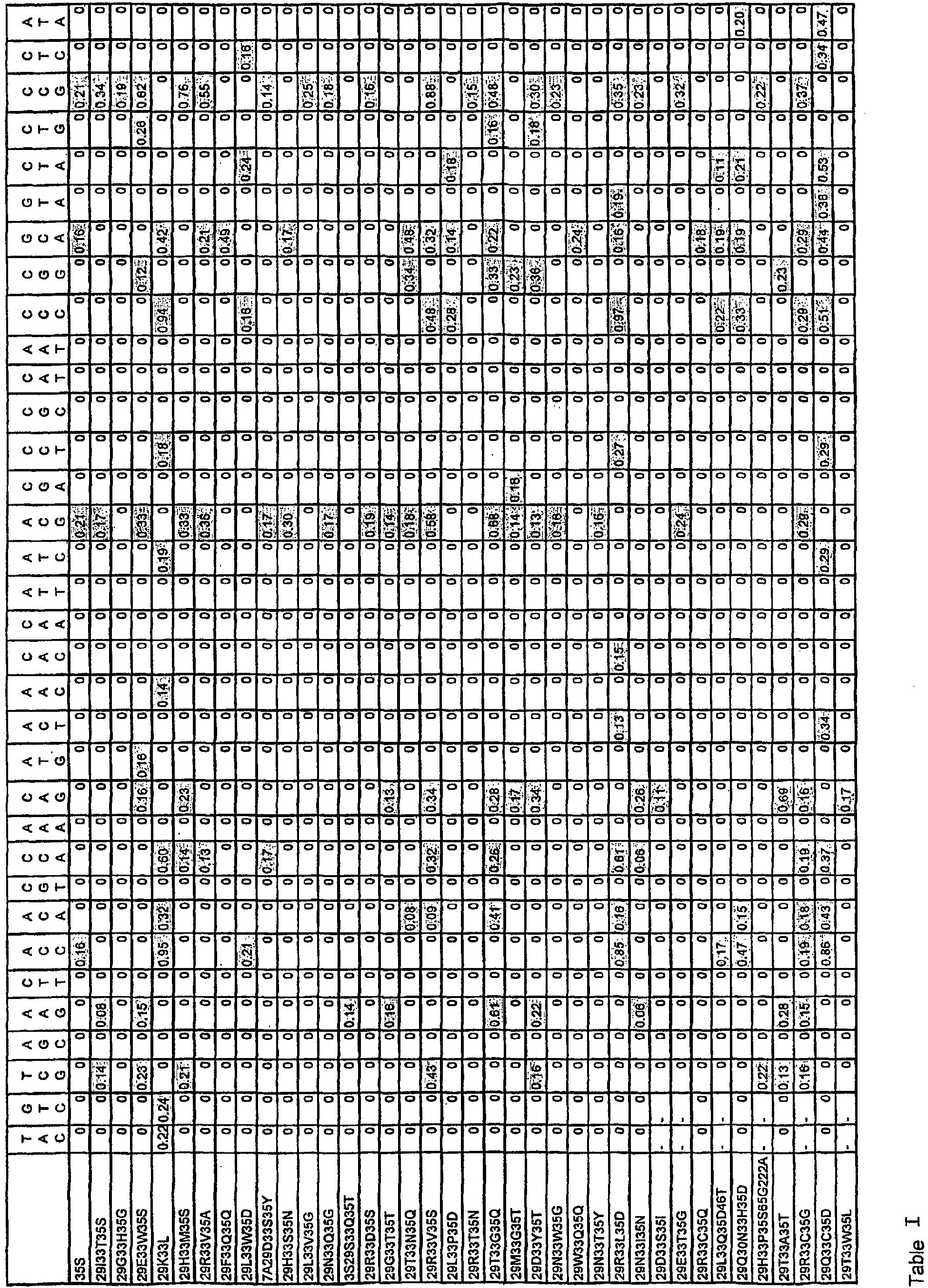

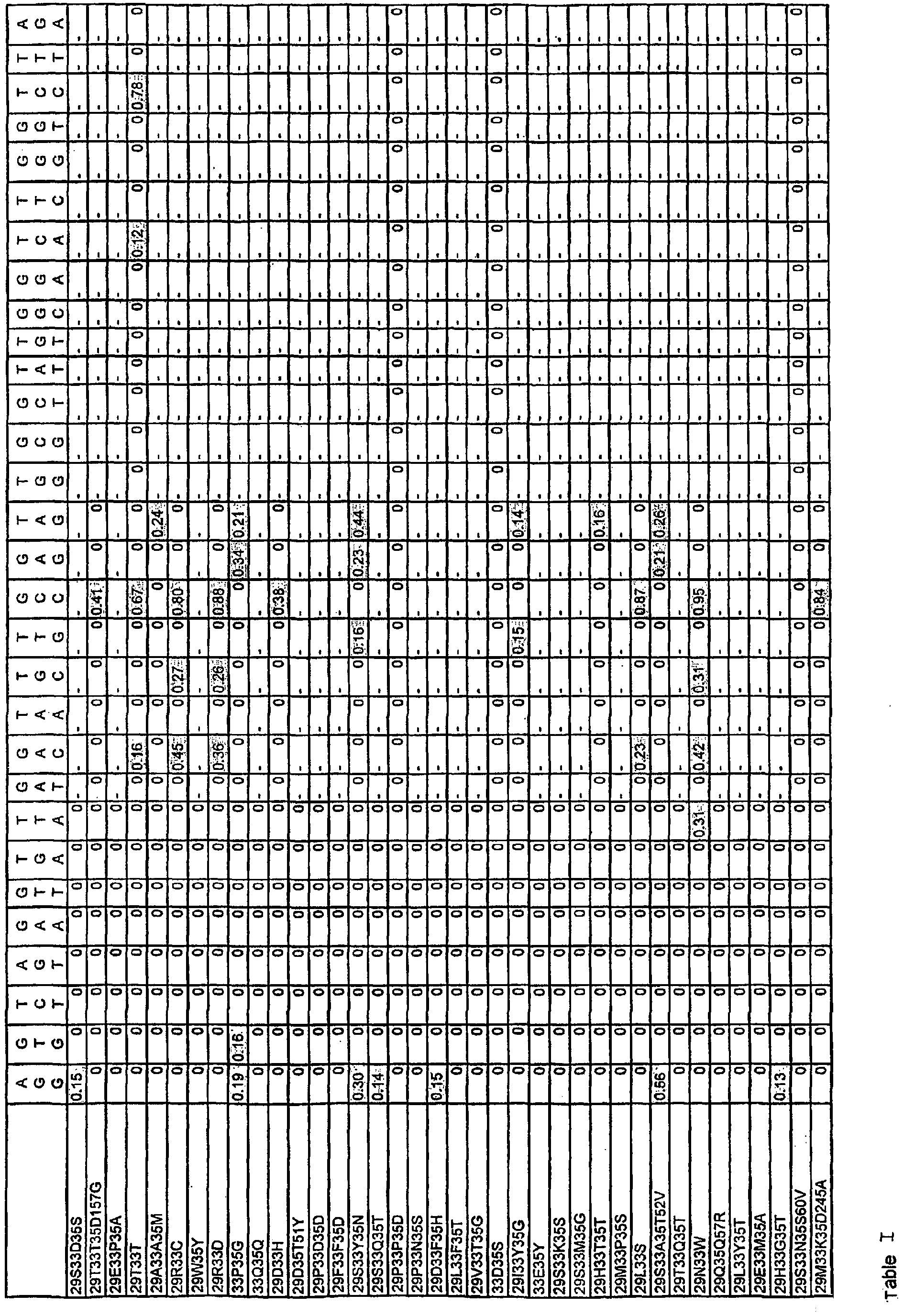

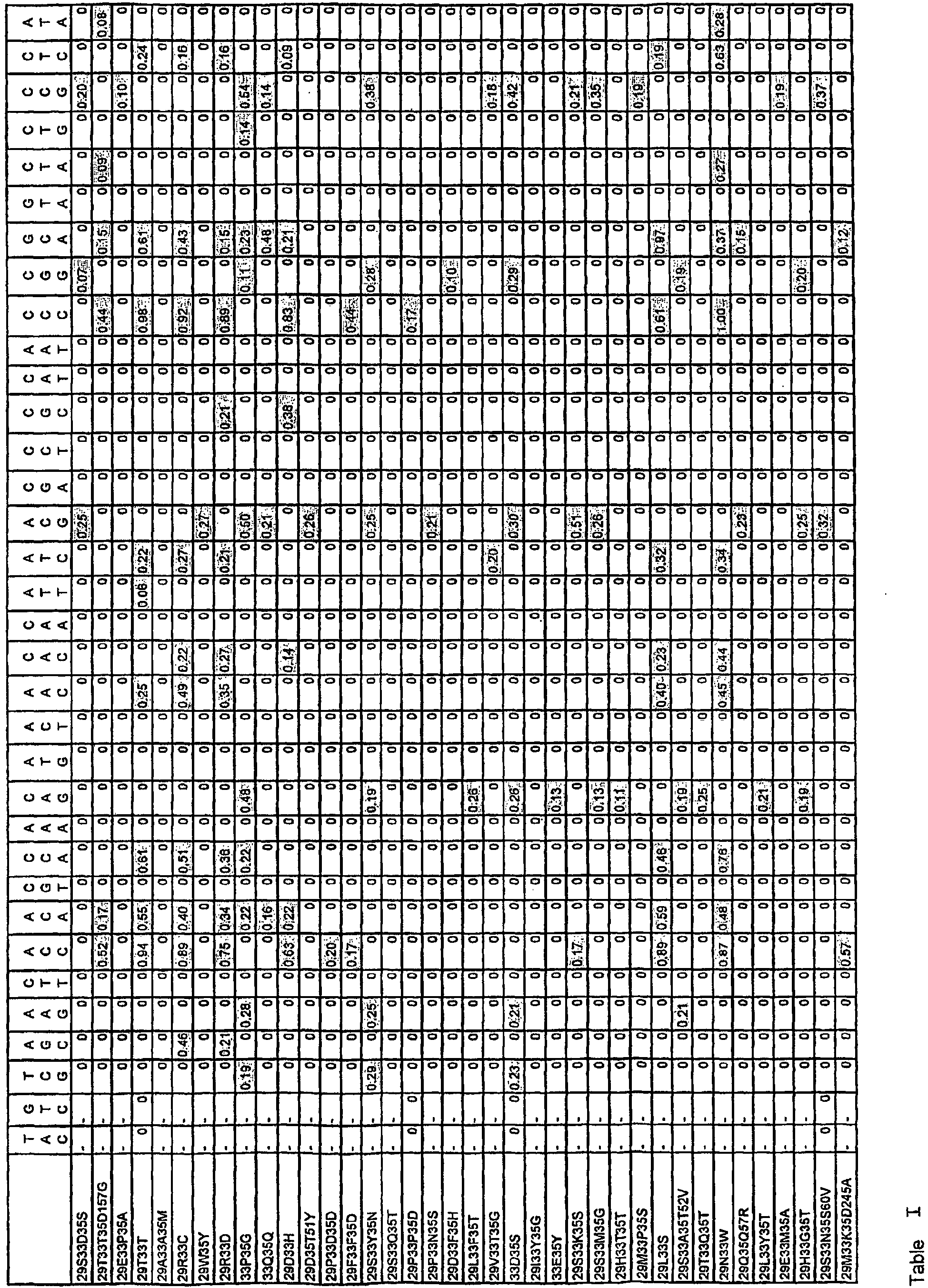

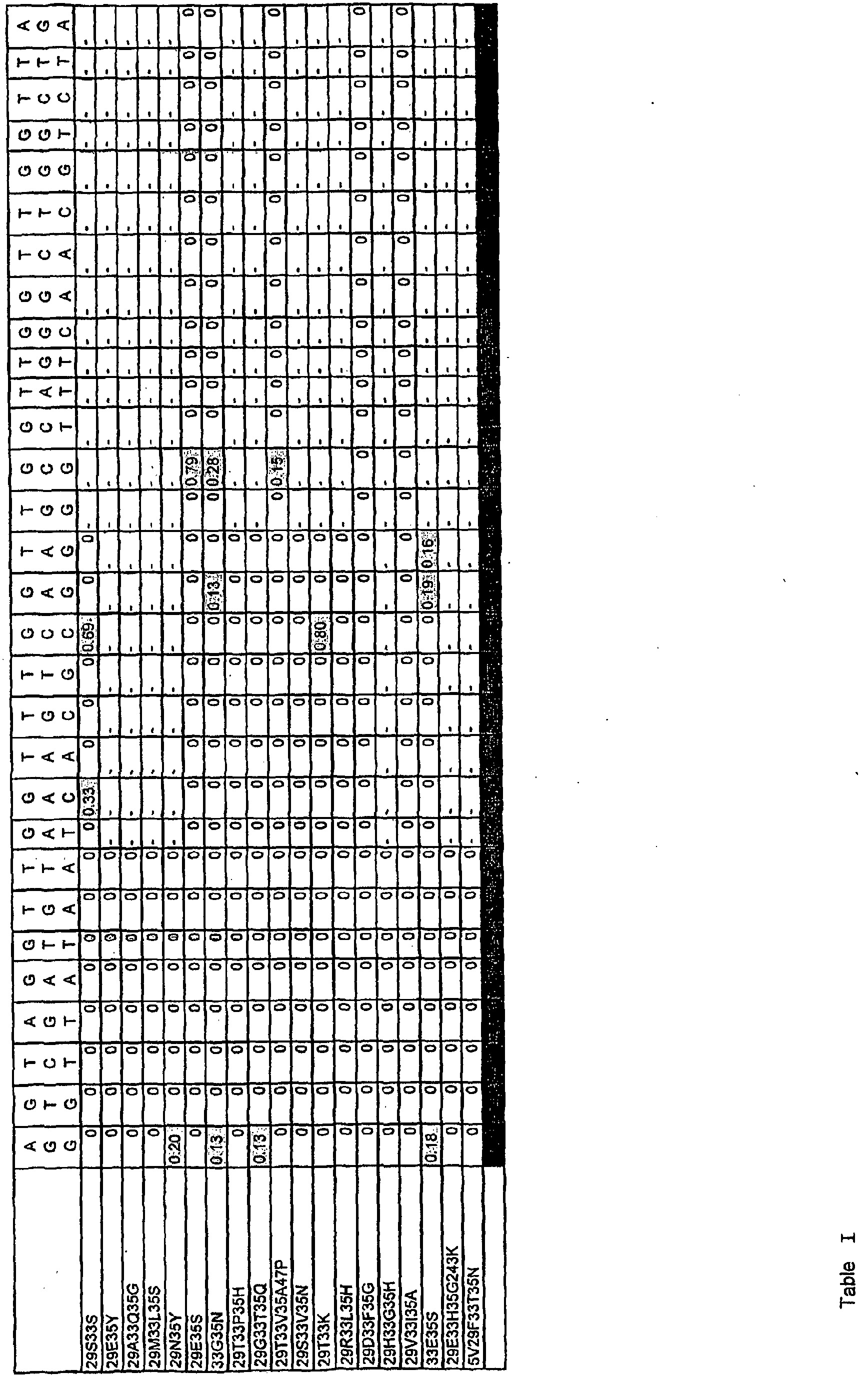

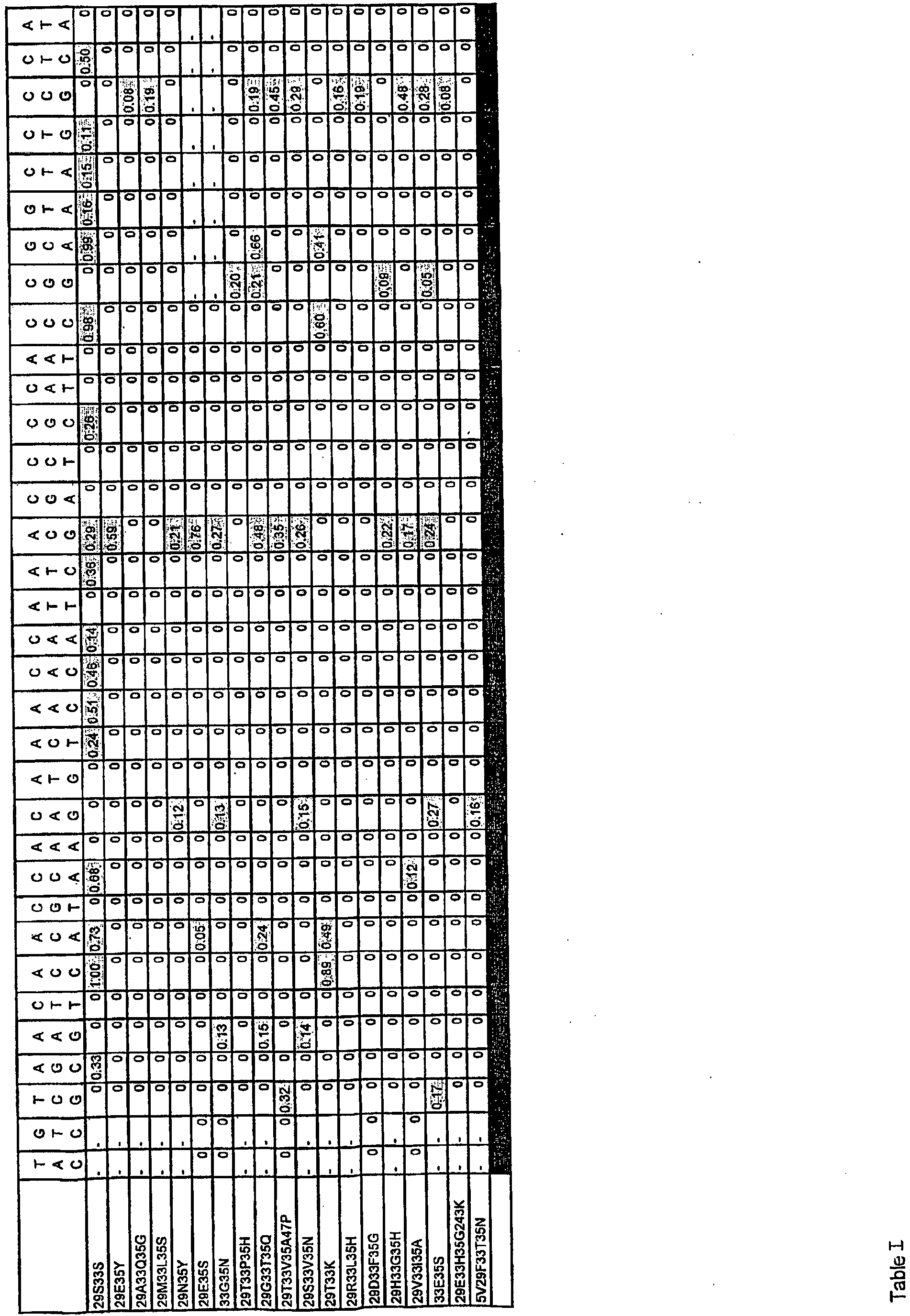

### EXAMPLE 3: Making of DmoCre derived mutants cleaving degenerated DC4NNN_P targets

The applicants have also developed another *DmoCre* scaffold active in yeast and CHO cells, this scaffold as well as being modified at residue 20, a G20S substitution, is also modified at residues corresponding to residues 19 and 109 (I19D and F109Y modifications) of SEQ ID NO: 22 and was named *DmoCre*4 (SEQ ID NO: 9) by the inventors.

To study the possibility of finding additional specificities for the *DmoCre*4 protein (SEQ ID NO: 9), the applicants investigated the three adjacent nucleotides at position+2 to +4 of the C12D34 DNA target. The structure displayed in Figure 2 allowed them to examine closely the contacts between these three base pairs and the protein residues (Figure 8). The inventors have identified three *DmoCre*4 residues corresponding to residues D75, T76 and R77 of SEQ ID NO: 22, that are in contact with the DNA target. In order to isolate new cleavage specificities for the *DmoCre*4 protein, a *DmoCre*4 mutant library mutated at positions 75, 76 or 77 (D4Clib4) was built, transformed into yeast and screened using the yeast screening assay against the 64 targets degenerated at position +2 to +4 that the inventors called DC4NNN (SEQ ID NO: 36). Such an approach has been already thoroughly described for the *I-CreI* protein (Smith J et al., Nucleic Acids Res. 2006; Arnould S, et al., J Mol Biol. 2006; 355:443-58, Arnould S et al., . J Mol Biol. 2007; 371:49-65).

### Material and Methods

### Construction of the 64 target vectors:

The targets were cloned as follow: oligonucleotides corresponding to each of the 64 target sequences flanked by gateway cloning sequence were ordered from Proligo:
5'TGGCATACAAGTTTTCGCCGGANNNTACGACGTTTTGAC AATCGTCTGTCA 3'(SEQ ID NO: 10). Double-stranded target DNA, generated by PCR amplification of the single stranded oligonucleotide, was cloned using the Gateway protocol (Invitrogen) into yeast reporter vector (pCLS1055, Figure 4). Yeast reporter vector was transformed into *S*. *cerevisiae* strain FYBL2-7B (MAT-α, ura3Δ851, trp1 Δ 63, leu2 Δ 1, lys2 Δ202).

### Construction of the DmoCre4 D4Clib4 mutant library:

In order to generate *DmoCre*4 derived coding sequences containing mutations at positions 75, 76 and 77, two separate overlapping PCR reactions were carried out that amplify the 5' end (aa positions 1-74) or the 3' end (positions 66-264) of the *DmoCre*4 coding sequence. For the 3' end, PCR amplification is carried out using a primer specific to the vector (pCLS0542, Figure 5) (Gal10R 5'-ACAACCTTGATTGGAGACTTGACC-3') (SEQ ID NO: 11) and a primer specific to the *DmoCre* coding sequence for amino acids 66-83 (DClib4For 5'-AAATCTAAAATCCAGATCGTTAAGGGTNNKNNKNNKTATGAGCTGCGTGT GAGC-3') (SEQ ID NO: 12). The NNK codons at positions 75 to 77 allow the degeneracy at these positions among the 20 possible amino acids. For the 5' end, PCR amplification is carried out using a primer specific to the vector pCLS0542 (Gal10F 5'-GCAACTTTAGTGCTGACACATACAGG-3') (SEQ ID NO: 13) and a primer specific to the *DmoCre* coding sequence for amino acids 66-74 (DClib4Rev 5'-ACCCTTAACGATCTGGATTTTAGATTT-3') (SEQ ID NO: 14). Then, 25ng of each of the two overlapping PCR fragments and 75ng of vector DNA (pCLS0542) linearized by digestion with NcoI and EagI were used to transform the yeast *Saccharomyces cerevisiae* strain FYC2-6A (MAT-α, trp1Δ63, leu2 Δ1, his3 Δ200) using a high efficiency LiAc transformation protocol (Gietz R D et al., Methods Enzymol. 2002; 350:87-96). An intact *DmoCre* coding sequence is generated by *in vivo* homologous recombination in yeast. The D4Clib4 nucleic diversity is 32³ = 32768. After transformation, 4464 clones were picked, representing about 14% of the library diversity.

### Mating of meganuclease expressing clones and screening in yeast:

Experiments were performed as described in Example 2 above.

### Results

Using the yeast screening assay that has been described above, the 4464 clones that constitute the *DmoCre*4 D4Clib4 library were screened against all the 64 DC4NNN targets except for the DC4GAA target. The screen gave 1194 positive clones able to cleave at least one DC4NNN target (SEQ ID NO: 36). These clones were not characterized at the sequence level. The initial *DmoCre*4 protein is able to cleave 4 out of 63 DC4NNN targets. The D4Clib4 hitmap displayed in Figure 9 shows that by introducing mutations at positions 75, 76 and 77 in the *DmoCre4* coding sequence, 21 DC4NNN targets are now being cleaved by *DmoCre4* derived mutants. Our screening approach has therefore allowed us to widen the *DmoCre4* cleavage spectrum for DC4NNN targets and to isolate new cleavage specificities.

### EXAMPLE 4: Making of DmoCre derived mutants cleaving the 5CAGD34 target

The inventors have previously shown that they were able to modify the *I-CreI* protein specificity toward palindromic DNA targets derived from C1221 and degenerated at positions ±5, ±4, ±3 (Arnould et al, J Mol Biol. 2006; 355:443-58). By introducing mutations in the *I-CreI* coding sequence at positions 44, 68, 70, 75 and 77, they were able to obtain *I-CreI* derived mutants that cleave the 5CAG_P target (SEQ ID NO: 32).

In the present example, the inventors show that by introducing these same mutations in the *DmoCre*2 or *DmoCre*4 coding sequences, they can generate *DmoCre* derived mutants that cleave the 5CAGD34 target (SEQ ID NO: 33) (Figure 10). To generate these *DmoCre* derived mutants, they took 36 *I-CreI* mutants able to cleave the 5CAG_P target (SEQ ID NO: 32). The coding sequence of the *I-CreI* moiety was removed from the *DmoCre*2 or the *DmoCre*4 proteins by restriction enzyme digestion and replaced by the 5CAG_P cutter coding sequences. The two mutant libraries DCSca2_5CAG and DCSca4_5CAG based respectively on the *DmoCre*2 and *DmoCre*4 proteins were built and screened against the 5CAGD34 target (SEQ ID NO: 33) using the yeast screening assay described previously herein.

### Material and Methods

### Construction of the 5CAGD34 target vector

The target was cloned as follow: an oligoncleotide corresponding to the target sequence flanked by gateway cloning sequences was ordered from Proligo: 5' TGGCATACAAGTTTTCGCCGGAACTTAC**CTG**GTTTTGACAATCGTCTG TCA 3' (SEQ ID NO: 15). Double-stranded target DNA, generated by PCR amplification of the single stranded oligonucleotide, was cloned using the Gateway protocol (Invitrogen) into yeast reporter vector (pCLS1055, Figure 4). Yeast reporter vector was transformed into *S*. *cerevisiae* strain FYBL2-7B (MAT-α, ura3Δ851, trp1Δ63, leu2Δ1, lys2Δ202).

### Construction of the DCsca2_5CAG mutant library

In order to generate *DmoCre*2 derived coding sequences that contain mutations in the *I-CreI* moiety sequence responsible for the 5CAG_P target cleavage, a PCR reaction was carried out that amplified the region between aa 13-148 for each of the *I-CreI* derived 5CAG_P cutters. PCR amplification was carried out using the primers CreNgoLib (5' CGTGAGCAGCTGGCGTTCCTGGCCGGCTTTGTGGAC GGTGAC-3' (SEQ ID NO: 16)) and CreMluLib (5'-ACGAACGGTTTCAGAAGT GGTTTTACGCGTCTTAG-3' (SEQ ID NO: 17)).

The 36 PCR fragments were then pooled. The yeast expression vector for the *DmoCre*2 protein was then digested with NgoMIV and MluI removing a fragment covering residues 111 to 238 of the *DmoCre*2 protein. Finally, 25 ng of the overlapping PCR pool and 75 ng of the digested vector DNA were used to transform the yeast *Saccharomyces cerevisiae* strain FYC2-6A (MAT-α, trp1Δ63, leu2Δ1, his3Δ200) using a high efficiency LiAc transformation protocol (Gietz R D et al., Methods Enzymol. 2002; 350:87-96). An intact *DmoCre* coding sequence containing the mutations characteristic of the 5CAG_P cutters was generated by *in vivo* homologous recombination in yeast. After transformation, 186 clones were picked, representing about 5 times the library diversity.

### Construction of the DCSca4_5CAG mutant library

In order to generate *DmoCre4* derived coding sequences that contain mutations in the *I-CreI* moiety sequence responsible for the 5CAG_P target cleavage, a PCR reaction was carried out that amplified the region between aa 13-148 for each of the *I-CreI* derived 5CAG_P cutters. PCR amplification was carried out using the primers CreMluLib and CreNgoLibY (5' CGTGAGCAGCTGGCGTACCTGGCC GGCTTTGTGGACGGTGAC-3') (SEQ ID NO: 18), which takes into account the F109Y mutation characteristic of the *DmoCre4* protein. The 36 PCR fragments were then pooled. The yeast expression vector for the *DmoCre*4 protein was then digested with the restriction enzymes NgoMIV and MluI removing a fragment covering residues 111 to 238 of the *DmoCre*4 protein. Finally, 25ng of the PCR pool and 75ng of the digested vector DNA were used to transform the yeast *Saccharomyces cerevisiae* strain FYC2-6A (MAT-α, trp1Δ63, leu2Δ1, his3Δ200) using a high efficiency LiAc transformation protocol (Gietz R D et al., Methods Enzymol. 2002; 350:87-96). An intact *DmoCre* coding sequence containing the mutations characteristic of the 5CAG_P cutters was generated by *in vivo* homologous recombination in yeast. After transformation, 186 clones were picked, representing about 5 times the library diversity.

### Mating of meganuclease expressing clones and screening in yeast:

Experiments were performed as described in Example 2 above.

### Results

Using the yeast screening assay that has been described above in Example 1, the 186 clones that constitute the DCSca2_5CAG library and the 186 clones that constitute the DCSca4_5CAG library were screened against the 5CAGD34 (SEQ ID NO: 33) target. The first library gave 32 positive clones and the second one 40 positive clones with an overall stronger cleavage efficiency. Examples of positives are shown on Figure 11. The clones that cleave the 5CAGD34 target (SEQ ID NO: 33) do not cleave the 5CAG_P target (SEQ ID NO: 32) (data not shown). So the inventors have demonstrated that it is possible to introduce specific *I-CreI* mutations in the *DmoCre* scaffold to cleave efficiently the combined target.

### EXAMPLE 5: Making of a DmoCre derived mutant cleaving the RAG1.10.2D34 target

The RAG1.10.2 DNA palindromic target (SEQ ID NO: 34) derives from the *I-CreI* C1221 target (SEQ ID NO: 29) (Figure 12). The inventors have previously shown how, by combining 10GTT (e.g. nucleotides 8, 9 and 10 are G, T and T respectively) and 5CAG *I-CreI* derived mutants and then performing a random mutagenesis step on the first isolated RAG1.10.2 cutters, they were able to obtain a *I-CreI* derived mutant that cleaves very strongly the RAG1.10.2 target (WO2008/010009). This mutant called M2 bears the following mutations in comparison to the wild-type *I-CreI* sequence: N30R, Y33N, Q44A, R68Y, R70S, I77R of SEQ ID NO: 24. These same mutations were introduced in the *I-CreI* moiety of the *DmoCre*2 protein and the activity of the resulting *DmoCre* mutant called DmoM2 (SEQ ID NO: 71) against the RAG1.10.2D34 (SEQ ID NO: 35) combined target was probed using the yeast cleavage assay.

### Material and Methods

### Construction of the RAG1.10.2D34 target vector

The target was cloned as follow: an oligoncleotide corresponding to the target sequence flanked by gateway cloning sequences was ordered from Proligo: 5' TGGCATACAAGTTTTCGCCGGAACTTACCTGAGAACAACAATCGTCTG TCA 3' (SEQ ID NO: 19). Double-stranded target DNA, generated by PCR amplification of the single stranded oligonucleotide, was cloned using the Gateway protocol (Invitrogen) into yeast reporter vector (pCLS1055, Figure 4). Yeast reporter vector was transformed into *S*. *cerevisiae* strain FYBL2-7B (MAT α, ura3Δ851, trp1Δ63, leu2Δ1, lys2Δ202).

### Construction of the DmoM2

In order to generate a *DmoCre*2 derived coding sequence that contains mutations in the *I-CreI* moiety specific to the RAG1.10.2 M2 mutant, a PCR reaction was carried out that amplify the region between aa 9-146 of the M2 mutant. PCR amplification is carried out using the primers CreNgoFor (5' TTCCTGCTGTACCTGGCCGGCTTTGTGG-3' (SEQ ID NO: 20)) and CreMluRev (5'- TTCAGAAGTGGTTTTACGCGTCTTAG -3' (SEQ ID NO: 21)). The PCR fragment was then digested with the restriction enzymes NgoMIV and MluI as was the yeast expression vector containing the ORF for the *DmoCre*2 protein. A ligation reaction was performed and *E*. *coli* DH5 α was transformed with the ligation mixture. The resulting DmoM2 mutant was then amplified and sequenced.

### Mating of meganuclease expressing clones and screening in yeast:

Experiments were performed as described in Example 2 above.

### Results

Using this yeast cleavage assay, activity of the *DmoM2* mutant against the combined RAG1.10.2D34 target (SEQ ID NO: 35) and other different targets was probed. Figure 13 shows that, the *DmoM2* cleaves specifically the combined RAG1.10.2D34 target (SEQ ID NO: 35). Therefore, the inventors have demonstrated that it is possible to introduce into the *I-CreI* moiety of the *DmoCre* scaffold mutations that were previously isolated in the *I-CreI* scaffold during a combinatorial and/or optimization experiment for a target sequence, in order to cleave efficiently a combined *DmoCre* target which comprises a portion of the target sequence.

### EXAMPLE 6: Making of a DmoCre derived mutants cleaving the RAG1.10.2D34 or RAG1.10.3D34 targets

The RAG1.10.2 and RAG1.10.3 DNA palindromic targets (SEQ ID NO: 34 and 3 8) derive from the *I-CreI* C1221 target (SEQ ID NO: 29) (Figure 14). The inventors have previously shown how, by combining 10GTT and 5CAG *I-CreI* derived mutants, they were able to obtain *I-CreI* derived mutants that cleave very strongly the RAG1.10.2 target or by combining 10TGG and 5GAG *I-CreI* derived mutants, they were able to obtain *I-CreI* derived mutants that cleave very strongly the RAG1.10.3 target (WO2008/010009 and W02008/010093). By using the same methodology as described in Example 4, the coding sequence of the *I-CreI* moiety was removed from the *DmoCre*2 protein by restriction enzyme digestion and replaced either by the RAG1.10.2 cutters coding sequences or by the RAG1.10.3 cutters coding sequences. Table II below sums up the mutations in the I-CreI moiety in reference to residue numbering in I-*Cre*I sequence SEQ ID NO: 24 for RAG1.10.2 cutters coding sequences and Table III below sums up the mutations in the I-*Cre*I moiety for RAG1.10.3 cutters coding sequences. To generate DCSca2_RAG1.10.2 and DCSca2_RAG1.10.3 mutant libraries, 33 RAG1.10.2 cutters and 35 RAG1.10.3 cutters were respectively used. The two mutant libraries were then screened respectively against the RAG1.10.2D34 and RAG1.10.3D34 targets and also against the parental targets using the previously described yeast screening assay.

### RAG1.10.2 Cutters

**Table II**

| **N**° | |
|---|---|
| 1 | 30K33A44R68Y70S77T |
| 2 | 30R33C44A68Y70S75Y77N |
| 3 | 30R33C44R68Y70S77T |
| 4 | 30K33A44N68Y70S75Y77R82T |
| 5 | 33R38T44A68S70S73I77R |
| 6 | 30K33A44A68Y70S75Y77K |
| 7 | 30R33T44A68Y70S75Y77R |
| 8 | 30R32D44A68Y70S75Y77K |
| 9 | 30R33N44R68Y70S77T |
| 10 | 30R33N44A68S70P75Y |
| 11 | 30K33G44A68Y70S75Y77K |
| 12 | 30R33C44R68Y70S77T |
| 13 | 33R38T44A68Y70S75Y77K |
| 14 | 30R33N44K68Y70S75Y77N |
| 15 | 30R33C44A68Y70S75Y77V |
| 16 | 30R33N44N68Y70S75Y77K |
| 17 | 30R33S44N68Y70S75Y77R82T |
| 18 | 30R33C44A68Y70S75Y77K |
| 19 | 30K33A44A68S70S73I77R |
| 20 | 30K33S44A68Y70S75Y77K |
| 21 | 30R32D44A68Y70S75Y77K |
| 22 | 30K33A44T68Y70S75Y77R |
| 23 | 30R33N44A68Y70S75Y77K |
| 24 | 30K33G44A68Y70S75Y77K |
| 25 | 30R33C44N68Y70S75Y77K |
| 26 | 30R32D44A68S70S73I77R |
| 27 | 30R33Q44A68Y70S75Y77R |
| 28 | 33R38T44A68Y70S75Y77K |
| 29 | 30R33N44T68Y70S75Y77R |
| 30 | 30K33G44A68Y70S75Y77K |
| 31 | 33H38G44A68Y70S75Y77K129M |
| 32 | 30R33N44N68Y70S75Y77R82T |
| 33 | 30K33S44A68S70S73I77R |

**Table III**

| | **RAG1.10.3 Cutters** |
|---|---|
| **N°** | |
| 1 | 28N33S38R40R44A70T75N |
| 2 | 28Q33S38R40K44Y68N70S75N77V |
| 3 | 28N33S38R40R44A68A70N75N |
| 4 | 28A33S38R40K44A68A70N75N129A |
| 5 | 30H33M38A44A70S75Y |
| 6 | 28N33S38R40R44Y70S75Y77Q |
| 7 | 28N33S38R40R44A68T70N75N |
| 8 | 28N33S38R40R44A68T70N75N |
| 9 | 28N33S38R44A68T70N75N |
| 10 | 28A33S38R40K44T70S75Y |
| 11 | 32D33C44A70S75Y77Q |
| 12 | 28Q33S38R40K44N68S70S75N77V |
| 13 | 30H33M38A44A70S75Y77V |
| 14 | 30H33M38A40R44A68H70Q75N |
| 15 | 30H33M38A44A70S75Y77T |
| 16 | 28N33S38R40R44N68Y70S75Y77V |
| 17 | 30R33Q44D68N70S75N |
| 18 | 28N33S38R40R44Y70S75Q77V |
| 19 | 28N33S38R40R44A70N75N |
| 20 | 33T38A40K44N68Y70S75Y77N |
| 21 | 28Q33S38R40K44A68H70Q75N |
| 22 | 28Q33S38R40K44Y70S75Y77Q |
| 23 | 28Q33S38R40K44A68A70N75N103S153G |
| 24 | 28N33S38R40R44T70S75Y |
| 25 | 28N33S38R40R44N70S75Y77V |
| 26 | 28N33S38P40R44A68T70N75N129A |
| 27 | 28N33S38R4OR44S70S75Y77Q |
| 28 | 28N33S38R40R44T70S75Y |
| 29 | 28Q33S38R40K44N68Y70S75Y77V |
| 30 | 28Q33S38R40K44T70S75Y |
| 31 | 28Q33S38R40K44A68H70H75N |
| 32 | 28N33S38R40R44A70N75N |
| 33 | 28Q33S38R40K44A70S75N |
| 34 | 28N33S38R40R44A70N75N |
| 35 | 30H33M38A44A70N75N |

### Material and Methods

### Construction of the RAG1.10.3D34 target vector

The target was cloned as follow: an oligoncleotide corresponding to the target sequence flanked by gateway cloning sequences was ordered from Proligo: 5'TGGCATACAAGTTTTCGCCGGAACTTACGTCAGCCAGACAATCGTCTGTC A-3' (SEQ ID NO: 19). Double-stranded target DNA, generated by PCR amplification of the single stranded oligonucleotide, was cloned using the Gateway protocol (Invitrogen) into yeast reporter vector (pCLS1055, Figure 4). Yeast reporter vector was transformed into *S. cerevisiae* strain FYBL2-7B (MAT-α, ura3Δ851, trp1Δ63, leu2Δ1, lys2Δ202).

### Construction of the DCSca2_RAG1.10.2 mutant library

In order to generate *DmoCre2* derived coding sequences that contain mutations in the *I-CreI* moiety sequence responsible for RAG1.10.2 target cleavage, a PCR reaction was carried out that amplified the region between aa 13-148 for each of the 33 *I-CreI* derived RAG1.10.2 cutters, in addition the primers also comprise portions homologous at either end to the sequence of the expression vector comprising DmoCre2. PCR amplification is carried out using the primers CreNgoLib (5' CGTGAGCAGCTGGCGTTCCTGGCCGGCTTTGTGGACGGTGAC-3' (SEQ ID NO: 16)) and CreMluLib (5'-ACGAACGGTTTCAGAAGT GGTTTTACGCGTCTTAG-3' (SEQ ID NO: 17)).

The 33 PCR fragments were then pooled. The yeast expression vector for the *DmoCre*2 protein was then digested with NgoMIV and MluI removing a fragment covering residues 111 to 238 of the *DmoCre*2 protein. Finally, 25 ng of the PCR pool and 75 ng of the digested vector DNA were used to transform the yeast *Saccharomyces cerevisiae* strain FYC2-6A (MAT-α, trp1Δ63, leu2Δ1, his3Δ200) using a high efficiency LiAc transformation protocol (Gietz R D et al., Methods Enzymol. 2002; 350:87-96). An intact *DmoCre* coding sequence containing the mutations characteristic of the RAG1.10.2 cutters was generated by *in vivo* homologous recombination in yeast. After transformation, 186 clones were picked, representing about 5 times the library diversity.

### Construction of the DCSca2_RAG1.10.3 mutant library

The methodology was the same as for the DCSca2_RA.G1.10.2 mutant library, except a pool of 35 RAG1.10.3 cutters was used.

### Mating of meganuclease expressing clones and screening in yeast:

Experiments were performed as described in Example 2 above.

### Results

Using the yeast screening assay that has been described above in Example 2, the 186 clones that constitute the DCSca2_RAG1.10.2 mutant library and the 186 clones that constitute the DCSca2_RAG1.10.3 mutant library were screened respectively against the RAG1.10.2D34 and RAG1.10.3D34 targets. The DCSca2_RAG1.10.2 library yielded 36 positive clones, 24 clones among them were rearrayed and submitted to a secondary round of screening shown in Figure 15. The 24 clones represent 8 unique sequences (see Table IV) and are specific of the RAG1.10.2D34 target: they do not cleave the RAG1.10.2, C1221 and C12D34 targets. The DCSca2_RAG1.10.3 library gave 52 positive clones, 33 clones among them were rearrayed and submitted to a secondary screening shown in Figure 15. The 33 clones represent 6 unique sequences (see Table IV) and are specific to the RAG1.10.3D34 target: they do not cleave the RAG1.10.3, C1221 and C12D34 targets. So the inventors have demonstrated that it is possible to introduce specific *I-CreI* mutations in the *DmoCre* scaffold using a library approach to cleave efficiently and specifically the combined target.

### EXAMPLE 7: Refinement of RAG1.10.2D34 and RAG1.10.3D34 meganucleases by random mutagenesis.

To improve the cleavage efficiency of the RAG1.10.2D34 and RAG1.10.3D34 cutters identified in example 6, a round of random mutagenesis was undertaken on selected RAG1.10.2D34 and RAG1.10.3D34 cutters isolated in example 6. For each target, three mutants among those described in Example 6 were chosen, see Table IV. Their DNA was pooled and used as template for the PCR randomization. A mutant library was built in the yeast and screened against the adequate target.

### Material and Methods

### Construction of libraries by random mutagenesis

On each pool of mutants, random mutagenesis by PCR using Mn²⁺ at a concentration of 0.3 mM was performed. Primers used are preATGCreFor (5'GCATAAATTACTATACTTCTATAGACACGCAAACACAAATACACAGCG GCCTTGCCACC-3' (SEQ ID NO: 40)) and ICreIpostRev (5'-GGCTCGAGGAGCTCGTCTAGAGGATCGGTCGAGTTATCAGTCGGCCGC-3' (SEQ ID NO: 41)).

Approximately 25ng of the PCR product and 75ng of vector DNA (pCLS542, Figure 5) linearized by digestion with *NcoI* and *EagI* were used to transform the yeast *Saccharomyces cerevisiae* strain FYC2-6A (MAT-α, trp1Δ63, leu2Δ1, his3Δ200) using a high efficiency LiAc transformation protocol (Gietz and Woods 2002). Expression plasmids containing an intact coding sequence for the *DmoCre* mutant was generated by *in vivo* homologous recombination between overlapping portions of the PCR product and digested vector, in yeast.

### Mating of meganuclease expressing clones and screening in yeast:

Experiments were performed as described in Example 2 above.

### Results

Table IV below shows the sequence of the eight RAG1.10.2D34 cutters and the six RAG1.10.3D34 cutters. Among them, the three first of each class of cutters (underlined in Table IV) were chosen to perform the randomizing PCR. Their sequences derive from the *DmoCre2* protein and differ at residues at positions 126, 128, 130, 131, 136, 138, 142, 166, 168, 173 and 175 (SEQ ID NO: 2). These positions correspond to the positions 28, 30, 32, 33, 38, 40, 44, 68, 70, 75 and 77 of I-*CreI* (SEQ ID NO: 24) respectively. As indicated above these RAG1.10.2D34 and RAG1.10.3D34 cutters also comprise the mutations present in DmoCre2, namely the L15Q, I19D and G20S mutations, which are all located in the *I-DmoI* N-terminal LAGLIDADG alpha-helix.

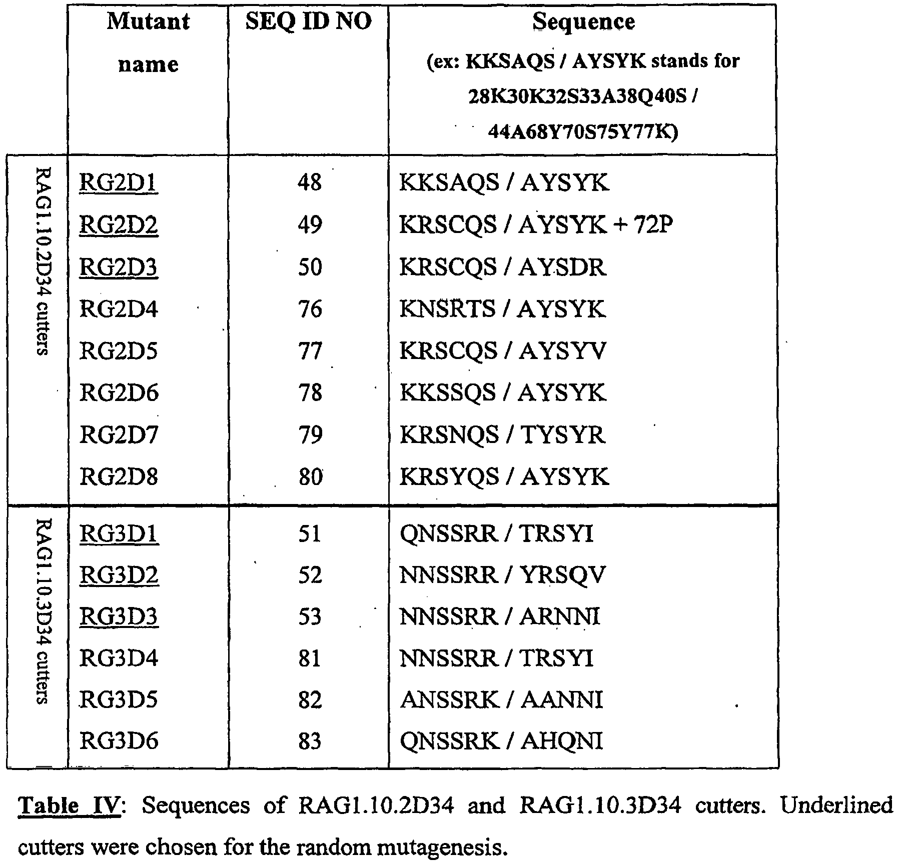

The mutant libraries created from the randomizing PCR were then screened with our yeast screening assay against their respective target. The RG2D2 and RG3D3 mutants were used as a control. Mutants presenting an activity increase in comparison to the control mutants were selected and submitted to a secondary round of screening screening shown in Figure 16. For each target, three mutants with improved cleavage activity have been circled. These selected mutants were isolated and sequenced and Table V shows their sequences.

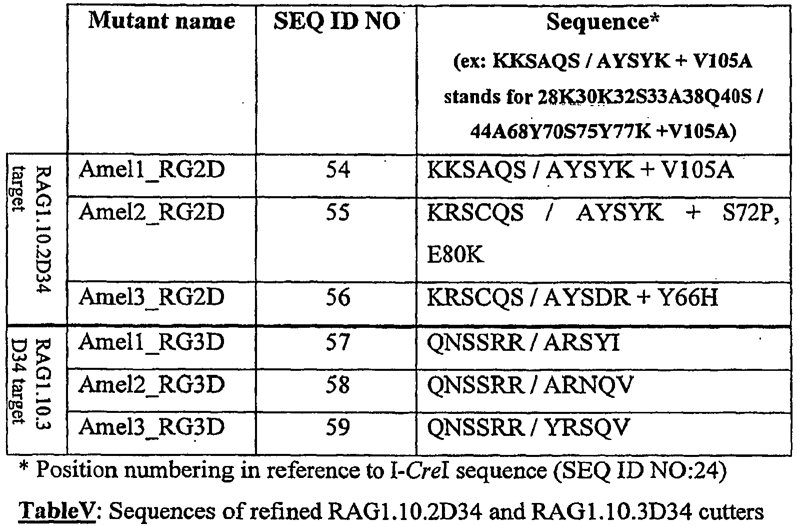

Table V shows that the cleavage activity improvement for the RAG1.10.2D34 target comes from the introduction of the V105A, E80K and Y66H mutations in I-*Cre*I moiety (position numbering in reference to I*-Cre*I sequence SEQ ID NO:24). In the case of the RAG1.10.3D34 target, the activity increase is not provided by additional mutations but by an exchange of mutations between the three RAG1.10.3D34 cutters that were used to perform the mutagenesis.

### EXAMPLE 8: Making of new DmoCre derived mutants cleaving degenerated DC4NNN_P targets

To search for DmoCre scaffolds with specificities for the DC4NNN targets (SEQ ID NO: 36), a new mutant library based on the *DmoCre2* protein was generated in yeast. As mentioned in Example 3, the three residues D75, T76 and R77 of SEQ ID NO: 22, contact the three bases at position +2 to +4 of the C12D34 target. Residue T41 of SEQ ID NO: 22, is also involved and establishes also a Van der Waals contact with the methyl group of the thymine located at position +4 of the C12D34 target. It was thought by the inventors that mutation of this residue could provide new specificities for the *DmoCre2* protein toward the DC4NNN targets. Therefore, in order to isolate new cleavage specificities for the *DmoCre2* protein, a *DmoCre2* mutant library (D4Clib2Bis) mutated at positions corresponding to residues 41, 75 or 77 of SEQ ID NO: 22 (I-*Dmo*I moiety) was constructed and transformed into yeast and screened using the yeast screening assay against the 64 targets degenerated at position +2 to +4 (DC4NNN SEQ ID NO: 36).

### Material and Methods

### Construction of the DmoCre2 D4Clib2Bis mutant library:

In order to generate *DmoCre*2 derived coding sequences containing mutations at positions 41, 75 and 77 of SEQ ID NO:22 (I-*Dmo*I moiety), different PCR reactions were carried out. The first PCR reaction, using a primer specific to the vector pCLS0542 (Gal10F 5'-GCAACTTTAGTGCTGACACATACAGG-3' (SEQ ID NO: 13)) and the primer DCaa49-37Rev (5'-TTTAATCAGGTTTTCAGACTTCTG**MNN**GATCACAACACG-3' (SEQ ID NO: 42)), which amplifies the 5' end (aa positions 1-49) of the *DmoCre*2 coding sequence. For the 3' end amplification, two PCR reactions were carried out. The first one amplifies the region between residues 42 to 74 of *DmoCre2* using the primers DCaa42-50For (5'-CAGAAGTCTGAAAACCTGATTAAACAA-3' (SEQ ID NO: 43)) and DCaa74-66Rev (5'-ACCCTTAACGATCTGGATTTTAGATTT-3' (SEQ ID NO: 44)). The second one amplifies the 3'-end (positions 68-264) of *DmoCre2* using the primer DCaa68-81For (5'-AAAATCCAGATCGTTAAGGGT**NNK**ACC**NNK**TATGAGCTGCGT-3' (SEQ ID NO: 45)) and a primer specific to the vector (pCLS0542, Figure 5) (Gal10R 5'-ACAACCTTGATTGGAGACTTGACC-3' (SEQ ID NO: 4)).

The two PCR fragments were purified and used as a template in an assembly PCR performed with the DCaa42-50For and Gal10R primers.

Then, 25ng of each of the two overlapping PCR fragments (positions 1-49 and 42-264) and overlapping 75ng of vector DNA (pCLS0542) linearized by digestion with NcoI and EagI were used to transform the yeast *Saccharomyces cerevisiae* strain FYC2-6A (MAT-α, trp1Δ63, leu2Δ1, his3Δ200) using a high efficiency LiAc transformation protocol (Gietz R D et al., Methods Enzymol. 2002; 350:87-96). An intact *DmoCre* coding was generated by *in vivo* homologous recombination in yeast. After transformation, 2232 clones were picked.

### Mating of meganuclease expressing clones and screening in yeast:

Experiments were performed as described in Example 2 above.

### Results

Using the yeast screening assay that has been described above, the 2232 clones that constitute the *DmoCre*2 D4Clib2Bis library were screened against all the 64 DC4NNN targets except for the DC4TTC target. The screen gave 335 positive clones able to cleave at least one DC4NNN target (SEQ ID NO: 36). These clones were rearranged, sequenced (221 unique sequences were isolated) and submitted to a secondary round of screening. The initial *DmoCre*2 protein is able to cleave 4 out of 63 DC4NNN targets. The D4Clib2Bis hitmap displayed in Figure 17 shows that by introducing mutations at positions 41, 75 and 77 in the *DmoCre*2 coding sequence, 32 DC4NNN targets are now being cleaved by these *DmoCre*2 derived mutants.

This number has to be compared to the 21 DC4NNN targets that were cleaved by the mutant library described in Example 3. Mutating position 41 in this screening approach has therefore allowed the inventors to widen the *DmoCre*2 cleavage spectrum for DC4NNN targets and to isolate new cleavage specificities.

### EXAMPLE 9: Making of new DmoCre derived mutants cleaving degenerated DC7NNN_P targets

To study the possibility of engineering new sequence specificities for the *DmoCre2* protein, the Applicants investigated the three adjacent nucleotides at position +5 to +7 of the C12D34 DNA target. The structure displayed in Figure 2 allowed examining closely the contacts between these three base pairs and the *DmoCre2* protein residues.

Figure 18A shows the molecular surface of the hybrid enzyme bound to its DNA target. The area of binding that has been chosen for randomization (base pairs at positions +5, +6, +7 and protein residues 37 and 81) has been highlighted. The 64 targets degenerated at position +5 to +7 are called DC7NNN (SEQ ID NO: 37). The DC7NNN target is 5' CAAAACGTCGTAAGTNNNGGCG 3' (SEQ ID NO 37), wherein NNN represent positions +5 to +7 and all combinations of A, C, G and T in these positions make up the 64 target DC7NNN sequences. Figure 18B is a zoomed in view showing the two arginine residues 37 and 81 of SEQ ID NO: 22, in interaction with the DNA. Dashed lines represent hydrogen bonds. Mutating one or two of these arginine residues leads to a sharp decrease or a complete loss of cleavage activity of DmoCre2 toward the DC7NNN targets.

A closer inspection of the structure shows that the arginine residue 37 is in hydrophobic contact with leucine residue 27 of SEQ ID NO: 22 (Figure 18C). Therefore, a mutation at position 27 could compensate for a mutation of the arginine 37.

In order to isolate new cleavage specificities for the *DmoCre2* protein, a *DmoCre2* mutant library mutated at positions 27 and 37 (D7Clib2) was built, transformed into yeast and screened using a yeast screening assay, see below, against all the 64 DC7NNN targets except for the DC7GAC.

### Material and Methods

### Construction of the 64 target vectors:

The targets were cloned as follows: oligonucleotides corresponding to each of the 64 target sequences flanked by gateway cloning sequence were ordered from Proligo:
5'TGGCATACAAGTTTTCGCCNNNACTTACGACGTTTTGACAATCGTCTGTC A-3', (SEQ ID NO: 3). Double-stranded target DNA, generated by PCR amplification of the single stranded oligonucleotide, was cloned using the Gateway protocol (Invitrogen) into yeast reporter vector (pCLS1055, Figure 4). Yeast reporter vector was transformed into *S. cerevisiae* strain FYBL2-7B (MAT-α, ura3 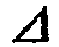 851, trp1 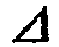 63, leu2 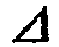 1, lys2 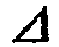 202).

### Construction of the DmoCre2 DClib2 mutant library:

In order to generate *DmoCre2* derived coding sequences containing mutations at positions 27 and 37, two separate overlapping PCR reactions were carried out that amplify the 5' end (aa positions 1-43) or the 3' end (positions 38-264) of the *DmoCre* coding sequence. For the 3' end, PCR amplification is carried out using a primer specific to the vector (pCLS0542, Figure 5) (Gal10R 5'-ACAACCTTGATTGGAGACTTGACC-3' (SEQ ID NO: 4)) and a primer specific to the *DmoCre* coding sequence for amino acids 38-46 (DC37For 5'GTTGTGATCACCCAGAAGTCTGAAAAC-3' (SEQ ID NO: 46)). For the 5' end, PCR amplification is carried out using a primer specific to the vector pCLS0542 (Gal10F 5'-GCAACTTTAGTGCTGACACATACAGG-3' (SEQ ID NO: 6)) and a primer specific to the *DmoCre* coding sequence for amino acids 23-43 (DC3727ScanRev 5'-CTTCTGGGTGATCACAAC**MNN**ATATTCGCTACGGTT ACCTTTATATTT**MNN**CTTGTACAGGCC-3' (SEQ ID NO: 47)).

The MNN code in the oligonucleotide resulting in a NNK codon at positions 27 and 37 allows the degeneracy at these positions among the 20 possible amino acids. Then, 25 ng of each of the two overlapping PCR fragments and 75 ng of overlapping vector DNA (pCLS0542) linearized by digestion with NcoI and EagI was used to transform the yeast *Saccharomyces cerevisiae* strain FYC2-6A (MAT-α, trp1Δ63, leu2Δ1, his3Δ200) using a high efficiency LiAc transformation protocol (Gietz et al., Methods Enzymol. 2002; 350:87-96).

An intact coding sequence containing both groups of mutations was generated by *in vivo* homologous recombination in yeast. The D7Clib2 nucleic diversity is 32² = 1024, after transformation, 1116 clones were picked, representing approximately the whole library diversity.

### Mating of meganuclease expressing clones and screening in yeast:

Experiments were performed as described in Example 2 above.

### Results

Using the yeast screening assay that has been described above, the 1116 clones that constitute the *DmoCre*2 D4Clib2Bis library were screened against all the 64 DC4NNN targets except for the DC4GTC target. The screen gave 174 positive clones able to cleave at least one DC7NNN target. These clones were rearranged, sequenced (75 unique sequences were isolated) and submitted to a secondary round of screening. The initial *DmoCre*2 protein was able to cleave 9 out of 63 DC7NNN targets (DC7CCC, DC7TCC, DC7ACC, DC7GCC, DC7TTC, DC7ATC, DC7TCT, DC7ACT and DC7TTT). The D7Clib2 hitmap displayed in Figure 19 shows that by introducing mutations at positions 27 and 37 in the *DmoCre*2 coding sequence, 19 DC7NNN targets are now being cleaved by *DmoCre*2 derived mutants. Our screening approach has therefore allowed us to widen the *DmoCre*2 cleavage spectrum for DC7NNN targets and to isolate new cleavage specificities.

### EXAMPLE 10: Making of new DmoCre derived mutants combining two sets of mutations and cleaving the combined DC10TGG4ACT target

The possibility of combining different sets of mutations previously isolated for the *DmoCre2* protein to cleave a combined target was investigated. First, eight DmoCre2 derived mutants mutated at residues corresponding to positions 75, 76 and 77 in wild type *I-DmoI* (SEQ ID NO: 22); and able to cleave the DC4ACT target were chosen, see Table VI for the sequence at residues corresponding to positions 75-77 in SEQ ID NO: 22; these mutants were used to create a mutant library (SeqDC10NNN4ACT) degenerated at DmoCre2 residues corresponding to amino acids positions 29 and 33 in SEQ ID NO: 22. The resulting library was finally screened in yeast against the combined DC10TGG4ACT target.

### Material and Methods

### Construction of the DC10TGG4ACT target vector:

The target was cloned as follows: an oligonucleotide corresponding to the target sequence flanked by gateway cloning sequence was ordered from Proligo: 5'TGGCATACAAGTTTTC**CCA**GGA**AGT**TACGACGTTTTGACAATCGTCTGT CA-3' SEQ ID NO: 60. Double-stranded target DNA, generated by PCR amplification of the single stranded oligonucleotide, was cloned using the Gateway protocol (Invitrogen) into yeast reporter vector (pCLS1055, Figure 4). Yeast reporter vector was transformed into *S. cerevisiae* strain FYBL2-7B (MAT α, ura3 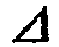 851, trp1 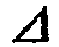 63, leu2 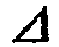 1, lys2 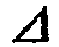 202).

### Construction of the DmoCre2 SeqDC10NNN4ACT mutant library:

First, the DNA coding for the eight DmoCre2 mutants able to cleave the DC4ACT target were pooled, Then, this DNA pool was used as a template for two separate overlapping PCR reactions in order to generate *DmoCre2* derived coding sequences containing mutations at positions 29 and 33. The first PCR reaction amplifies the 5' end of *DmoCre2* coding sequence (aa positions 1-40) using the primers Gal10F (5'-GCAACTTTAGTGCTGACACATACAGG-3' SEQ ID NO: 6) and D10CreRev2 (5'- GATCACAACACGATATTCGCT**MNN**GTTACCTTT**MNN** TTTCAGCTTGTA-3' SEQ ID NO: 61) and the second PCR reaction amplifies the 3' end (positions 34-264) of the *DmoCre2* coding sequence using the primers specific Gal10R (5'-ACAACCTTGATTGGAGACTTGACC-3' SEQ ID NO: 4) and D10CreFor2 (5'-AGC**GAA**TATCGTGTTGTGATCACCCAGAAGTCTG-3' SEQ ID NO: 62).

The MNN code in the D10CreRev2 oligonucleotide resulting in a NNK codon at positions 29 and 33 allows the degeneracy at these positions among the 20 possible amino acids. Then, 25 ng of each of the two overlapping PCR fragments and 75 ng of overlapping vector DNA (pCLS0542, Figure 5) linearized by digestion with NcoI and EagI were used to transform the yeast *Saccharomyces cerevisiae* strain FYC2-6A (MAT α, ura3Δ851, trp1Δ63, leu2Δ1, lys2Δ202) using a high efficiency LiAc transformation protocol (Gietz et al., Methods Enzymol. 2002; 350:87-96). An intact coding sequence was generated by *in vivo* homologous recombination in yeast After transformation, 2232 clones of the SeqDC10NNN4ACT library were picked.

### Mating of meganuclease expressing clones and screening in yeast:

Experiments were performed as described in Example 2 above.

### Results

Eight *DmoCre2* derived mutants able to cleave the DC4ACT target were chosen. These mutants carry mutations at residues corresponding to positions 75, 76 and 77 in SEQ ID NO: 22 and are listed in Table VI below.

**Table VI: Sequence (aa 75 to 77) of the eight DC4ACT cutters that were chosen to create the SeqDC10NNN4ACT library**

| **Mutant Name** | **SEQ ID NO** | **Sequence, aa 75 to 77** |
|---|---|---|
| Mt1-DC4ACT | 63 | RSV |
| Mt2-DC4ACT | 64 | HSC |
| Mt3-DC4ACT | 65 | NGA |
| Mt4-DC4ACT | 66 | HTS |
| Mt5-DC4ACT | 67 | RTV |
| Mt6-DC4ACT | 68 | ATN |
| Mt7-DC4ACT | 69 | CTC |
| Mt8-DC4ACT | 70 | TTV |

The SeqDC10NNN4ACT library was then screened using our yeast screening assay toward the combined DC10TGG4ACT target. The screening assay gave 11 positive clones and part of the screening is shown in Figure 20, where three positive clones are black circled. Thus, we show here that it is possible to associate mutations of residues interacting with nucleotides at positions +8 to +10 of the C12D34 target with mutations of residues interacting with nucleotides at positions +2 to +4 of the C 12D34 target in order to cleave a combined target.

### EXAMPLE 11: Making of new RAG1.10.3D34 derived mutants that cleave the RAG1.10.3DC4ACT and RAG1.10.3DC4TAT targets

Taking the refined RAG1.10.3D34 cutter described in Example 7 (Amel2_RG3D mutant SEQ ID NO: 58), a mutant library (RAG1.10.3DC4NNN) was built that degenerates the residues of Amel2_RG3D (SEQ ID NO: 58) corresponding to positions 75, 76 and 77 in wild type I-*Dmo* (SEQ ID NO: 22); in order to find potential cutters for the two following targets (Figure 14): RAG1.10.3DC4ACT (5'-CTGGCTGAGGTA**ACT**TCCGGCG-3' SEQ ID NO: 72) and RAG1.10.3DC4TAT (5'- CTGGCTGAGGTA**TAT**TCCGGCG-3' SEQ ID NO: 73).

### Material and Methods

### Construction of the RAG1.10.3DC4ACT and RAG1.10.3DC4TAT target vector:

The target was cloned as follows: an oligonucleotide corresponding to the complement of the above target sequence flanked by gateway cloning sequence was ordered from Proligo: 5'TGGCATACAAGTTTTCGCCGGAAGTTACCTCAG CCAGACAATCGTCTGTCA-3' SEQ ID NO: 74 (for the RAG1.10.3DC4ACT target) and 5'TGGCATACAAGTTTTCGCCGGAATATACCTCAGCCAGACAAT CGTCTGTCA-3' SEQ ID NO: 75 (for the RAG1.10.3DC4TAT target). Double-stranded target DNA, generated by PCR amplification of the single stranded oligonucleotide, was cloned using the Gateway protocol (Invitrogen) into yeast reporter vector (pCLS1055, Figure 4). Yeast reporter vector was transformed into *S. cerevisiae* strain FYBL2-7B (MAT *a*, ura3 Δ 851, trp1 Δ63, leu2 Δ1, lys2 Δ202).

### Construction of the RAG1.10.3DC4NNN mutant library

Using the DNA of the Amel2_RG3D (SEQ ID NO: 58) as a template, the inventors used the same protocol as described in the Example 3 for the D4Clib4 generation to build the RAG1.10.3DC4NNN mutant library. 2232 clones were picked.

### Mating of meganuclease expressing clones and screening in yeast:

Experiments were performed as described in Example 2 above.

### Results

The 2232 clones constituting the RAG1.10.3DC4NNN library were screened against the two targets RAG1.10.3DC4ACT (SEQ ID NO: 72) and RAG1.10.3DC4TAT (SEQ ID NO: 73) using our yeast screening assay. The screen yielded 68 positive clones toward the RAG1.10.3DC4ACT target (Figure 21, A) and 26 positive clones toward the RAG1.10.3DC4TAT target (Figure 21, B). The Amel2_RG3D mutant (top right dot control) did not cleave the RAG1.10.3DC4ACT and RAG1.10.3DC4TAT targets. Each positive clone was found to cleave only one of the two targets. These results show the specificity of the mutants we have obtained. This screening proves therefore that after having introduced mutation in the Cre moiety of the *DmoCre* protein (Amel2_RG3D mutant), it is possible to further engineer the protein by adding mutations in the I*-Dmo*I moiety to cleave specifically the combined target.

## Claims

1. A polypeptide, comprising the sequence of an *I-DmoI* endonuclease or a chimeric derivative thereof, including at least a variant of the first *I-DmoI* domain and **characterized in that** it comprises the substitution of at least the residues in positions 19, 20 and optionally 15, and the substitution of at least one of the residues in positions 27, 29, 33, 35, 37, 75, 76, 77 or 81 of said first *I-DmoI* domain; and
wherein said polypeptide recognises an *I-DmoI* DNA target half-site which differs from a wildtype *I-DmoI* DNA target half-site SEQ ID NO: 30, in at least one of positions ±2, ±3, ±4, ±5, ±6, ±7, ±8, ±9, ±10, the residue in position 20 is changed to serine (G20S), the isoleucine in position 19 is changed to aspartic acid (I19D) to render the first *I-DmoI* domain more active, and the *I-DmoI* domain variant cleaves at least one target that is not cleaved by the parent *I-DmoI* domain and cleaves less targets than the parent *I-DmoI* domain.

2. A polypeptide according to claim 1, wherein the leucine in position 15 is changed to glutamine (L15Q).

3. A polypeptide according to claim 1 or 2, wherein the substitution of at least one of the residues in positions 29, 33 or 35 by any amino acid, alters the recognition of said polypeptide for an *I-DmoI* DNA target half-site which differs from a wildtype *I-DmoI* DNA target half-site SEQ ID NO: 30, in at least one of positions ±8, ±9, ±10.

4. A polypeptide according to claim 1, 2 or 3, wherein the substitution of at least one of the residues in positions 75, 76 or 77 by any amino acid, alters the recognition of said polypeptide for an *I-DmoI* DNA target half-site which differs from a wildtype *I-DmoI* DNA target half-site SEQ ID NO: 30, in at least one of positions ±2 ±3, ±4.

5. A polypeptide according to claim 1, 2, 3 or 4, wherein the substitution of at least one of the residues in positions 27, 37 or 81 by any amino acid, alters the recognition of said polypeptide for an *I-DmoI* DNA target half-site which differs from a wildtype *I-DmoI* DNA target half-site SEQ ID NO: 30, in at least one of positions ±5, ±6, ±7.

6. A polypeptide according to any one of claims 1 to 5, wherein said polypeptide is a chimeric-Dmo endonuclease consisting of the fusion of said first I-*Dmo* I domain to a sequence of a dimeric LAGLIDADG homing endonuclease or to a domain of another monomeric LAGLIDADG homing endonuclease.

7. A polypeptide according to anyone of claims I to 6, **characterized in that** the first *I-DmoI* domain is at the NH₂-terminus of said chimeric-*Dmo* endonuclease.

8. A polypeptide according to claim 6, **characterized in that** said dimeric LAGLIDADG homing endonuclease is *I-CreI.*

9. A polynucleotide, **characterized in that** it is encoding the polypeptide according to anyone of claims 1 to 8.

10. A vector, **characterized in that** it comprises the polynucleotide according to claim 9.

11. A host cell, a non-human transgenic animal, or a transgenic plant **characterized in that** it is modified, or that all or part of its cells is modified by the polynucleotide according to claim 9 or the vector according to claim 10.

12. Use of a polypeptide according to anyone of claims 1 to 8, a polynucleotide according to claim 9, a vector according to claim 10, or a cell, a non-human animal, or a plant, according to claim 11, for the selection and/or the screening of meganucleases with novel DNA target specificity.

## Patentansprüche

1. Polypeptid, das die Sequenz einer *I-DmoI* Endonuklease oder eines chimären Derivats davon umfasst, das wenigstens eine Variante der ersten *I-DmoI* Domäne umfasst und **dadurch gekennzeichnet ist, dass** es die Substitution wenigstens der Reste in den Positionen 19, 20 und optional 15 und die Substitution wenigstens eines der Reste in den Positionen 27, 29, 33, 35, 75,76, 77 oder 81 der ersten *I-DmoI* Domäne umfasst; und
wobei die das Polypeptid eine *I-DmoI* DNS Halb-Zielstelle erkennt, die sich von einer Wildtyp DNS Halb-Zielstelle SEQ ID NO: 30 an wenigstens einer der Positionen ±2, ±3, ±4, ±5, ±6, ±7, ±8, ±9, ±10 unterscheidet, der Rest in Position 20 zu Serin (G20S) verändert ist, das Isoleucin in Position 19 zu Asparaginsäure verändert ist (I19D), um die erste I-*DmoI* Domäne aktiver zu machen, und die *I-DmoI* Domänenvariante wenigstens ein Ziel spaltet, das nicht von der ursprünglichen *I-DmoI* Domäne gespalten wird, und weniger Ziele spaltet, als die ursprünglichen *I-DmoI* Domäne.

2. Polypeptid nach Anspruch 1, wobei das Leucin in Position 15 zu Glutamin verändert ist (L15Q).

3. Polypeptid nach Anspruch 1 oder 2, wobei die Substitution wenigstens eines der Reste in den Positionen 29, 33 oder 35 durch irgendeine Aminosäure die Erkennung des Polypeptids für eine *I-DmoI* DNS Halb-Zielstelle verändert, die sich von einer Wildtyp-*I-DmoI* DNS Halb-Zielstelle SEQ ID NO: 30 in wenigstens einer der Positionen ±8, ±9, ±10 unterscheidet.

4. Polypeptid nach einem der Ansprüche 1, 2 oder 3, wobei die Substitution wenigstens eines der Reste in den Positionen 75, 76 oder 77 durch irgendeine Aminosäure die Erkennung des Polypeptids für eine *I-DmoI* DNS Halb-Zielstelle verändert, die sich von einer Wildtyp-*I-DmoI* DNS Halb-Zielstelle in wenigstens einer der Positionen ±2, ±3, ±4 unterscheidet.

5. Polypeptid nach einem der Ansprüche 1, 2, 3 oder 4, wobei die Substitution wenigstens eines der Reste in den Positionen 27, 37 oder 81 durch irgendeine Aminosäure die Erkennung des Polypeptids für eine *I-DmoI* DNS Halb-Zielstelle verändert, die sich von einer Wildtyp-*I-DmoI* DNS Halb-Zielstelle SEQ ID NO: 30 in wenigstens einer der Positionen ±5, ±6, ±7 unterscheidet.

6. Polypeptid nach einem der Ansprüche 1 bis 5, wobei das Polypeptid eine chimäre *I-DmoI* Endonuklease ist, die aus der Verbindung der ersten *I-DmoI* Domäne mit einer Sequenz einer dimeren LAGLIDADG Homing Endonuklease oder mit einer Domäne einer anderen monomeren LAGLIDADG Homing Endonuklease besteht.

7. Polypeptid nach einem der Ansprüche 1 bis 6, **dadurch gekennzeichnet, dass** die erste *I-DmoI* Domäne sich am NH₂-Terminus der chimären *Dmo* Endonuklease befindet.

8. Polypeptid nach Anspruch 6, **dadurch gekennzeichnet, dass** die dimere LAGLIDADG Homing Endonuklease *I-CreI* ist.e

9. Polynukleotid, **dadurch gekennzeichnet, dass** es für das Polypeptid nach einem der Ansprüche 1 bis 8 kodiert.

10. Vektor, **dadurch gekennzeichnet, dass** er das Polynukleotid nach Anspruch 9 umfasst.

11. Wirtszelle, nicht humanes transgenes Tier oder transgene Pflanze, **dadurch gekennzeichnet, dass** sie/es verändert ist, oder dass alle oder ein Teil seiner/ihrer Zellen durch das Polynukleotid nach Anspruch 9 oder den Vektor nach Anspruch 10 verändert sind.

12. Verwendung eines Polypeptids nach einem der Ansprüche 1 bis 8, eines Polynukleotids nach Anspruch 9, eines Vektors nach Anspruch 10 oder einer Zelle, eines nicht humanen Tiers oder einer Pflanze, nach Anspruch 11, für die Selektion und/oder das Screening von Meganukleasen mit neuer DNS Zielspezifität.

## Revendications

1. Polypeptide, comprenant la séquence d'une endonucléase *I-DmoI* ou d'un dérivé chimérique de celle-ci, incluant au moins un variant du premier domaine de *I-DmoI* et **caractérisé en ce qu'**il comprend la substitution au moins des résidus en positions 19, 20 et éventuellement 15, et la substitution au moins d'un des résidus en positions 27, 29, 33, 35, 37, 75, 76, 77 ou 81 dudit premier domaine de *I-DmoI* ; et
dans lequel ledit polypeptide reconnait un hémi-site cible d'ADN de *I-DmoI* qui diffère d'un hémi-site cible d'ADN de *I-DmoI* du type sauvage SEQ ID NO: 30, par au moins une des positions ±2, ±3, ±4, ±5, ±6, ±7, ±8, ±9, ±10, le résidu en position 20 est remplacé par une serine (G20S), l'isoleucine en position 19 est remplacée par un acide aspartique (I19D) pour rendre le premier domaine de *I-DmoI* plus actif, et le variant du domaine de *I-DmoI* clive au moins une cible qui n'est pas clivée par le domaine de *I-DmoI* parental et clive moins de cibles que le domaine de *I-DmoI* parental.

2. Polypeptide selon la revendication 1, dans lequel la leucine en position 15 est remplacée par une glutamine (L15Q).

3. Polypeptide selon la revendication 1 ou 2, dans lequel la substitution d'au moins un des résidus en positions 29, 33 ou 35 par n'importe quel acide aminé, modifie la reconnaissance dudit polypeptide pour un hémi-site cible d'ADN de *I-DmoI* qui diffère d'un hémi-site cible d'ADN de *I-DmoI* du type sauvage SEQ ID NO: 30, par au moins une des positions ±8, ±9, ±10.

4. Polypeptide selon la revendication 1, 2 ou 3, dans lequel la substitution d'au moins un des résidus en positions 75, 76 ou 77 par n'importe quel acide aminé, modifie la reconnaissance dudit polypeptide pour un hémi-site cible d'ADN de *I-DmoI* qui diffère d'un hémi-site cible d'ADN de *I-DmoI* du type sauvage SEQ ID NO: 30, par au moins une des positions ±2 ±3, ±4.

5. Polypeptide selon la revendication 1, 2, 3 ou 4, dans lequel la substitution d'au moins un des résidus en positions 27, 37 ou 81 par n'importe quel acide aminé, modifie la reconnaissance dudit polypeptide pour un hémi-site cible d'ADN de *I-DmoI* qui diffère d'un hémi-site cible d'ADN de *I-DmoI* du type sauvage SEQ ID NO: 30, par au moins une des positions ±5, ±6, ±7.

6. Polypeptide selon l'une des revendications 1 à 5, dans lequel ledit polypeptide est une endonucléase *Dmo* chimérique constituée de la fusion dudit premier domaine de *I-Dmo* I à une séquence d'une endonucléase LAGLIDADG dimérique de type homing ou au domaine d'une autre endonucléase LAGLIDADG monomérique de type homing.

7. Polypeptide selon l'une des revendications 1 à 6, **caractérisé en ce que** le premier domaine de *I-DmoI* est à l'extrémité NH₂-terminale de ladite endonucléase *Dmo* chimérique.

8. Polypeptide selon la revendication 6, **caractérisé en ce que** ladite endonucléase LAGLIDADG dimérique de type homing est *I-CreI.*

9. Polynucléotide, **caractérisé en ce qu'**il code le polypeptide selon l'une des revendications 1 à 8.

10. Vecteur, **caractérisé en ce qu'**il comprend le polynucléotide selon la revendication 9.

11. Cellule hôte, animal transgénique non humain, ou plante transgénique **caractérisé en ce qu'**il est modifié, ou que tout ou partie de ses cellules est modifié par le polynucléotide selon la revendication 9 ou le vecteur selon la revendication 10.

12. Utilisation d'un polypeptide selon l'une des revendications 1 à 8, d'un polynucléotide selon la revendication 9, d'un vecteur selon la revendication 10, ou d'une cellule, d'un animal non humain ou d'une plante, selon la revendication 11, pour la sélection et/ou le criblage de méganucléases ayant une nouvelle spécificité de cible d'ADN.


### SEQUENCE LISTING

<110> CELLECTIS GRIZOT Sylvestre DUCHATEAU Philippe
<120> Improved chimeric Meganuclease Enzymes and uses thereof
<130> BLO F1546/21
<160> 83
<170> PatentIn version 3.3
<210> 1
   <211> 110
   <212> PRT
   <213> Artificial
<220>
   <223> I-DmoI domain
<400> 1
   Met Val His Asn Asn Glu Asn Val Ser Gly Ile Ser Ala Tyr Leu Leu
   1               5                   10                  15
   Gly Leu Ile Ile Gly Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Ile Ala Phe
               100                 105                 110
<210> 2
   <211> 264
   <212> PRT
   <213> Artificial
<220>
   <223> DmoCre2
<400> 2
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
                   20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Asn
           115                 120                 125
   Gln Ser Tyr Lys Phe Lys His Gln Leu Ser Leu Thr Phe Gln Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Arg Asp Arg Gly Ser Val Ser Asp Tyr Ile Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Glu
           195                 200                 205
   Gln Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro Ala Ala Asp
               260
<210> 3
   <211> 51
   <212> DNA
   <213> Artificial
<220>
   <223> Cloning oligonucleotide 1
<220>
   <221> misc_feature
   <222> (17)..(19)
   <223> n is a, c, g, or t
<400> 3
   tggcatacaa gttttcnnng gaacttacga cgttttgaca atcgtctgtc a 51
<210> 4
   <211> 24
   <212> DNA
   <213> Artificial
<220>
   <223> PCR primer 1
<400> 4
   acaaccttga ttggagactt gacc 24
<210> 5
   <211> 33
   <212> DNA
   <213> Artificial
<220>
   <223> PCR Primer 2
<400> 5
   tatcgtgttg tgatcaccca gaagtctgaa aac 33
<210> 6
   <211> 26
   <212> DNA
   <213> Artificial
<220>
   <223> PCR Primer 3
<400> 6
   gcaactttag tgctgacaca tacagg 26
<210> 7
   <211> 63
   <212> DNA
   <213> Artificial
<220>
   <223> PCR Primer 4
<220>
   <221> misc_feature
   <222> (26)..(27)
   <223> n is a, c, g, or t
<220>
   <221> misc_feature
   <222> (32)..(33)
   <223> n is a, c, g, or t
<220>
   <221> misc_feature
   <222> (44)..(45)
   <223> n is a, c, g, or t
<400> 7
   cttctgggtg atcacaacac gatamnngct mnngttacct ttmnntttca gcttgtacag 60
   gcc 63
<210> 8
   <211> 22
   <212> DNA
   <213> Artificial
<220>
   <223> DC10NNN target
<220>
   <221> misc_feature
   <222> (19)..(21)
   <223> n is a, c, g, or t
<400> 8
   caaaacgtcg taagttccnn ng 22
<210> 9
   <211> 264
   <212> PRT
   <213> Artificial
<220>
   <223> DmoCre4
<400> 9
   Met Val His Asn Asn Glu Asn Val Ser Gly Ile Ser Ala Tyr Leu Leu
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Tyr Leu Ala
   100 105 110
   Gly Phe Val Asp Gly Asp Gly Ser Ile Ile Ala Gln Ile Lys Pro Asn
   115 120 125
   Gln Ser Tyr Lys Phe Lys His Gln Leu Ser Leu Thr Phe Gln Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Arg Gly Ser Val Ser Asp Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 10
   <211> 51
   <212> DNA
   <213> Artificial
<220>
   <223> DC4NNN cloning oligonucleotide
<220>
   <221> misc_feature
   <222> (23)..(25)
   <223> n is a, c, g, or t
<400> 10
   tggcatacaa gttttcgccg gannntacga cgttttgaca atcgtctgtc a 51
<210> 11
   <211> 24
   <212> DNA
   <213> Artificial
<220>
   <223> Gal10R PCR primer
<400> 11
   acaaccttga ttggagactt gacc 24
<210> 12
   <211> 54
   <212> DNA
   <213> Artificial
<220>
   <223> DClib4For PCR Primer
<220>
   <221> misc_feature
   <222> (28)..(29)
   <223> n is a, c, g, or t
<220>
   <221> misc_feature
   <222> (31)..(32)
   <223> n is a, c, g, or t
<220>
   <221> misc_feature
   <222> (34)..(35)
   <223> n is a, c, g, or t
<400> 12
   aaatctaaaa tccagatcgt taagggtnnk nnknnktatg agctgcgtgt gagc 54
<210> 13
   <211> 26
   <212> DNA
   <213> Artificial
<220>
   <223> Gal10F PCR Primer
<400> 13
   gcaactttag tgctgacaca tacagg 26
<210> 14
   <211> 27
   <212> DNA
   <213> Artificial
<220>
   <223> DClib4Rev PCR Primer
<400> 14
   acccttaacg atctggattt tagattt 27
<210> 15
   <211> 51
   <212> DNA
   <213> Artificial
<220>
   <223> SCAGD34 cloning oligonucleotide
<400> 15
   tggcatacaa gttttcgccg gaacttacct ggttttgaca atcgtctgtc a 51
<210> 16
   <211> 42
   <212> DNA
   <213> Artificial
<220>
   <223> CreNgoLib PCR Primer
<400> 16
   cgtgagcagc tggcgttcct ggccggcttt gtggacggtg ac 42
<210> 17
   <211> 35
   <212> DNA
   <213> Artificial
<220>
   <223> CreMluLib PCR Primer
<400> 17
   acgaacggtt tcagaagtgg ttttacgcgt cttag 35
<210> 18
   <211> 42
   <212> DNA
   <213> Artificial
<220>
   <223> CreNgoLibY PCR Primer
<400> 18
   cgtgagcagc tggcgtacct ggccggcttt gtggacggtg ac 42
<210> 19
   <211> 51
   <212> DNA
   <213> Artificial
<220>
   <223> RAG1.10.2034 cloning oligonucleotide
<400> 19
   tggcatacaa gttttcgccg gaacttacct gagaacaaca atcgtctgtc a 51
<210> 20
   <211> 28
   <212> DNA
   <213> Artificial
<220>
   <223> CreNgoFor PCR Primer
<400> 20
   ttcctgctgt acctggccgg ctttgtgg 28
<210> 21
   <211> 26
   <212> DNA
   <213> Artificial
<220>
   <223> CreMluRev PCR Primer
<400> 21
   ttcagaagtg gttttacgcg tcttag 26
<210> 22
   <211> 194
   <212> PRT
   <213> Desulfurococcus mobilis
<400> 22
   Met His Asn Asn Glu Asn Val Ser Gly Ile Ser Ala Tyr Leu Leu Gly
   1 5 10 15
   Leu Ile Ile Gly Asp Gly Gly Leu Tyr Lys Leu Lys Tyr Lys Gly Asn
   20 25 30
   Arg Ser Glu Tyr Arg Val Val Ile Thr Gln Lys Ser Glu Asn Leu Ile
   35 40 45
   Lys Gln His Ile Ala Pro Leu Met Gln Phe Leu Ile Asp Glu Leu Asn
   50 55 60
   Val Lys Ser Lys Ile Gln Ile Val Lys Gly Asp Thr Arg Tyr Glu Leu
   65 70 75 80
   Arg Val Ser Ser Lys Lys Leu Tyr Tyr Tyr Phe Ala Asn Met Leu Glu
   85 90 95
   Arg Ile Arg Leu Phe Asn Met Arg Glu Gln Ile Ala Phe Ile Lys Gly
   100 105 110
   Leu Tyr Val Ala Glu Gly Asp Lys Thr Leu Lys Arg Leu Arg Ile Trp
   115 120 125
   Asn Lys Asn Lys Ala Leu Leu Glu Ile Val Ser Arg Trp Leu Asn Asn
   130 135 140
   Leu Gly Val Arg Asn Thr Ile His Leu Asp Asp His Arg His Gly Val
   145 150 155 160
   Tyr Val Leu Asn Ile Ser Leu Arg Asp Arg Ile Lys Phe Val His Thr
   165 170 175
   Ile Leu Ser Ser His Leu Asn Pro Leu Pro Pro Glu Arg Ala Gly Gly
   180 185 190
   Tyr Thr
<210> 23
   <211> 24
   <212> DNA
   <213> Chlamydomonas reinhardtii
<400> 23
   tcaaaacgtc gtacgacgtt ttga 24
<210> 24
   <211> 163
   <212> PRT
   <213> chlamydomonas reinhardtii
<400> 24
   Met Asn Thr Lys Tyr Asn Lys Glu Phe Leu Leu Tyr Leu Ala Gly Phe
   1 5 10 15
   Val Asp Gly Asp Gly Ser Ile Ile Ala Gln Ile Lys Pro Asn Gln Ser
   20 25 30
   Tyr Lys Phe Lys His Gln Leu Ser Leu Ala Phe Gln Val Thr Gln Lys
   35 40 45
   Thr Gln Arg Arg Trp Phe Leu Asp Lys Leu Val Asp Glu Ile Gly Val
   50 55 60
   Gly Tyr Val Arg Asp Arg Gly Ser Val Ser Asp Tyr Ile Leu Ser Glu
   65 70 75 80
   Ile Lys Pro Leu His Asn Phe Leu Thr Gln Leu Gln Pro Phe Leu Lys
   85 90 95
   Leu Lys Gln Lys Gln Ala Asn Leu Val Leu Lys Ile Ile Trp Arg Leu
   100 105 110
   Pro Ser Ala Lys Glu Ser Pro Asp Lys Phe Leu Glu Val Cys Thr Trp
   115 120 125
   Val Asp Gln Ile Ala Ala Leu Asn Asp Ser Lys Thr Arg Lys Thr Thr
   130 135 140
   Ser Glu Thr Val Arg Ala Val Leu Asp Ser Leu Ser Glu Lys Lys Lys
   145 150 155 160
   Ser Ser Pro
<210> 25
   <211> 11
   <212> DNA
   <213> Artificial
<220>
   <223> Degenerate X-DmoI target half site
<220>
   <221> misc_feature
   <222> (2)..(4)
   <223> n is a, c, g, or t
<220>
   <221> misc_feature
   <222> (8)..(10)
   <223> n is a, c, g, or t
<400> 25
   annntccnnn g 11
<210> 26
   <211> 11
   <212> DNA
   <213> Desulfurococcus mobilis
<400> 26
   aagttccggc g 11
<210> 27
   <211> 110
   <212> PRT
   <213> Artificial
<220>
   <223> I-DmoI domain from DmoCre4
<400> 27
   Met Val His Asn Asn Glu Asn Val Ser Gly Ile Ser Ala Tyr Leu Leu
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Tyr
   100 105 110
<210> 28
   <211> 22
   <212> DNA
   <213> Artificial
<220>
   <223> C1234 target site
<400> 28 22
   caaaacgtcg tgagacagtt tc 22
<210> 29
   <211> 22
   <212> DNA
   <213> Artificial
<220>
   <223> C1221 target site
<400> 29
   caaaacgtcg tacgacgttt tg 22
<210> 30
   <211> 22
   <212> DNA
   <213> Artificial
<220>
   <223> D1234 target sequence
<400> 30
   cgttgccggg taagttccgg cg 22
<210> 31
   <211> 22
   <212> DNA
   <213> Artificial
<220>
   <223> C12D34 target sequence
<400> 31
   caaaacgtcg taagttccgg cg 22
<210> 32
   <211> 22
   <212> DNA
   <213> Artificial
<220>
   <223> 5CAG_P target sequence
<400> 32
   caaaaccagg tacctggttt tg 22
<210> 33
   <211> 22
   <212> DNA
   <213> Artificial
<220>
   <223> 5CAGD34 target sequence
<400> 33
   caaaaccagg taagttccgg cg 22
<210> 34
   <211> 22
   <212> DNA
   <213> Artificial
<220>
   <223> RAG1.10.2 target sequence
<400> 34
   tgttctcagg tacctgagaa ca 22
<210> 35
   <211> 22
   <212> DNA
   <213> Artificial
<220>
   <223> RAG1.10.2D34
<400> 35
   tgttctcagg taagttccgg cg 22
<210> 36
   <211> 22
   <212> DNA
   <213> Artificial
<220>
   <223> DC4NNN target
<220>
   <221> misc_feature
   <222> (13)..(15)
   <223> n is a, c, g, or t
<400> 36
   caaaacgtcg tannntccgg cg 22
<210> 37
   <211> 22
   <212> DNA
   <213> Artificial
<220>
   <223> DC7NNN target sequence
<220>
   <221> misc_feature
   <222> (16)..(18)
   <223> n is a, c, g, or t
<400> 37
   caaaacgtcg taagtnnngg cg 22
<210> 38
   <211> 22
   <212> DNA
   <213> Artificial
<220>
   <223> RAG1.10.3 target
<400> 38
   ttggctgagg tacctcagcc aa 22
<210> 39
   <211> 22
   <212> DNA
   <213> Artificial
<220>
   <223> RAG1-10.3D34 target
<400> 39
   ttggctgagg taagttccgg cg 22
<210> 40
   <211> 59
   <212> DNA
   <213> Artificial
<220>
   <223> preATGCreFor primer
<400> 40
   gcataaatta ctatacttct atagacacgc aaacacaaat acacagcggc cttgccacc 59
<210> 41
   <211> 48
   <212> DNA
   <213> Artificial
<220>
   <223> IcreIpostRev primer
<400> 41
   ggctcgagga gctcgtctag aggatcgctc gagttatcag tcggccgc 48
<210> 42
   <211> 39
   <212> DNA
   <213> Artificial
<220>
   <223> DCaa49-37Rev primer
<220>
   <221> misc_feature
   <222> (26)..(27)
   <223> n is a, c, g, or t
<400> 42
   tttaatcagg ttttcagact tctgmnngat cacaacacg 39
<210> 43
   <211> 27
   <212> DNA
   <213> Artificial
<220>
   <223> DCaa42-50For primer
<400> 43
   cagaagtctg aaaacctgat taaacaa 27
<210> 44
   <211> 27
   <212> DNA
   <213> Artificial
<220>
   <223> DCaa74-66Rev primer
<400> 44
   acccttaacg atctggattt tagattt 27
<210> 45
   <211> 42
   <212> DNA
   <213> Artificial
<220>
   <223> DCaa68-81For primer
<220>
   <221> misc_feature
   <222> (22)..(23)
   <223> n is a, c, g, or t
<220>
   <221> misc_feature
   <222> (28)..(29)
   <223> n is a, c, g, or t
<400> 45
   aaaatccaga tcgttaaggg tnnkaccnnk tatgagctgc gt 42
<210> 46
   <211> 27
   <212> DNA
   <213> Artificial
<220>
   <223> DC37For primer
<400> 46
   gttgtgatca cccagaagtc tgaaaac 27
<210> 47
   <211> 63
   <212> DNA
   <213> Artificial
<220>
   <223> DC3727ScanRev primer
<220>
   <221> misc_feature
   <222> (20)..(21)
   <223> n is a, c, g, or t
<220>
   <221> misc_feature
   <222> (50)..(51)
   <223> n is a, c, g, or t
<400> 47
   cttctgggtg atcacaacmn natattcgct acggttacct ttatatttmn ncttgtacag 60
   gcc 63
<210> 48
   <211> 264
   <212> PRT
   <213> Artificial
<220>
   <223> RG2D1 mutant
<400> 48
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Lys
           115                 120                 125
   Gln Ser Ala Lys Phe Lys His Gln Leu Ser Leu Thr Phe Ala Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Tyr Asp Ser Gly Ser Val Ser Tyr Tyr Lys Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Glu
           195                 200                 205
   Gln Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
   245                 250                 255
   Lys Lys Ser Ser Pro Ala Ala Asp
               260
<210> 49
   <211> 264
   <212> PRT
   <213> Artificial
<220>
   <223> RG2D2 mutant
<400> 49
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Arg
           115                 120                 125
   Gln Ser Cys Lys Phe Lys His Gln Leu Ser Leu Thr Phe Ala Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Tyr Asp Ser Gly Pro Val Ser Tyr Tyr Lys Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Glu
           195                 200                 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 50
   <211> 264
   <212> PRT
   <213> Artificial
<220>
   <223> RG2D3 mutant
<400> 50
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Arg
   115 120 125
   Gln Ser Cys Lys Phe Lys His Gln Leu Ser Leu Thr Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Tyr Asp Ser Gly Ser Val Ser Asp Tyr Arg Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 51
   <211> 264
   <212> PRT
   <213> Artificial
<220>
   <223> RG3D1 mutant
<400> 51
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Gln Pro Asn
   115 120 125
   Gln Ser Ser Lys Phe Lys His Arg Leu Arg Leu Thr Phe Thr Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Ser Gly Ser Val Ser Tyr Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 52
   <211> 264
   <212> PRT
   <213> Artificial
<220>
   <223> RG3D2 mutant
<400> 52
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Asn Pro Asn
   115 120 125
   Gln Ser Ser Lys Phe Lys His Arg Leu Arg Leu Thr Phe Tyr Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Ser Gly Ser Val Ser Gln Tyr Val Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 53
   <211> 264
   <212> PRT
   <213> Artificial
<220>
   <223> RG3D3 mutant
<400> 53
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Asn Pro Asn
   115 120 125
   Gln Ser Ser Lys Phe Lys His Arg Leu Arg Leu Thr Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Asn Gly Ser Val Ser Asn Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 54
   <211> 264
   <212> PRT
   <213> Artificial
<220>
   <223> Amel1_RG2D mutant
<400> 54
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Lys
           115                 120                 125
   Gln Ser Ala Lys Phe Lys His Gln Leu Ser Leu Thr Phe Ala Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Tyr Asp Ser Gly Ser Val Ser Tyr Tyr Lys Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Ala Leu Lys Ile Ile Glu
           195                 200                 205
   Gln Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro Ala Ala Asp
               260
<210> 55
   <211> 264
   <212> PRT
   <213> Artificial
<220>
   <223> Amel2_RG2D mutant
<400> 55
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Arg
           115                 120                 125
   Gln Ser Cys Lys Phe Lys His Gln Leu Ser Leu Thr Phe Ala Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Tyr Asp Ser Gly Pro Val Ser Tyr Tyr Lys Leu
                   165                 170                 175
   Ser Lys Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Glu
           195                 200                 205
   Gln Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro Ala Ala Asp
               260
<210> 56
   <211> 264
   <212> PRT
   <213> Artificial
<220>
   <223> Amel3_RG2D mutant
<400> 56
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Arg
   115 120 125
   Gln Ser Cys Lys Phe Lys His Gln Leu Ser Leu Thr Phe Ala Val Thr
   130 135 140
   Gln Lys Thr Gln Arg Arg Trp Phe Leu Asp Lys Leu Val Asp Glu Ile
   145 150 155 160
   Gly Val Gly His Val Tyr Asp Ser Gly Ser Val Ser Asp Tyr Arg Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 57
   <211> 264
   <212> PRT
   <213> Artificial
<220>
   <223> Amel1_RG3D mutant
<400> 57
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Gln Pro Asn
   115 120 125
   Gln Ser Ser Lys Phe Lys His Arg Leu Arg Leu Thr Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Ser Gly Ser Val Ser Tyr Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 58
   <211> 264
   <212> PRT
   <213> Artificial
<220>
   <223> Amel2_RG3D mutant
<400> 58
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Gln Pro Asn
   115 120 125
   Gln Ser Ser Lys Phe Lys His Arg Leu Arg Leu Thr Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Asn Gly Ser Val Ser Gln Tyr Val Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 59
   <211> 264
   <212> PRT
   <213> Artificial
<220>
   <223> Amel3_RG3D mutant
<400> 59
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Gln Pro Asn
   115 120 125
   Gln Ser Ser Lys Phe Lys His Arg Leu Arg Leu Thr Phe Tyr Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Ser Gly Ser Val Ser Gln Tyr Val Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 60
   <211> 51
   <212> DNA
   <213> Artificial
<220>
   <223> cloning oligonucleotide ex10
<400> 60
   tggcatacaa gttttcccag gaagttacga cgttttgaca atcgtctgtc a 51
<210> 61
   <211> 48
   <212> DNA
   <213> Artificial
<220>
   <223> D10CreRev2
<220>
   <221> misc_feature
   <222> (23)..(24)
   <223> n is a, c, g, or t
<220>
   <221> misc_feature
   <222> (35)..(36)
   <223> n is a, c, g, or t
<400> 61
   gatcacaaca cgatattcgc tmnngttacc tttmnntttc agcttgta 48
<210> 62
   <211> 34
   <212> DNA
   <213> Artificial
<220>
   <223> D10CreFor2
<400> 62
   agcgaatatc gtgttgtgat cacccagaag tctg 34
<210> 63
   <211> 264
   <212> PRT
   <213> Artificial
<220>
   <223> Mt1-DC4ACT
<400> 63
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Arg Ser Val Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Asn
           115                 120                 125
   Gln Ser Tyr Lys Phe Lys His Gln Leu Ser Leu Thr Phe Gln Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Arg Gly Ser Val Ser Asp Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 64
   <211> 264
   <212> PRT
   <213> Artificial
<220>
   <223> Mt2-DC4ACT
<400> 64
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly His Ser Cys Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Asn
   115 120 125
   Gln Ser Tyr Lys Phe Lys His Gln Leu Ser Leu Thr Phe Gln Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Arg Gly Ser Val Ser Asp Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 65
   <211> 264
   <212> PRT
   <213> Artificial
<220>
   <223> Mt3-DC4ACT
<400> 65
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asn Gly Ala Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Asn
   115 120 125
   Gln Ser Tyr Lys Phe Lys His Gln Leu Ser Leu Thr Phe Gln Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Arg Gly Ser Val Ser Asp Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 66
   <211> 264
   <212> PRT
   <213> Artificial
<220>
   <223> Mt4-DC4ACT
<400> 66
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly His Thr Ser Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Asn
   115 120 125
   Gln Ser Tyr Lys Phe Lys His Gln Leu Ser Leu Thr Phe Gln Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Arg Gly Ser Val Ser Asp Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 67
   <211> 264
   <212> PRT
   <213> Artificial
<220>
   <223> Mt5-DC4ACT
<400> 67
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Arg Thr Val Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Asn
           115                 120                 125
   Gln Ser Tyr Lys Phe Lys His Gln Leu Ser Leu Thr Phe Gln Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Arg Asp Arg Gly Ser Val Ser Asp Tyr Ile Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Glu
           195                 200                 205
   Gln Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro Ala Ala Asp
               260
<210> 68
   <211> 264
   <212> PRT
   <213> Artificial
<220>
   <223> Mt6-DC4ACT
<400> 68
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Ala Thr Asn Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Asn
           115                 120                 125
   Gln Ser Tyr Lys Phe Lys His Gln Leu Ser Leu Thr Phe Gln Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Arg Asp Arg Gly Ser Val Ser Asp Tyr Ile Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Glu
           195                 200                 205
   Gln Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro Ala Ala Asp
               260
<210> 69
   <211> 264
   <212> PRT
   <213> Artificial
<220>
   <223> Mt7-DC4ACT
<400> 69
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Cys Thr Cys Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Asn
           115                 120                 125
   Gln Ser Tyr Lys Phe Lys His Gln Leu Ser Leu Thr Phe Gln Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Arg Asp Arg Gly Ser Val Ser Asp Tyr Ile Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Glu
           195                 200                 205
   Gln Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro Ala Ala Asp
               260
<210> 70
   <211> 264
   <212> PRT
   <213> Artificial
<220>
   <223> Mt8-DC4ACT
<400> 70
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Thr Thr Val Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Asn
   115 120 125
   Gln Ser Tyr Lys Phe Lys His Gln Leu Ser Leu Thr Phe Gln Val Thr
   130 135 140
   Gln Lys Thr Gln Arg Arg Trp Phe Leu Asp Lys Leu Val Asp Glu Ile
   145 150 155 160
   Gly Val Gly Tyr Val Arg Asp Arg Gly Ser Val Ser Asp Tyr Ile Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 71
   <211> 264
   <212> PRT
   <213> Artificial
<220>
   <223> DmoM2
<400> 71
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Arg
   115 120 125
   Gln Ser Asn Lys Phe Lys His Gln Leu Ser Leu Thr Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Tyr Asp Ser Gly Ser Val Ser Asp Tyr Arg Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Glu
   195 200 205
   Gln Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro Ala Ala Asp
   260
<210> 72
   <211> 22
   <212> DNA
   <213> Artificial
<220>
   <223> RAG1.10.3DC4ACT
<400> 72
   ctggctgagg taacttccgg cg 22
<210> 73
   <211> 22
   <212> DNA
   <213> Artificial
<220>
   <223> RAG1.10.3DC4TAT
<400> 73
   ctggctgagg tatattccgg cg 22
<210> 74
   <211> 51
   <212> DNA
   <213> Artificial
<220>
   <223> RAG1.10.3DC4ACT + gateway
<400> 74
   tggcatacaa gttttcgccg gaagttacct cagccagaca atcgtctgtc a 51
<210> 75
   <211> 51
   <212> DNA
   <213> Artificial
<220>
   <223> RAG1.10.3DC4TAT + gateway
<400> 75
   tggcatacaa gttttcgccg gaatatacct cagccagaca atcgtctgtc a 51
<210> 76
   <211> 261
   <212> PRT
   <213> Artificial
<220>
   <223> RG2D4 mutant
<400> 76
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Asn
   115 120 125
   Gln Ser Arg Lys Phe Lys His Thr Leu Ser Leu Ala Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Tyr Asp Ser Gly Ser Val Ser Tyr Tyr Lys Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Trp
   195 200 205
   Arg Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro
   260
<210> 77
   <211> 261
   <212> PRT
   <213> Artificial
<220>
   <223> RG2D5 mutant
<400> 77
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Arg
   115 120 125
   Gln Ser Cys Lys Phe Lys His Gln Leu Ser Leu Ala Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Tyr Asp Ser Gly Ser Val Ser Tyr Tyr Val Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Trp
   195 200 205
   Arg Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro
   260
<210> 78
   <211> 261
   <212> PRT
   <213> Artificial
<220>
   <223> RG2D6 mutant
<400> 78
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Lys
           115                 120                 125
   Gln Ser Ser Lys Phe Lys His Gln Leu Ser Leu Ala Phe Ala Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Tyr Asp Ser Gly Ser Val Ser Tyr Tyr Lys Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Trp
           195                 200                 205
   Arg Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro
               260
<210> 79
   <211> 261
   <212> PRT
   <213> Artificial
<220>
   <223> RG2D7 mutant
<400> 79
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Lys Pro Arg
   115 120 125
   Gln Ser Asn Lys Phe Lys His Gln Leu Ser Leu Ala Phe Thr Val Thr
   130 135 140
   Gln Lys Thr Gln Arg Arg Trp Phe Leu Asp Lys Leu Val Asp Glu Ile
   145 150 155 160
   Gly Val Gly Tyr Val Tyr Asp Ser Gly Ser Val Ser Tyr Tyr Arg Leu
   165 170 175
   Ser Glu Ile Lys Pro Leu His Asn Phe Leu Thr Gln Leu Gln Pro Phe
   180 185 190
   Leu Lys Leu Lys Gln Lys Gln Ala Asn Leu Val Leu Lys Ile Ile Trp
   195 200 205
   Arg Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro
   260
<210> 80
   <211> 261
   <212> PRT
   <213> Artifical
<400> 80
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Lys Pro Arg
           115                 120                 125
   Gln Ser Tyr Lys Phe Lys His Gln Leu Ser Leu Ala Phe Ala Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Tyr Asp Ser Gly Ser Val Ser Tyr Tyr Lys Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Trp
           195                 200                 205
   Arg Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro
               260
<210> 81
   <211> 261
   <212> PRT
   <213> Artificial
<220>
   <223> RG3D4 mutant
<400> 81
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Asn Pro Asn
           115                 120                 125
   Gln Ser Ser Lys Phe Lys His Arg Leu Arg Leu Ala Phe Thr Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val Arg Asp Ser Gly Ser Val Ser Tyr Tyr Ile Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Trp
   Arg Leu Pro Ser Ala Lys Glu Ser Pro Asp Lys Phe Leu Glu Val Cys
   210 215 220
   Thr Trp Val Asp Gln Ile Ala Ala Leu Asn Asp Ser Lys Thr Arg Lys
   225 230 235 240
   Thr Thr Ser Glu Thr Val Arg Ala Val Leu Asp Ser Leu Ser Glu Lys
   245 250 255
   Lys Lys Ser Ser Pro
   260
<210> 82
   <211> 261
   <212> PRT
   <213> Artificial
<220>
   <223> RG3D5 mutant
<400> 82
   Met Val His Asn Asn Glu Asn Val Ser Gly Ile Ser Ala Tyr Leu Gln
   1 5 10 15
   Gly Leu Ile Asp Ser Asp Gly Gly Leu Tyr Lys Leu Lys Tyr Lys Gly
   20 25 30
   Asn Arg Ser Glu Tyr Arg Val Val Ile Thr Gln Lys Ser Glu Asn Leu
   35 40 45
   Ile Lys Gln His Ile Ala Pro Leu Met Gln Phe Leu Ile Asp Glu Leu
   50 55 60
   Asn Val Lys Ser Lys Ile Gln Ile Val Lys Gly Asp Thr Arg Tyr Glu
   65 70 75 80
   Leu Arg Val Ser Ser Lys Lys Leu Tyr Tyr Tyr Phe Ala Asn Met Leu
   85 90 95
   Glu Arg Ile Arg Leu Phe Asn Met Arg Glu Gln Leu Ala Phe Leu Ala
   100 105 110
   Gly Phe Val Asp Gly Asp Gly Ser Ile Ile Ala Gln Ile Ala Pro Asn
   115 120 125
   Gln Ser Ser Lys Phe Lys His Arg Leu Lys Leu Ala Phe Ala Val Thr
   130 135 140
   Gln Lys Thr Gln Arg Arg Trp Phe Leu Asp Lys Leu Val Asp Glu Ile
   145 150 155 160
   Gly Val Gly Tyr Val Ala Asp Asn Gly Ser Val Ser Asn Tyr Ile Leu
                   165                 170                 175
   Ser Glu Ile Lys Pro Leu His Asn Phe Leu Thr Gln Leu Gln Pro Phe
               180                 185                 190
   Leu Lys Leu Lys Gln Lys Gln Ala Asn Leu Val Leu Lys Ile Ile Trp
           195                 200                 205
   Arg Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro
               260
<210> 83
   <211> 261
   <212> PRT
   <213> Artificial
<220>
   <223> RG3D6 mutant
<400> 83
   Met Val His Asn Asn Glu Asn Val Ser Gly Ile Ser Ala Tyr Leu Gln
   1               5                   10                  15
   Gly Leu Ile Asp Ser Asp Gly Gly Leu Tyr Lys Leu Lys Tyr Lys Gly
               20                  25                  30
   Asn Arg Ser Glu Tyr Arg Val Val Ile Thr Gln Lys Ser Glu Asn Leu
           35                  40                  45
   Ile Lys Gln His Ile Ala Pro Leu Met Gln Phe Leu Ile Asp Glu Leu
       50                  55                  60
   Asn Val Lys Ser Lys Ile Gln Ile Val Lys Gly Asp Thr Arg Tyr Glu
   65                  70                  75                  80
   Leu Arg Val Ser Ser Lys Lys Leu Tyr Tyr Tyr Phe Ala Asn Met Leu
                   85                  90                  95
   Glu Arg Ile Arg Leu Phe Asn Met Arg Glu Gln Leu Ala Phe Leu Ala
               100                 105                 110
   Gly Phe Val Asp Gly Asp Gly Ser Ile Ile Ala Gln Ile Gln Pro Asn
                   115                 120                 125
   Gln Ser Ser Lys Phe Lys His Arg Leu Lys Leu Ala Phe Ala Val Thr
       130                 135                 140
   Gln Lys Thr Gln Arg Arg Trp Phe Leu Asp Lys Leu Val Asp Glu Ile
   145                 150                 155                 160
   Gly Val Gly Tyr Val His Asp Gln Gly Ser Val Ser Asn Tyr Ile Leu
                   165                 170                 175
   Ser Glu Ile Lys Pro Leu His Asn Phe Leu Thr Gln Leu Gln Pro Phe
                   180                 185                 190
   Leu Lys Leu Lys Gln Lys Gln Ala Asn Leu Val Leu Lys Ile Ile Trp
           195                 200                 205
   Arg Leu Pro Ser Ala Lys Glu Ser Pro Asp Lys Phe Leu Glu Val Cys
       210                 215                 220
   Thr Trp Val Asp Gln Ile Ala Ala Leu Asn Asp Ser Lys Thr Arg Lys
   225                 230                 235                 240
   Thr Thr Ser Glu Thr Val Arg Ala Val Leu Asp Ser Leu Ser Glu Lys
                   245                 250                 255
   Lys Lys Ser Ser Pro
                   260